# EUROPEAN PATENT APPLICATION

(11) **EP 2 006 377 A2**
(43) Date of publication of application: **24.12.2008**
(21) Application number: 07739717.2
(22) Date of filing: 27.03.2007
(51) Int. Cl.: C12N 15/00

(54) **NOVEL TUMOR ANTIGEN PEPTIDES**

(30) Priority: 28.03.2006 JP 2006086820
(71) Applicant: Dainippon Sumitomo Pharma Co., Ltd., Osaka 541-8524 (JP); Sato, Noriyuki, Sapporo-shi, Hokkaido 062-0042 (JP)
(72) Inventor: SATO, Noriyuki, Sapporo-shi, Hokkaido 062-0042 (JP); TORIGOE, Toshihiko, Sapporo-shi, Hokkaido 060-0001 (JP); HIROHASHI, Yoshihiko, Sapporo-shi, Hokkaido 063-0814 (JP); HARADA, Kenji, Osaka-shi, Osaka 541-8524 (JP)
(74) Representative: Vossius & Partner
(86) International application number: PCT/JP2007/056279
(87) International publication number: WO 2007/119515

(57) **Abstract**

The invention relates to novel tumor antigen proteins and peptides derived therefrom and use of the same in the filed of cancer immunity. Specifically, the inventions relates to a peptide which comprises a partial peptide derived from Lengsin, BJ-TSA-9, C20orf42, BUB1, C10orf3 or HIFPH3 and is capable of binding to an HLA antigen and is recognized by a CTL, and a pharmaceutical composition comprising the peptide and a pharmaceutically acceptable carrirer.

SEQUENCE LISTING

<110>  Sato, Noriyuki
       Dainippon Sumitomo Pharma Co., Ltd.

<120>  Novel tumor antigen peptides

<130>  P3095 EP S3

<150>  JP 2006-086820
<151>  2006-03-28

<160>  213

<170>  PatentIn version 3.1

<210>  1
<211>  1947
<212>  DNA
<213>  Homo sapiens

<220>
<221>  CDS
<222>  (35)..(1564)
<223>

<400>  1
gtggtttcta acgctgaact taaaaagtgt tgag atg aat aat gaa gag gac ctt      55
                                      Met Asn Asn Glu Glu Asp Leu
                                      1               5

ctg cag gag gac tca aca aga gat gaa ggc aat gag act gaa gcc aac      103
Leu Gln Glu Asp Ser Thr Arg Asp Glu Gly Asn Glu Thr Glu Ala Asn
        10                  15                  20

agc atg aac aca tta aga agg aca agg aag aaa gtc act aaa cca tat      151
Ser Met Asn Thr Leu Arg Arg Thr Arg Lys Lys Val Thr Lys Pro Tyr
    25                  30                  35

gtt tgt tca act gaa gtg gga gaa acg gat atg tcc aat tca aat gat      199
Val Cys Ser Thr Glu Val Gly Glu Thr Asp Met Ser Asn Ser Asn Asp
40                  45                  50                  55

tgc atg agg gac agc agt caa att ttg acc cca cct caa ctc tct tct      247
Cys Met Arg Asp Ser Ser Gln Ile Leu Thr Pro Pro Gln Leu Ser Ser
                60                  65                  70

aga atg aaa cac att aga caa gcc atg gcc aaa aat cgc ctc cag ttt      295
Arg Met Lys His Ile Arg Gln Ala Met Ala Lys Asn Arg Leu Gln Phe
            75                  80                  85

gta cga ttt gaa gca aca gac ctc cac ggc gtg tcc agg tct aag act      343
Val Arg Phe Glu Ala Thr Asp Leu His Gly Val Ser Arg Ser Lys Thr
        90                  95                  100

atc cct gca cac ttt ttt caa gag aaa gtg agc cat ggt gtt tgc atg      391
Ile Pro Ala His Phe Phe Gln Glu Lys Val Ser His Gly Val Cys Met
    105                 110                 115

ccc cga ggt tat ctt gaa gtg ata cca aat cca aag gac aat gaa atg      439
Pro Arg Gly Tyr Leu Glu Val Ile Pro Asn Pro Lys Asp Asn Glu Met
120                 125                 130                 135

aat aac ata aga gcc aca tgt ttt aat agc gac ata gtc cta atg cca 487
Asn Asn Ile Arg Ala Thr Cys Phe Asn Ser Asp Ile Val Leu Met Pro
                140                 145                 150

gag tta tca acc ttt aga gtt ttg cca tgg gct gac aga act gca aga 535
Glu Leu Ser Thr Phe Arg Val Leu Pro Trp Ala Asp Arg Thr Ala Arg
            155                 160                 165

gtg ata tgt gat acc ttc act gtg act ggt gag cct ctt ttg act tcc 583
Val Ile Cys Asp Thr Phe Thr Val Thr Gly Glu Pro Leu Leu Thr Ser
        170                 175                 180

cca agg tac att gca aag agg cag ctg agc cat ctg cag gcc tct ggc 631
Pro Arg Tyr Ile Ala Lys Arg Gln Leu Ser His Leu Gln Ala Ser Gly
    185                 190                 195

ttt tcc ctg ctt tct gct ttc atc tat gat ttt tgc att ttt ggt gtg 679
Phe Ser Leu Leu Ser Ala Phe Ile Tyr Asp Phe Cys Ile Phe Gly Val
200                 205                 210                 215

ccc gaa att tta aat tca aag att ata tct ttt cct gct tta aca ttt 727
Pro Glu Ile Leu Asn Ser Lys Ile Ile Ser Phe Pro Ala Leu Thr Phe
                220                 225                 230

tta aat aac cat gat cag ccc ttc atg cag gaa ctt gtt gat ggc ttg 775
Leu Asn Asn His Asp Gln Pro Phe Met Gln Glu Leu Val Asp Gly Leu
            235                 240                 245

tat cac act gga gcc aat gtc gag agt ttt tcc tcc tct acc agg cct 823
Tyr His Thr Gly Ala Asn Val Glu Ser Phe Ser Ser Ser Thr Arg Pro
        250                 255                 260

ggt cag atg gaa atc tct ttc ctg cct gaa ttt ggc att agc tca gct 871
Gly Gln Met Glu Ile Ser Phe Leu Pro Glu Phe Gly Ile Ser Ser Ala
    265                 270                 275

gat aat gca ttt acc ctc aga aca ggt gtc aaa gaa gtg gca agg aaa 919
Asp Asn Ala Phe Thr Leu Arg Thr Gly Val Lys Glu Val Ala Arg Lys
280                 285                 290                 295

tat aat tac att gcc agc ttc ttc att gag act gga ttt tgt gat tca 967
Tyr Asn Tyr Ile Ala Ser Phe Phe Ile Glu Thr Gly Phe Cys Asp Ser
                300                 305                 310

ggg att ttg tct cat agt ctc tgg gat gtc gat agg aag aaa aac atg 1015
Gly Ile Leu Ser His Ser Leu Trp Asp Val Asp Arg Lys Lys Asn Met
            315                 320                 325

ttc tgc agc act tct gga act gag cag ctc acg atc act ggg aaa aaa 1063
Phe Cys Ser Thr Ser Gly Thr Glu Gln Leu Thr Ile Thr Gly Lys Lys
        330                 335                 340

tgg ttg gca gga ctc ttg aag cac tct gct gcg ctc agc tgc ctg atg 1111
Trp Leu Ala Gly Leu Leu Lys His Ser Ala Ala Leu Ser Cys Leu Met
    345                 350                 355

gcg cct tct gtt agc tgc cga aag cgt tat tcc aag gac agg aaa gac 1159
Ala Pro Ser Val Ser Cys Arg Lys Arg Tyr Ser Lys Asp Arg Lys Asp
360                 365                 370                 375

ctg aag aag agt gtg cct aca aca tgg gga tac aat gac aac agc tgt 1207
Leu Lys Lys Ser Val Pro Thr Thr Trp Gly Tyr Asn Asp Asn Ser Cys
                380                 385                 390

ata ttt aat atc aaa tgt cat gga gag aaa ggc acc cgg ata gaa aat     1255
Ile Phe Asn Ile Lys Cys His Gly Glu Lys Gly Thr Arg Ile Glu Asn
            395                 400                 405

aaa cta ggc tca gca aca gca aac cct tac ttg gtg ctg gct gca act     1303
Lys Leu Gly Ser Ala Thr Ala Asn Pro Tyr Leu Val Leu Ala Ala Thr
        410                 415                 420

gtt gct gcc ggc tta gat gga ctt cat agc agt aat gag gtc ttg gct     1351
Val Ala Ala Gly Leu Asp Gly Leu His Ser Ser Asn Glu Val Leu Ala
    425                 430                 435

ggt cca gat gag agc aca gac ttt tac caa gtg gaa cct tct gag atc     1399
Gly Pro Asp Glu Ser Thr Asp Phe Tyr Gln Val Glu Pro Ser Glu Ile
440                 445                 450                 455

cct tta aaa cta gaa gat gcc ctt gtg gca ctg gaa gaa gat caa tgc     1447
Pro Leu Lys Leu Glu Asp Ala Leu Val Ala Leu Glu Glu Asp Gln Cys
                460                 465                 470

cag aga cag gct cta gga gaa acc ttt att cga tat ttt gtt gcc atg     1495
Gln Arg Gln Ala Leu Gly Glu Thr Phe Ile Arg Tyr Phe Val Ala Met
            475                 480                 485

aag aaa tat gag ttg gag aat gaa gaa ata gct gca gag aga aat aaa     1543
Lys Lys Tyr Glu Leu Glu Asn Glu Glu Ile Ala Ala Glu Arg Asn Lys
        490                 495                 500

ttc tta gag tat ttt att tag aatagagctc acaactactc ctttagacat        1594
Phe Leu Glu Tyr Phe Ile
    505

gtaattgtta cttaaagcta atctaccaaa acaaaaagac tgaacttttg taattaacaa   1654

cagcaacaat aacaagatta cgaatgcttt tgactttttt ttttgtccat ggaatatttg   1714

acagatgaag taggactgct gagaagattc tgataacaga aacaaacaac aaagttgtgg   1774

tgaagctata atatgcaaac ttaccagatc ttgtcagtca tttcctatgt gtatgttgac   1834

ctggataaga atatccaatt ttgggtggca ataaatatat tcgcacatta agaaaaaaaa   1894

gacttaagca ttagaaagca aaatttaaat gactaaaaaa aaaaaaaaaa aaa          1947


<210>  2
<211>  509
<212>  PRT
<213>  Homo sapiens

<400>  2

Met Asn Asn Glu Glu Asp Leu Leu Gln Glu Asp Ser Thr Arg Asp Glu
1               5                   10                  15


Gly Asn Glu Thr Glu Ala Asn Ser Met Asn Thr Leu Arg Arg Thr Arg
            20                  25                  30


Lys Lys Val Thr Lys Pro Tyr Val Cys Ser Thr Glu Val Gly Glu Thr
        35                  40                  45

Asp Met Ser Asn Ser Asn Asp Cys Met Arg Asp Ser Ser Gln Ile Leu
    50                  55                  60

Thr Pro Pro Gln Leu Ser Ser Arg Met Lys His Ile Arg Gln Ala Met
65                  70                  75                  80

Ala Lys Asn Arg Leu Gln Phe Val Arg Phe Glu Ala Thr Asp Leu His
                85                  90                  95

Gly Val Ser Arg Ser Lys Thr Ile Pro Ala His Phe Phe Gln Glu Lys
            100                 105                 110

Val Ser His Gly Val Cys Met Pro Arg Gly Tyr Leu Glu Val Ile Pro
        115                 120                 125

Asn Pro Lys Asp Asn Glu Met Asn Asn Ile Arg Ala Thr Cys Phe Asn
    130                 135                 140

Ser Asp Ile Val Leu Met Pro Glu Leu Ser Thr Phe Arg Val Leu Pro
145                 150                 155                 160

Trp Ala Asp Arg Thr Ala Arg Val Ile Cys Asp Thr Phe Thr Val Thr
                165                 170                 175

Gly Glu Pro Leu Leu Thr Ser Pro Arg Tyr Ile Ala Lys Arg Gln Leu
            180                 185                 190

Ser His Leu Gln Ala Ser Gly Phe Ser Leu Leu Ser Ala Phe Ile Tyr
        195                 200                 205

Asp Phe Cys Ile Phe Gly Val Pro Glu Ile Leu Asn Ser Lys Ile Ile
    210                 215                 220

Ser Phe Pro Ala Leu Thr Phe Leu Asn Asn His Asp Gln Pro Phe Met
225                 230                 235                 240

Gln Glu Leu Val Asp Gly Leu Tyr His Thr Gly Ala Asn Val Glu Ser
                245                 250                 255

Phe Ser Ser Ser Thr Arg Pro Gly Gln Met Glu Ile Ser Phe Leu Pro
            260                 265                 270

Glu Phe Gly Ile Ser Ser Ala Asp Asn Ala Phe Thr Leu Arg Thr Gly
        275                 280                 285

Val Lys Glu Val Ala Arg Lys Tyr Asn Tyr Ile Ala Ser Phe Phe Ile

290                 295                 300

Glu Thr Gly Phe Cys Asp Ser Gly Ile Leu Ser His Ser Leu Trp Asp
305                 310                 315                 320

Val Asp Arg Lys Lys Asn Met Phe Cys Ser Thr Ser Gly Thr Glu Gln
                325                 330                 335

Leu Thr Ile Thr Gly Lys Lys Trp Leu Ala Gly Leu Leu Lys His Ser
            340                 345                 350

Ala Ala Leu Ser Cys Leu Met Ala Pro Ser Val Ser Cys Arg Lys Arg
        355                 360                 365

Tyr Ser Lys Asp Arg Lys Asp Leu Lys Lys Ser Val Pro Thr Thr Trp
    370                 375                 380

Gly Tyr Asn Asp Asn Ser Cys Ile Phe Asn Ile Lys Cys His Gly Glu
385                 390                 395                 400

Lys Gly Thr Arg Ile Glu Asn Lys Leu Gly Ser Ala Thr Ala Asn Pro
                405                 410                 415

Tyr Leu Val Leu Ala Ala Thr Val Ala Ala Gly Leu Asp Gly Leu His
            420                 425                 430

Ser Ser Asn Glu Val Leu Ala Gly Pro Asp Glu Ser Thr Asp Phe Tyr
        435                 440                 445

Gln Val Glu Pro Ser Glu Ile Pro Leu Lys Leu Glu Asp Ala Leu Val
    450                 455                 460

Ala Leu Glu Glu Asp Gln Cys Gln Arg Gln Ala Leu Gly Glu Thr Phe
465                 470                 475                 480

Ile Arg Tyr Phe Val Ala Met Lys Lys Tyr Glu Leu Glu Asn Glu Glu
                485                 490                 495

Ile Ala Ala Glu Arg Asn Lys Phe Leu Glu Tyr Phe Ile
            500                 505

<210>  3
<211>  2946
<212>  DNA
<213>  Homo sapiens

<220>
<221>  CDS
<222>  (346)..(1650)

<223>

<400> 3
ggaaagccgg ctcaccttcg cctccccctg cggctgggag gagaggaaat atcccatggc 60

tgactgtgcc aaggaggtgt ctgagccagc cctcccggcc cgagggcagg gcaggtggcc 120

ctgagagata agccaatccc gcagctgcag atgaggagtt ctgagaagca ttgctcagga 180

cagcggtaaa tcacttcttg gaggtgccct gcacgccggt cctgggagca ggcggcctcc 240

cgggggtgcg ggagccccac tcctccgtgg tgtgttccat ttgcttccca catctggagg 300

agctgacgtg ccagcctccc ccagcaccac ccagggacgg gaggc atg agc cgg tca 357
Met Ser Arg Ser
1

agg cac ctg ggc aaa atc cgg aag cgt ctg gaa gat gtc aag agc cag 405
Arg His Leu Gly Lys Ile Arg Lys Arg Leu Glu Asp Val Lys Ser Gln
5                   10                  15                  20

tgg gtc cgg cca gcc agg gct gac ttt agt gac aac gag agt gcc cgg 453
Trp Val Arg Pro Ala Arg Ala Asp Phe Ser Asp Asn Glu Ser Ala Arg
                25                  30                  35

ctg gcc acg gac gcc ctc ttg gat ggg ggt tct gaa gcc tac tgg cgg 501
Leu Ala Thr Asp Ala Leu Leu Asp Gly Gly Ser Glu Ala Tyr Trp Arg
            40                  45                  50

gtg ctc agc cag gaa ggc gag gtg gac ttc ttg tcc tcg gtg gag gcc 549
Val Leu Ser Gln Glu Gly Glu Val Asp Phe Leu Ser Ser Val Glu Ala
        55                  60                  65

cag tac atc cag gcc cag gcc agg gag ccc ccg tgt ccc cca gac acc 597
Gln Tyr Ile Gln Ala Gln Ala Arg Glu Pro Pro Cys Pro Pro Asp Thr
    70                  75                  80

ctg gga ggg gcg gaa gca ggc cct aag gga ctg gac tcc agc tcc cta 645
Leu Gly Gly Ala Glu Ala Gly Pro Lys Gly Leu Asp Ser Ser Ser Leu
85                  90                  95                  100

cag tcc ggc acc tac ttc cct gtg gcc tca gag ggc agc gag ccg gcc 693
Gln Ser Gly Thr Tyr Phe Pro Val Ala Ser Glu Gly Ser Glu Pro Ala
                105                 110                 115

cta ctg cac agc tgg gcc tca gct gag aag ccc tac ctg aag gaa aaa 741
Leu Leu His Ser Trp Ala Ser Ala Glu Lys Pro Tyr Leu Lys Glu Lys
            120                 125                 130

tcc agc gcc act gtg tac ttc cag acc gtc aag cac aac aac atc aga 789
Ser Ser Ala Thr Val Tyr Phe Gln Thr Val Lys His Asn Asn Ile Arg
        135                 140                 145

gac ctc gtc cgc cgc tgc atc acc cgg act agc cag gtc ctg gtc atc 837
Asp Leu Val Arg Arg Cys Ile Thr Arg Thr Ser Gln Val Leu Val Ile
    150                 155                 160

ctg atg gat gtg ttc acg gat gtg gag atc ttc tgt gac att cta gag 885
Leu Met Asp Val Phe Thr Asp Val Glu Ile Phe Cys Asp Ile Leu Glu
165                 170                 175                 180

gca gcc aac aag cgt ggg gtg ttc gtt tgt gtg ctc ctg gac cag gga 933
Ala Ala Asn Lys Arg Gly Val Phe Val Cys Val Leu Leu Asp Gln Gly

185                 190                 195

ggt gtg aag ctc ttc cag gag atg tgt gac aaa gtc cag atc tct gac     981
Gly Val Lys Leu Phe Gln Glu Met Cys Asp Lys Val Gln Ile Ser Asp
            200                 205                 210

agt cac ctc aag aac att tcc atc cgg agt gtg gaa gga gag ata tac     1029
Ser His Leu Lys Asn Ile Ser Ile Arg Ser Val Glu Gly Glu Ile Tyr
        215                 220                 225

tgt gcc aag tca ggc agg aaa ttc gct ggc caa atc cgg gag aag ttc     1077
Cys Ala Lys Ser Gly Arg Lys Phe Ala Gly Gln Ile Arg Glu Lys Phe
    230                 235                 240

atc atc tcg gac tgg aga ttt gtc ctg tct gga tct tac agc ttc acc     1125
Ile Ile Ser Asp Trp Arg Phe Val Leu Ser Gly Ser Tyr Ser Phe Thr
245                 250                 255                 260

tgg ctc tgc gga cac gtg cac cgg aac atc ctc tcc aag ttc aca ggc     1173
Trp Leu Cys Gly His Val His Arg Asn Ile Leu Ser Lys Phe Thr Gly
                265                 270                 275

cag gcg gtg gag ctg ttt gac gag gag ttc cgc cac ctc tac gcc tcc     1221
Gln Ala Val Glu Leu Phe Asp Glu Glu Phe Arg His Leu Tyr Ala Ser
            280                 285                 290

tcc aag cct gtg atg ggc ctg aag tcc ccg cgg ctg gtc gcc ccc gtc     1269
Ser Lys Pro Val Met Gly Leu Lys Ser Pro Arg Leu Val Ala Pro Val
        295                 300                 305

ccg ccc gga gca gcc ccg gcc aat ggc cgc ctt agc agc agc agt ggc     1317
Pro Pro Gly Ala Ala Pro Ala Asn Gly Arg Leu Ser Ser Ser Ser Gly
    310                 315                 320

tcc gcc agt gac cgc acg tcc tcc aac ccc ttc agc ggc cgc tcg gca     1365
Ser Ala Ser Asp Arg Thr Ser Ser Asn Pro Phe Ser Gly Arg Ser Ala
325                 330                 335                 340

ggc agc cac ccc ggt acc cga agt gtg tcc gcg tct tca ggg ccc tgt     1413
Gly Ser His Pro Gly Thr Arg Ser Val Ser Ala Ser Ser Gly Pro Cys
                345                 350                 355

agc ccc gcg gcc cca cac ccg cct cca ccg ccc cgg ttc cag ccc cac     1461
Ser Pro Ala Ala Pro His Pro Pro Pro Pro Pro Arg Phe Gln Pro His
            360                 365                 370

caa ggc cct tgg gga gcc ccg agt ccc cag gcc cac ctc tcc ccg cgg     1509
Gln Gly Pro Trp Gly Ala Pro Ser Pro Gln Ala His Leu Ser Pro Arg
        375                 380                 385

ccc cac gac ggc ccg ccc gcc gct gtc tac agc aac ctg ggg gcc tac     1557
Pro His Asp Gly Pro Pro Ala Ala Val Tyr Ser Asn Leu Gly Ala Tyr
    390                 395                 400

agg ccc acg cgg ctg cag ctg gag cag ctg ggc ctg gtg ccg agg ctg     1605
Arg Pro Thr Arg Leu Gln Leu Glu Gln Leu Gly Leu Val Pro Arg Leu
405                 410                 415                 420

act cca acc tgg agg ccc ttc ctg cag gcc tcc cct cac ttc tga         1650
Thr Pro Thr Trp Arg Pro Phe Leu Gln Ala Ser Pro His Phe
                425                 430

aggtcccatc ccctgctgcc ctccgcaggc ccagggctgg gcactccctg agacccaaag   1710

acccacctca acgacgagtg gcgttgagcc acttcccttt gaaaagacac tcaaaatcac 1770

tgccatggtt caatgttccc aggccccagg ccatccactt gccggccccc accagttctt 1830

gggttccccg ctctagtttg acctgtgcag cacattccag aaggttccag ggaggttgtg 1890

gggcagctag aggacaaaat catgaaaaca gagtccctgt cttccagaga tcatccgggg 1950

ctttaatatt aatggccccc aaaactccgt aagaagcagg aaatgcagcc caagttttac 2010

aaatgggtaa acagaggcac tgagagatag atggtagttt ggtacttctg gttcccagtg 2070

cccaggaatg gtccactccc aagaaattca ggaaagaaag actgaggaga aggtgtggga 2130

acattctgga tgtttcggga gagttgggga aactcctcct cttaggaaag gctaatacta 2190

gggtatcctt gggcccaatg aattaggggt gaggccccag aacccgttat ctatgagttg 2250

tatgggggag ccatctgaag ctgtagccac cagggatgca gctagctgag gagtttgggg 2310

tgttgggttg gacaaggcag gttagtagac tcagattctt gcttcaaaga gccttgggct 2370

ggcctggagg tccctggagt ctagactgga cctaggagct tgagttgtca ggggccagga 2430

ctggccccac tgcagtgccc aggccagtct tgagcagcag ggagggctca gctgtcccca 2490

gatccaggtg cctctgacca gcctggtcac ctcctgagga ataaatgctg aacctcacaa 2550

gccccatcat tcatttcttc tcaattcaca gtgcccctct ttgtttctgg ggtggaacta 2610

ggtcctgagg gcacagccta gctgagtgca aagaaatata ggatgcttag aaagcataca 2670

ggaggggcca ggcgtggtgg ctcatgcctg taatcccaga actttgggat gccaaggtgg 2730

ttggattacc tgagatcagg tggattacct ggtctcgaga ccagcctgac caatatggtg 2790

aaaccccgtc tctactaaaa atacaaaaat taggctgaga caggagaatt gcttgaaccc 2850

aggaagcaga ggttgcaatg agctgagatt gcatcactgc actccagcat gggcaacaaa 2910

gcaagactcc gtcacagaaa aaaaaaaaaa aaaaaa 2946

<210> 4
<211> 434
<212> PRT
<213> Homo sapiens

<400> 4

Met Ser Arg Ser Arg His Leu Gly Lys Ile Arg Lys Arg Leu Glu Asp
1               5                   10                  15

Val Lys Ser Gln Trp Val Arg Pro Ala Arg Ala Asp Phe Ser Asp Asn
            20                  25                  30

Glu Ser Ala Arg Leu Ala Thr Asp Ala Leu Leu Asp Gly Gly Ser Glu
        35                  40                  45

Ala Tyr Trp Arg Val Leu Ser Gln Glu Gly Glu Val Asp Phe Leu Ser
    50                  55                  60

Ser Val Glu Ala Gln Tyr Ile Gln Ala Gln Ala Arg Glu Pro Pro Cys
65                  70                  75                  80

Pro Pro Asp Thr Leu Gly Gly Ala Glu Ala Gly Pro Lys Gly Leu Asp
                85                  90                  95

Ser Ser Ser Leu Gln Ser Gly Thr Tyr Phe Pro Val Ala Ser Glu Gly
            100                 105                 110

Ser Glu Pro Ala Leu Leu His Ser Trp Ala Ser Ala Glu Lys Pro Tyr
        115                 120                 125

Leu Lys Glu Lys Ser Ser Ala Thr Val Tyr Phe Gln Thr Val Lys His
    130                 135                 140

Asn Asn Ile Arg Asp Leu Val Arg Arg Cys Ile Thr Arg Thr Ser Gln
145                 150                 155                 160

Val Leu Val Ile Leu Met Asp Val Phe Thr Asp Val Glu Ile Phe Cys
                165                 170                 175

Asp Ile Leu Glu Ala Ala Asn Lys Arg Gly Val Phe Val Cys Val Leu
            180                 185                 190

Leu Asp Gln Gly Gly Val Lys Leu Phe Gln Glu Met Cys Asp Lys Val
        195                 200                 205

Gln Ile Ser Asp Ser His Leu Lys Asn Ile Ser Ile Arg Ser Val Glu
    210                 215                 220

Gly Glu Ile Tyr Cys Ala Lys Ser Gly Arg Lys Phe Ala Gly Gln Ile
225                 230                 235                 240

Arg Glu Lys Phe Ile Ile Ser Asp Trp Arg Phe Val Leu Ser Gly Ser
                245                 250                 255

Tyr Ser Phe Thr Trp Leu Cys Gly His Val His Arg Asn Ile Leu Ser
            260                 265                 270

Lys Phe Thr Gly Gln Ala Val Glu Leu Phe Asp Glu Glu Phe Arg His
        275                 280                 285

Leu Tyr Ala Ser Ser Lys Pro Val Met Gly Leu Lys Ser Pro Arg Leu
    290                 295                 300

Val Ala Pro Val Pro Pro Gly Ala Ala Pro Ala Asn Gly Arg Leu Ser
305                 310                 315                 320

Ser Ser Ser Gly Ser Ala Ser Asp Arg Thr Ser Ser Asn Pro Phe Ser
                325                 330                 335

Gly Arg Ser Ala Gly Ser His Pro Gly Thr Arg Ser Val Ser Ala Ser
            340                 345                 350

Ser Gly Pro Cys Ser Pro Ala Ala Pro His Pro Pro Pro Pro Pro Arg
        355                 360                 365

Phe Gln Pro His Gln Gly Pro Trp Gly Ala Pro Ser Pro Gln Ala His
    370                 375                 380

Leu Ser Pro Arg Pro His Asp Gly Pro Pro Ala Ala Val Tyr Ser Asn
385                 390                 395                 400

Leu Gly Ala Tyr Arg Pro Thr Arg Leu Gln Leu Glu Gln Leu Gly Leu
                405                 410                 415

Val Pro Arg Leu Thr Pro Thr Trp Arg Pro Phe Leu Gln Ala Ser Pro
            420                 425                 430

His Phe

<210>  5
<211>  5168
<212>  DNA
<213>  Homo sapiens

<220>
<221>  CDS
<222>  (790)..(2823)
<223>

<400>  5
ctggagtctc ccctggtcgg gagcctcagc cttctggaga ctgacaccac cctcttactc      60

agagacgaat cgttttgtcg ttgccttcac ctccctgacc acaagtgctc cggggctctt     120

tctaagggag gcagctgttc taggcgggag gctggagtct cctgggcctg gacgcacccc     180

ggggtgtgag tgatgggtat gcctgaaagg aggggaagtg gcgcggcttt aatcatctgg     240

gctagtcccc ggcgggcctg ggggaacagg gaaactggag gcggccttaa agcgtcagga     300

tgagacatcg caagaggagc tgcagatact gagcgtgcgc cccgggttct cgccgccttc     360

tctccgccga gcagcccttc ggccaccctt tgcccttaaa aatctgcaga ctgcgcctcc     420

tctccgcggg agcgagacct agcaggcccg gggctgggcg tgccctcgcc tgccacgctg     480

cgcgctgccc tcagccgggc cgctggggcc gtgcagtgca ccgggcacgc cgcgccaggc 540

tgggggcagg caccgagcct ccgtgggagg tcccgaggca gcttcgcctg ctcgccctgg 600

ctccagccct cacctgccgc agccttagct gagcagccgc cgccactggg cgccccccgc 660

tccccacttc gccagcgccc gctcctcggc tcggcccggg gtagtttgta gggacgcagc 720

tctccacgtg cgcgactgcg aggctggacg ctacgggctc ctggaaagga gacaccagca 780

tttgccaca atg ctg tca tcc act gac ttt aca ttt gct tcc tgg gag ctt 831
Met Leu Ser Ser Thr Asp Phe Thr Phe Ala Ser Trp Glu Leu
1               5                   10

gtg gtc cgc gtt gac cat ccc aat gaa gag cag cag aaa gac gtc aca 879
Val Val Arg Val Asp His Pro Asn Glu Glu Gln Gln Lys Asp Val Thr
15                  20                  25                  30

ctg aga gta tct gga gac ctt cat gtt gga gga gtg atg ctc aag tta 927
Leu Arg Val Ser Gly Asp Leu His Val Gly Gly Val Met Leu Lys Leu
                35                  40                  45

gta gaa cag atc aat ata tcc caa gac tgg tca gac ttt gct ctt tgg 975
Val Glu Gln Ile Asn Ile Ser Gln Asp Trp Ser Asp Phe Ala Leu Trp
            50                  55                  60

tgg gaa cag aag cat tgc tgg ctt ctg aaa acc cac tgg acc ctg gac 1023
Trp Glu Gln Lys His Cys Trp Leu Leu Lys Thr His Trp Thr Leu Asp
        65                  70                  75

aaa tat ggg gtc cag gca gat gca aag ctt ctc ttc acc cct cag cat 1071
Lys Tyr Gly Val Gln Ala Asp Ala Lys Leu Leu Phe Thr Pro Gln His
    80                  85                  90

aaa atg ctg cgc ctt cgt ctg ccg aat ttg aag atg gtg agg ttg cga 1119
Lys Met Leu Arg Leu Arg Leu Pro Asn Leu Lys Met Val Arg Leu Arg
95                  100                 105                 110

gtc agc ttc tca gct gtg gtt ttt aaa gct gtc agt gat atc tgc aaa 1167
Val Ser Phe Ser Ala Val Val Phe Lys Ala Val Ser Asp Ile Cys Lys
                115                 120                 125

atc ctg aat att aga aga tca gaa gag ctt tcc ttg tta aag ccg tct 1215
Ile Leu Asn Ile Arg Arg Ser Glu Glu Leu Ser Leu Leu Lys Pro Ser
            130                 135                 140

ggt gac tat ttt aag aag aag aag aaa aaa gac aaa aat aat aag gaa 1263
Gly Asp Tyr Phe Lys Lys Lys Lys Lys Lys Asp Lys Asn Asn Lys Glu
        145                 150                 155

ccc ata att gaa gat att cta aac ctg gag agt tct cca aca gct tca 1311
Pro Ile Ile Glu Asp Ile Leu Asn Leu Glu Ser Ser Pro Thr Ala Ser
    160                 165                 170

ggt tca tca gta agt cct ggt tta tac agt aaa acc atg acc cct ata 1359
Gly Ser Ser Val Ser Pro Gly Leu Tyr Ser Lys Thr Met Thr Pro Ile
175                 180                 185                 190

tat gac ccc atc aat gga aca cca gca tca tcc acc atg act tgg ttc 1407
Tyr Asp Pro Ile Asn Gly Thr Pro Ala Ser Ser Thr Met Thr Trp Phe
                195                 200                 205

agt gac agc cct ttg acg gaa caa aac tgc agc atc ctc gca ttc agc 1455
Ser Asp Ser Pro Leu Thr Glu Gln Asn Cys Ser Ile Leu Ala Phe Ser
            210                 215                 220

caa ccc ccc cag tcc cca gaa gca ctt gcg gat atg tac cag cct cgg 1503
Gln Pro Pro Gln Ser Pro Glu Ala Leu Ala Asp Met Tyr Gln Pro Arg
        225                 230                 235

tct ctg gtt gat aaa gcc aag ctc aat gca ggt tgg cta gac tcc tca 1551
Ser Leu Val Asp Lys Ala Lys Leu Asn Ala Gly Trp Leu Asp Ser Ser
    240                 245                 250

cgc tcc ctt atg gaa caa ggc atc caa gag gat gag cag ctg ctc tta 1599
Arg Ser Leu Met Glu Gln Gly Ile Gln Glu Asp Glu Gln Leu Leu Leu
255                 260                 265                 270

cga ttt aaa tat tat tct ttc ttc gac ttg aat cct aaa tat gat gct 1647
Arg Phe Lys Tyr Tyr Ser Phe Phe Asp Leu Asn Pro Lys Tyr Asp Ala
                275                 280                 285

gtc cga ata aac caa ctc tat gag caa gcc agg tgg gcc att ctc tta 1695
Val Arg Ile Asn Gln Leu Tyr Glu Gln Ala Arg Trp Ala Ile Leu Leu
            290                 295                 300

gaa gaa att gat tgc aca gag gaa gaa atg ttg atc ttt gca gct cta 1743
Glu Glu Ile Asp Cys Thr Glu Glu Glu Met Leu Ile Phe Ala Ala Leu
        305                 310                 315

cag tac cac att agc aaa ctg tcg ttg tct gct gaa aca cag gat ttt 1791
Gln Tyr His Ile Ser Lys Leu Ser Leu Ser Ala Glu Thr Gln Asp Phe
    320                 325                 330

gca ggc gag tcc gag gtt gat gaa ata gaa gcg gcg ctt tct aat ttg 1839
Ala Gly Glu Ser Glu Val Asp Glu Ile Glu Ala Ala Leu Ser Asn Leu
335                 340                 345                 350

gaa gta acc cta gaa ggt gga aaa gcg gac agc ctt ttg gag gac att 1887
Glu Val Thr Leu Glu Gly Gly Lys Ala Asp Ser Leu Leu Glu Asp Ile
                355                 360                 365

act gat atc cct aaa ctt gca gat aat ctc aaa tta ttt agg ccc aag 1935
Thr Asp Ile Pro Lys Leu Ala Asp Asn Leu Lys Leu Phe Arg Pro Lys
            370                 375                 380

aag tta cta cca aaa gct ttc aaa caa tat tgg ttt atc ttt aaa gac 1983
Lys Leu Leu Pro Lys Ala Phe Lys Gln Tyr Trp Phe Ile Phe Lys Asp
        385                 390                 395

aca tcc ata gca tac ttt aaa aat aag gaa ctt gaa caa gga gaa cca 2031
Thr Ser Ile Ala Tyr Phe Lys Asn Lys Glu Leu Glu Gln Gly Glu Pro
    400                 405                 410

cta gaa aaa cta aat ctt aga ggc tgc gaa gtt gtg ccc gat gta aat 2079
Leu Glu Lys Leu Asn Leu Arg Gly Cys Glu Val Val Pro Asp Val Asn
415                 420                 425                 430

gta gca gga aga aaa ttt gga atc aag tta cta atc cct gtt gcc gat 2127
Val Ala Gly Arg Lys Phe Gly Ile Lys Leu Leu Ile Pro Val Ala Asp
                435                 440                 445

ggt atg aat gaa atg tat ttg aga tgt gac cat gag aat caa tac gcc 2175
Gly Met Asn Glu Met Tyr Leu Arg Cys Asp His Glu Asn Gln Tyr Ala
            450                 455                 460

caa tgg atg gct gcc tgc atg ttg gca tcg aag ggc aaa acc atg gca 2223
Gln Trp Met Ala Ala Cys Met Leu Ala Ser Lys Gly Lys Thr Met Ala
        465                 470                 475

gac agc tcc tac cag cca gag gtc ctc aac atc ctt tca ttt ctg agg 2271
Asp Ser Ser Tyr Gln Pro Glu Val Leu Asn Ile Leu Ser Phe Leu Arg
    480                 485                 490

atg aaa aac agg aac tct gca tct cag gtg gct tcc agt ctc gaa aac 2319
Met Lys Asn Arg Asn Ser Ala Ser Gln Val Ala Ser Ser Leu Glu Asn
495                 500                 505                 510

atg gat atg aac cca gaa tgt ttt gtg tca cca cgg tgt gca aag aaa 2367
Met Asp Met Asn Pro Glu Cys Phe Val Ser Pro Arg Cys Ala Lys Lys
                515                 520                 525

cac aaa tcc aaa cag ctg gcc gcc cgg atc ctg gag gcg cac cag aac 2415
His Lys Ser Lys Gln Leu Ala Ala Arg Ile Leu Glu Ala His Gln Asn
            530                 535                 540

gtg gcc cag atg ccc ctg gtc gaa gcc aag ctg cgg ttc atc cag gcg 2463
Val Ala Gln Met Pro Leu Val Glu Ala Lys Leu Arg Phe Ile Gln Ala
        545                 550                 555

tgg cag tca ctg cct gag ttt ggc ctc acc tac tac ctt gtc aga ttt 2511
Trp Gln Ser Leu Pro Glu Phe Gly Leu Thr Tyr Tyr Leu Val Arg Phe
    560                 565                 570

aaa gga agc aaa aaa gat gac att ctg gga gtt tca tat aac agg ttg 2559
Lys Gly Ser Lys Lys Asp Asp Ile Leu Gly Val Ser Tyr Asn Arg Leu
575                 580                 585                 590

att aaa att gat gca gcc acc ggg att cca gtg aca aca tgg aga ttc 2607
Ile Lys Ile Asp Ala Ala Thr Gly Ile Pro Val Thr Thr Trp Arg Phe
                595                 600                 605

aca aat atc aaa cag tgg aat gta aac tgg gaa acc cgg cag gtg gtc 2655
Thr Asn Ile Lys Gln Trp Asn Val Asn Trp Glu Thr Arg Gln Val Val
            610                 615                 620

atc gag ttt gac caa aac gtc ttt act gct ttc acc tgc ctg agt gca 2703
Ile Glu Phe Asp Gln Asn Val Phe Thr Ala Phe Thr Cys Leu Ser Ala
        625                 630                 635

gat tgc aag att gtg cac gag tac att ggc ggc tac att ttc ttg tcc 2751
Asp Cys Lys Ile Val His Glu Tyr Ile Gly Gly Tyr Ile Phe Leu Ser
    640                 645                 650

acc cgc tcc aag gac cag aat gaa aca ctc gat gag gac ttg ttc cac 2799
Thr Arg Ser Lys Asp Gln Asn Glu Thr Leu Asp Glu Asp Leu Phe His
655                 660                 665                 670

aaa ttg acc ggc ggt cag gat tga aacaagcacg cgtgctcggc tcacaccaac 2853
Lys Leu Thr Gly Gly Gln Asp
                675

aaggcaagcc aaaggcgccc ctccccagag ggatccctaa cgtgcccagc atgtagattc 2913

tggactaaca gacaacatac attcaccgct ggtcacccag atcctcattc aaacccactg 2973

ctggcacatc cctttcctta ctttgccctg tgctaccagc cacggaagga gcctctcttg 3033

ttttttctat aaaatgggta ggcaggagaa aagcaggtgc cctaagattg ctctaaggcc 3093

cagcatgtgg ttacagttct ctgacttgca gaacctgcca ggtgtatggc tacaagttat 3153

cctcgtgctg atctgtctca ttactaagtc aatggagaag acagaaaggt aaaaatcacg 3213

tgtagcaaga acaactctta tttcacaaac tcaggtatga aacgaaacgc ctgtccttca 3273

tggaactgct tttagctcct gtcttttcaa aatggcagag ggagttccta cacacacttt 3333

ttccctggag gccaaggtct aggggtagaa aggggagggg tggggctacc aggtagcagt 3393

tgacaaccca aggtcagagg agtggccctc agtgtcatct gtccacagtg atacctgcca 3453

agatgaccac tgacccacat ctggtcttag tcattggtct cctcagattt ctggggccac 3513

ctgcaagccc cattccattc ctacagatct ctcagccacc tgtaagtcct ttgtgaagat 3573

gtgggtgaca cagggggaca ggaaaaccca tttctcaacc cagatccatg tctccactgc 3633

ttctactctg ggttgggatt caggaagaca ggcacagtcc tctctgttca tagaaacacc 3693

tgccagtgtc aaggattcca gtcaggtgtc tatcccaact ggtcagggag agaagggcag 3753

acccattctc aaagaccacc atgtccaagg tctgacagct ccccactggc tgcccccaca 3813

ggggctttag gctggtctgg gtcatgggga agcgtccctc ttatcgctgg tctgtgttct 3873

cctggatttg gtatctatgt tggtacgact cctggccttt tatctaaagg actttggctt 3933

ttgtaaatca caagccaata atagactttt ttctccccct ctgttttttg ctgtgtcatc 3993

tctgccttga gactgccttg agacagtgct tgccttgaga gagtgagcca attaacagct 4053

gcctgaattg tcattttcca ttttggtttg ttagaggtgg gaggggtggg ttttgagaag 4113

gtcaaaagca ataccagaag taaagggaaa tatcagacaa tattttatta ttttttcata 4173

gatgttctgc cacacaaaga acttggggtg taaggataag gcaaaagctc caatcccatt 4233

tttcagttct cctaggatgc acccctcagg gagcctggcc agagttccga ggctcgtgag 4293

cgtcagctgt tgctttattt tccatcaaag ccctctgaga agtgagacct cagcaattcc 4353

gggagccaca tagagacaga cttggcaagg gaccccctgg ttctgagcca gtagctgcca 4413

tctggaaatt cctcttttag cctctcctta gaggtgaatg tgaatgaagc ctcccaggca 4473

cccgctgaat ttctgaggcc ttgcttaaag ctcagaagtg gtttaggcat ttggaaaatc 4533

tggttcacat cataaagaac ttgatttgaa atgttttcta tagaaacaag tgctaagtgt 4593

accgtattat acttgatgtt ggtcatttct cagtcctatt tctcagttct attattttag 4653

aacctagtca gttctttaag attataactg gtcctacatt aaaataatgc ttctcgatgt 4713

cagattttac ctgtttgctg ctgagaacat ctctgcctaa tttaccaaag ccagaccttc 4773

agttcaacat gcttccttag cttttcatag ttgtctgaca tttccatgaa aacaaaggaa 4833

ccaactttgt tttaaccaaa ctttgtttgg ttacagtttt caggggagcg tttcttccat 4893

gacacacagc aacatcccaa agaaataaac aagtgtgaca aaaaaaaaaa aaaacaaacc 4953

taaatgctac tgttccaaag agcaacttga tggttttttt taatactgag tgcaaaaggt 5013

cacccaaatt cctatgatga aattttaaat taatgggcac ctttcaacat catttgcttc 5073

cttatctaca gttgattcag aaatctgcat tttttattct tttatatgac ttttaagtaa 5133

aagatttata tggatttgaa aaaaaaaaaa aaaaa 5168

<210> 6
<211> 677
<212> PRT
<213> Homo sapiens

<400> 6

Met Leu Ser Ser Thr Asp Phe Thr Phe Ala Ser Trp Glu Leu Val Val
1               5                   10                  15

Arg Val Asp His Pro Asn Glu Glu Gln Gln Lys Asp Val Thr Leu Arg
            20                  25                  30

Val Ser Gly Asp Leu His Val Gly Gly Val Met Leu Lys Leu Val Glu
        35                  40                  45

Gln Ile Asn Ile Ser Gln Asp Trp Ser Asp Phe Ala Leu Trp Trp Glu
    50                  55                  60

Gln Lys His Cys Trp Leu Leu Lys Thr His Trp Thr Leu Asp Lys Tyr
65                  70                  75                  80

Gly Val Gln Ala Asp Ala Lys Leu Leu Phe Thr Pro Gln His Lys Met
                85                  90                  95

Leu Arg Leu Arg Leu Pro Asn Leu Lys Met Val Arg Leu Arg Val Ser
            100                 105                 110

Phe Ser Ala Val Val Phe Lys Ala Val Ser Asp Ile Cys Lys Ile Leu
        115                 120                 125

Asn Ile Arg Arg Ser Glu Glu Leu Ser Leu Leu Lys Pro Ser Gly Asp
    130                 135                 140

Tyr Phe Lys Lys Lys Lys Lys Lys Asp Lys Asn Asn Lys Glu Pro Ile
145                 150                 155                 160

Ile Glu Asp Ile Leu Asn Leu Glu Ser Ser Pro Thr Ala Ser Gly Ser
                165                 170                 175

Ser Val Ser Pro Gly Leu Tyr Ser Lys Thr Met Thr Pro Ile Tyr Asp
            180                 185                 190

Pro Ile Asn Gly Thr Pro Ala Ser Ser Thr Met Thr Trp Phe Ser Asp
        195                 200                 205

Ser Pro Leu Thr Glu Gln Asn Cys Ser Ile Leu Ala Phe Ser Gln Pro
    210                 215                 220

Pro Gln Ser Pro Glu Ala Leu Ala Asp Met Tyr Gln Pro Arg Ser Leu
225                 230                 235                 240

Val Asp Lys Ala Lys Leu Asn Ala Gly Trp Leu Asp Ser Ser Arg Ser
                245                 250                 255

Leu Met Glu Gln Gly Ile Gln Glu Asp Glu Gln Leu Leu Leu Arg Phe
            260                 265                 270

Lys Tyr Tyr Ser Phe Phe Asp Leu Asn Pro Lys Tyr Asp Ala Val Arg
        275                 280                 285

Ile Asn Gln Leu Tyr Glu Gln Ala Arg Trp Ala Ile Leu Leu Glu Glu
    290                 295                 300

Ile Asp Cys Thr Glu Glu Glu Met Leu Ile Phe Ala Ala Leu Gln Tyr
305                 310                 315                 320

His Ile Ser Lys Leu Ser Leu Ser Ala Glu Thr Gln Asp Phe Ala Gly
                325                 330                 335

Glu Ser Glu Val Asp Glu Ile Glu Ala Ala Leu Ser Asn Leu Glu Val
            340                 345                 350

Thr Leu Glu Gly Gly Lys Ala Asp Ser Leu Leu Glu Asp Ile Thr Asp
        355                 360                 365

Ile Pro Lys Leu Ala Asp Asn Leu Lys Leu Phe Arg Pro Lys Lys Leu
    370                 375                 380

Leu Pro Lys Ala Phe Lys Gln Tyr Trp Phe Ile Phe Lys Asp Thr Ser
385                 390                 395                 400

Ile Ala Tyr Phe Lys Asn Lys Glu Leu Glu Gln Gly Glu Pro Leu Glu
                405                 410                 415

Lys Leu Asn Leu Arg Gly Cys Glu Val Val Pro Asp Val Asn Val Ala
            420                 425                 430

Gly Arg Lys Phe Gly Ile Lys Leu Leu Ile Pro Val Ala Asp Gly Met

435                 440                 445

Asn Glu Met Tyr Leu Arg Cys Asp His Glu Asn Gln Tyr Ala Gln Trp
    450                 455                 460

Met Ala Ala Cys Met Leu Ala Ser Lys Gly Lys Thr Met Ala Asp Ser
465                 470                 475                 480

Ser Tyr Gln Pro Glu Val Leu Asn Ile Leu Ser Phe Leu Arg Met Lys
                485                 490                 495

Asn Arg Asn Ser Ala Ser Gln Val Ala Ser Ser Leu Glu Asn Met Asp
            500                 505                 510

Met Asn Pro Glu Cys Phe Val Ser Pro Arg Cys Ala Lys Lys His Lys
        515                 520                 525

Ser Lys Gln Leu Ala Ala Arg Ile Leu Glu Ala His Gln Asn Val Ala
    530                 535                 540

Gln Met Pro Leu Val Glu Ala Lys Leu Arg Phe Ile Gln Ala Trp Gln
545                 550                 555                 560

Ser Leu Pro Glu Phe Gly Leu Thr Tyr Tyr Leu Val Arg Phe Lys Gly
                565                 570                 575

Ser Lys Lys Asp Asp Ile Leu Gly Val Ser Tyr Asn Arg Leu Ile Lys
            580                 585                 590

Ile Asp Ala Ala Thr Gly Ile Pro Val Thr Thr Trp Arg Phe Thr Asn
        595                 600                 605

Ile Lys Gln Trp Asn Val Asn Trp Glu Thr Arg Gln Val Val Ile Glu
    610                 615                 620

Phe Asp Gln Asn Val Phe Thr Ala Phe Thr Cys Leu Ser Ala Asp Cys
625                 630                 635                 640

Lys Ile Val His Glu Tyr Ile Gly Gly Tyr Ile Phe Leu Ser Thr Arg
                645                 650                 655

Ser Lys Asp Gln Asn Glu Thr Leu Asp Glu Asp Leu Phe His Lys Leu
            660                 665                 670

Thr Gly Gly Gln Asp
        675

<210>  7
<211>  3486
<212>  DNA
<213>  Homo sapiens

<220>
<221>  CDS
<222>  (93)..(3350)
<223>

<400>  7
gagccgactg cggctgcgcg gggtattcga atcggcggcg gcttctagtt tgcggttcag      60

gtttggccgc tgccggccag cgtcctctgg cc atg gac acc ccg gaa aat gtc      113
                                    Met Asp Thr Pro Glu Asn Val
                                    1               5

ctt cag atg ctt gaa gcc cac atg cag agc tac aag ggc aat gac cct      161
Leu Gln Met Leu Glu Ala His Met Gln Ser Tyr Lys Gly Asn Asp Pro
        10                  15                  20

ctt ggt gaa tgg gaa aga tac ata cag tgg gta gaa gag aat ttt cct      209
Leu Gly Glu Trp Glu Arg Tyr Ile Gln Trp Val Glu Glu Asn Phe Pro
    25                  30                  35

gag aat aaa gaa tac ttg ata act tta cta gaa cat tta atg aag gaa      257
Glu Asn Lys Glu Tyr Leu Ile Thr Leu Leu Glu His Leu Met Lys Glu
40                  45                  50                  55

ttt tta gat aag aag aaa tac cac aat gac cca aga ttc atc agt tat      305
Phe Leu Asp Lys Lys Lys Tyr His Asn Asp Pro Arg Phe Ile Ser Tyr
                60                  65                  70

tgt tta aaa ttt gct gag tac aac agt gac ctc cat caa ttt ttt gag      353
Cys Leu Lys Phe Ala Glu Tyr Asn Ser Asp Leu His Gln Phe Phe Glu
            75                  80                  85

ttt ctg tac aac cat ggg att gga acc ctg tca tcc cct ctg tac att      401
Phe Leu Tyr Asn His Gly Ile Gly Thr Leu Ser Ser Pro Leu Tyr Ile
        90                  95                  100

gcc tgg gcg ggg cat ctg gaa gcc caa gga gag ctg cag cat gcc agt      449
Ala Trp Ala Gly His Leu Glu Ala Gln Gly Glu Leu Gln His Ala Ser
    105                 110                 115

gct gtc ctt cag aga gga att caa aac cag gct gaa ccc aga gag ttc      497
Ala Val Leu Gln Arg Gly Ile Gln Asn Gln Ala Glu Pro Arg Glu Phe
120                 125                 130                 135

ctg caa caa caa tac agg tta ttt cag aca cgc ctc act gaa acc cat      545
Leu Gln Gln Gln Tyr Arg Leu Phe Gln Thr Arg Leu Thr Glu Thr His
                140                 145                 150

ttg cca gct caa gct aga acc tca gaa cct ctg cat aat gtt cag gtt      593
Leu Pro Ala Gln Ala Arg Thr Ser Glu Pro Leu His Asn Val Gln Val
            155                 160                 165

tta aat caa atg ata aca tca aaa tca aat cca gga aat aac atg gcc      641
Leu Asn Gln Met Ile Thr Ser Lys Ser Asn Pro Gly Asn Asn Met Ala
        170                 175                 180

tgc att tct aag aat cag ggt tca gag ctt tct gga gtg ata tct tca      689
Cys Ile Ser Lys Asn Gln Gly Ser Glu Leu Ser Gly Val Ile Ser Ser

185                 190                 195

gct tgt gat aaa gag tca aat atg gaa cga aga gtg atc acg att tct       737
Ala Cys Asp Lys Glu Ser Asn Met Glu Arg Arg Val Ile Thr Ile Ser
200                 205                 210                 215

aaa tca gaa tat tct gtg cac tca tct ttg gca tcc aaa gtt gat gtt       785
Lys Ser Glu Tyr Ser Val His Ser Ser Leu Ala Ser Lys Val Asp Val
                220                 225                 230

gag cag gtt gtt atg tat tgc aag gag aag ctt att cgt ggg gaa tca       833
Glu Gln Val Val Met Tyr Cys Lys Glu Lys Leu Ile Arg Gly Glu Ser
            235                 240                 245

gaa ttt tcc ttt gaa gaa ttg aga gcc cag aaa tac aat caa cgg aga       881
Glu Phe Ser Phe Glu Glu Leu Arg Ala Gln Lys Tyr Asn Gln Arg Arg
        250                 255                 260

aag cat gag caa tgg gta aat gaa gac aga cat tat atg aaa agg aaa       929
Lys His Glu Gln Trp Val Asn Glu Asp Arg His Tyr Met Lys Arg Lys
    265                 270                 275

gaa gca aat gct ttt gaa gaa cag cta tta aaa cag aaa atg gat gaa       977
Glu Ala Asn Ala Phe Glu Glu Gln Leu Leu Lys Gln Lys Met Asp Glu
280                 285                 290                 295

ctt cat aag aag ttg cat cag gtg gtg gag aca tcc cat gag gat ctg      1025
Leu His Lys Lys Leu His Gln Val Val Glu Thr Ser His Glu Asp Leu
                300                 305                 310

ccc gct tcc cag gaa agg tcc gag gtt aat cca gca cgt atg ggg cca      1073
Pro Ala Ser Gln Glu Arg Ser Glu Val Asn Pro Ala Arg Met Gly Pro
            315                 320                 325

agt gta ggc tcc cag cag gaa ctg aga gcg cca tgt ctt cca gta acc      1121
Ser Val Gly Ser Gln Gln Glu Leu Arg Ala Pro Cys Leu Pro Val Thr
        330                 335                 340

tat cag cag aca cca gtg aac atg gaa aag aac cca aga gag gca cct      1169
Tyr Gln Gln Thr Pro Val Asn Met Glu Lys Asn Pro Arg Glu Ala Pro
    345                 350                 355

cct gtt gtt cct cct ttg gca aat gct att tct gca gct ttg gtg tcc      1217
Pro Val Val Pro Pro Leu Ala Asn Ala Ile Ser Ala Ala Leu Val Ser
360                 365                 370                 375

cca gcc acc agc cag agc att gct cct cct gtt cct ttg aaa gcc cag      1265
Pro Ala Thr Ser Gln Ser Ile Ala Pro Pro Val Pro Leu Lys Ala Gln
                380                 385                 390

aca gta aca gac tcc atg ttt gca gtg gcc agc aaa gat gct gga tgt      1313
Thr Val Thr Asp Ser Met Phe Ala Val Ala Ser Lys Asp Ala Gly Cys
            395                 400                 405

gtg aat aag agt act cat gaa ttc aag cca cag agt gga gca gag atc      1361
Val Asn Lys Ser Thr His Glu Phe Lys Pro Gln Ser Gly Ala Glu Ile
        410                 415                 420

aaa gaa ggg tgt gaa aca cat aag gtt gcc aac aca agt tct ttt cac      1409
Lys Glu Gly Cys Glu Thr His Lys Val Ala Asn Thr Ser Ser Phe His
    425                 430                 435

aca act cca aac aca tca ctg gga atg gtt cag gca acg cca tcc aaa      1457

Thr Thr Pro Asn Thr Ser Leu Gly Met Val Gln Ala Thr Pro Ser Lys
440                 445                 450                 455

gtg cag cca tca ccc acc gtg cac aca aaa gaa gca tta ggt ttc atc      1505
Val Gln Pro Ser Pro Thr Val His Thr Lys Glu Ala Leu Gly Phe Ile
                460                 465                 470

atg aat atg ttt cag gct cct aca ctt cct gat att tct gat gac aaa      1553
Met Asn Met Phe Gln Ala Pro Thr Leu Pro Asp Ile Ser Asp Asp Lys
            475                 480                 485

gat gaa tgg caa tct cta gat caa aat gaa gat gca ttt gaa gcc cag      1601
Asp Glu Trp Gln Ser Leu Asp Gln Asn Glu Asp Ala Phe Glu Ala Gln
        490                 495                 500

ttt caa aaa aat gta agg tca tct ggg gct tgg gga gtc aat aag atc      1649
Phe Gln Lys Asn Val Arg Ser Ser Gly Ala Trp Gly Val Asn Lys Ile
    505                 510                 515

atc tct tct ttg tca tct gct ttt cat gtg ttt gaa gat gga aac aaa      1697
Ile Ser Ser Leu Ser Ser Ala Phe His Val Phe Glu Asp Gly Asn Lys
520                 525                 530                 535

gaa aat tat gga tta cca cag cct aaa aat aaa ccc aca gga gcc agg      1745
Glu Asn Tyr Gly Leu Pro Gln Pro Lys Asn Lys Pro Thr Gly Ala Arg
                540                 545                 550

acc ttt gga gaa cgc tct gtc agc aga ctt cct tca aaa cca aag gag      1793
Thr Phe Gly Glu Arg Ser Val Ser Arg Leu Pro Ser Lys Pro Lys Glu
            555                 560                 565

gaa gtg cct cat gct gaa gag ttt ttg gat gac tca act gta tgg ggt      1841
Glu Val Pro His Ala Glu Glu Phe Leu Asp Asp Ser Thr Val Trp Gly
        570                 575                 580

att cgc tgc aac aaa acc ctg gca ccc agt cct aag agc cca gga gac      1889
Ile Arg Cys Asn Lys Thr Leu Ala Pro Ser Pro Lys Ser Pro Gly Asp
    585                 590                 595

ttc aca tct gct gca caa ctt gcg tct aca cca ttc cac aag ctt cca      1937
Phe Thr Ser Ala Ala Gln Leu Ala Ser Thr Pro Phe His Lys Leu Pro
600                 605                 610                 615

gtg gag tca gtg cac att tta gaa gat aaa gaa aat gtg gta gca aaa      1985
Val Glu Ser Val His Ile Leu Glu Asp Lys Glu Asn Val Val Ala Lys
                620                 625                 630

cag tgt acc cag gcg act ttg gat tct tgt gag gaa aac atg gtg gtg      2033
Gln Cys Thr Gln Ala Thr Leu Asp Ser Cys Glu Glu Asn Met Val Val
            635                 640                 645

cct tca agg gat gga aaa ttc agt cca att caa gag aaa agc cca aaa      2081
Pro Ser Arg Asp Gly Lys Phe Ser Pro Ile Gln Glu Lys Ser Pro Lys
        650                 655                 660

cag gcc ttg tcg tct cac atg tat tca gca tcc tta ctt cgt ctg agc      2129
Gln Ala Leu Ser Ser His Met Tyr Ser Ala Ser Leu Leu Arg Leu Ser
    665                 670                 675

cag cct gct gca ggt ggg gta ctt acc tgt gag gca gag ttg ggc gtt      2177
Gln Pro Ala Ala Gly Gly Val Leu Thr Cys Glu Ala Glu Leu Gly Val
680                 685                 690                 695

gag gct tgc aga ctc aca gac act gac gct gcc att gca gaa gat cca     2225
Glu Ala Cys Arg Leu Thr Asp Thr Asp Ala Ala Ile Ala Glu Asp Pro
                700                 705                 710

cca gat gct att gct ggg ctc caa gca gaa tgg atg cag atg agt tca     2273
Pro Asp Ala Ile Ala Gly Leu Gln Ala Glu Trp Met Gln Met Ser Ser
            715                 720                 725

ctt ggg act gtt gat gct cca aac ttc att gtt ggg aac cca tgg gat     2321
Leu Gly Thr Val Asp Ala Pro Asn Phe Ile Val Gly Asn Pro Trp Asp
        730                 735                 740

gat aag ctg att ttc aaa ctt tta tct ggg ctt tct aaa cca gtg agt     2369
Asp Lys Leu Ile Phe Lys Leu Leu Ser Gly Leu Ser Lys Pro Val Ser
    745                 750                 755

tcc tat cca aat act ttt gaa tgg caa tgt aaa ctt cca gcc atc aag     2417
Ser Tyr Pro Asn Thr Phe Glu Trp Gln Cys Lys Leu Pro Ala Ile Lys
760                 765                 770                 775

ccc aag act gaa ttt caa ttg ggt tct aag ctg gtc tat gtc cat cac     2465
Pro Lys Thr Glu Phe Gln Leu Gly Ser Lys Leu Val Tyr Val His His
                780                 785                 790

ctt ctt gga gaa gga gcc ttt gcc cag gtg tac gaa gct acc cag gga     2513
Leu Leu Gly Glu Gly Ala Phe Ala Gln Val Tyr Glu Ala Thr Gln Gly
            795                 800                 805

gat ctg aat gat gct aaa aat aaa cag aaa ttt gtt tta aag gtc caa     2561
Asp Leu Asn Asp Ala Lys Asn Lys Gln Lys Phe Val Leu Lys Val Gln
        810                 815                 820

aag cct gcc aac ccc tgg gaa ttc tac att ggg acc cag ttg atg gaa     2609
Lys Pro Ala Asn Pro Trp Glu Phe Tyr Ile Gly Thr Gln Leu Met Glu
    825                 830                 835

aga cta aag cca tct atg cag cac atg ttt atg aag ttc tat tct gcc     2657
Arg Leu Lys Pro Ser Met Gln His Met Phe Met Lys Phe Tyr Ser Ala
840                 845                 850                 855

cac tta ttc cag aat ggc agt gta tta gta gga gag ctc tac agc tat     2705
His Leu Phe Gln Asn Gly Ser Val Leu Val Gly Glu Leu Tyr Ser Tyr
                860                 865                 870

gga aca tta tta aat gcc att aac ctc tat aaa aat acc cct gaa aaa     2753
Gly Thr Leu Leu Asn Ala Ile Asn Leu Tyr Lys Asn Thr Pro Glu Lys
            875                 880                 885

gtg atg cct caa ggt ctt gtc atc tct ttt gct atg aga atg ctt tac     2801
Val Met Pro Gln Gly Leu Val Ile Ser Phe Ala Met Arg Met Leu Tyr
        890                 895                 900

atg att gag caa gtg cat gac tgt gaa atc att cat gga gac att aaa     2849
Met Ile Glu Gln Val His Asp Cys Glu Ile Ile His Gly Asp Ile Lys
    905                 910                 915

cca gac aat ttc ata ctt gga aac gga ttt ttg gaa cag gat gat gaa     2897
Pro Asp Asn Phe Ile Leu Gly Asn Gly Phe Leu Glu Gln Asp Asp Glu
920                 925                 930                 935

gat gat tta tct gct ggc ttg gca ctg att gac ctg ggt cag agt ata     2945
Asp Asp Leu Ser Ala Gly Leu Ala Leu Ile Asp Leu Gly Gln Ser Ile
                940                 945                 950

gat atg aaa ctt ttt cca aaa gga act ata ttc aca gca aag tgt gaa     2993
Asp Met Lys Leu Phe Pro Lys Gly Thr Ile Phe Thr Ala Lys Cys Glu
            955                 960                 965

aca tct ggt ttt cag tgt gtt gag atg ctc agc aac aaa cca tgg aac     3041
Thr Ser Gly Phe Gln Cys Val Glu Met Leu Ser Asn Lys Pro Trp Asn
        970                 975                 980

tac cag atc gat tac ttt ggg gtt gct gca aca gta tat tgc atg ctc     3089
Tyr Gln Ile Asp Tyr Phe Gly Val Ala Ala Thr Val Tyr Cys Met Leu
    985                 990                 995

ttt  ggc act tac atg aaa  gtg aaa aat gaa gga  gga gag tgt aag     3134
Phe  Gly Thr Tyr Met Lys  Val Lys Asn Glu Gly  Gly Glu Cys Lys
1000                 1005                 1010

cct  gaa ggt ctt ttt aga  agg ctt cct cat ttg  gat atg tgg aat     3179
Pro  Glu Gly Leu Phe Arg  Arg Leu Pro His Leu  Asp Met Trp Asn
1015                 1020                 1025

gaa  ttt ttt cat gtt atg  ttg aat att cca gat  tgt cat cat ctt     3224
Glu  Phe Phe His Val Met  Leu Asn Ile Pro Asp  Cys His His Leu
1030                 1035                 1040

cca  tct ttg gat ttg tta  agg caa aag ctg aag  aaa gta ttt caa     3269
Pro  Ser Leu Asp Leu Leu  Arg Gln Lys Leu Lys  Lys Val Phe Gln
1045                 1050                 1055

caa  cac tat act aac aag  att agg gcc cta cgt  aat agg cta att     3314
Gln  His Tyr Thr Asn Lys  Ile Arg Ala Leu Arg  Asn Arg Leu Ile
1060                 1065                 1070

gta  ctg ctc tta gaa tgt  aag cgt tca cga aaa  taa aatttggata     3360
Val  Leu Leu Leu Glu Cys  Lys Arg Ser Arg Lys
1075                 1080                 1085

tagacagtcc ttaaaaatca cactgtaaat atgaatctgc tcactttaaa cctgtttttt     3420

tttcatttat tgtttatgta aatgtttgtt aaaaataaat cccatggaat atttccatgt     3480

aaaaaa.                                                                3486

<210>  8
<211>  1085
<212>  PRT
<213>  Homo sapiens

<400>  8

Met Asp Thr Pro Glu Asn Val Leu Gln Met Leu Glu Ala His Met Gln
1               5                   10                  15

Ser Tyr Lys Gly Asn Asp Pro Leu Gly Glu Trp Glu Arg Tyr Ile Gln
            20                  25                  30

Trp Val Glu Glu Asn Phe Pro Glu Asn Lys Glu Tyr Leu Ile Thr Leu
        35                  40                  45

Leu Glu His Leu Met Lys Glu Phe Leu Asp Lys Lys Lys Tyr His Asn
    50                  55                  60

Asp Pro Arg Phe Ile Ser Tyr Cys Leu Lys Phe Ala Glu Tyr Asn Ser
65                  70                  75                  80

Asp Leu His Gln Phe Phe Glu Phe Leu Tyr Asn His Gly Ile Gly Thr
                85                  90                  95

Leu Ser Ser Pro Leu Tyr Ile Ala Trp Ala Gly His Leu Glu Ala Gln
            100                 105                 110

Gly Glu Leu Gln His Ala Ser Ala Val Leu Gln Arg Gly Ile Gln Asn
        115                 120                 125

Gln Ala Glu Pro Arg Glu Phe Leu Gln Gln Gln Tyr Arg Leu Phe Gln
    130                 135                 140

Thr Arg Leu Thr Glu Thr His Leu Pro Ala Gln Ala Arg Thr Ser Glu
145                 150                 155                 160

Pro Leu His Asn Val Gln Val Leu Asn Gln Met Ile Thr Ser Lys Ser
                165                 170                 175

Asn Pro Gly Asn Asn Met Ala Cys Ile Ser Lys Asn Gln Gly Ser Glu
            180                 185                 190

Leu Ser Gly Val Ile Ser Ser Ala Cys Asp Lys Glu Ser Asn Met Glu
        195                 200                 205

Arg Arg Val Ile Thr Ile Ser Lys Ser Glu Tyr Ser Val His Ser Ser
    210                 215                 220

Leu Ala Ser Lys Val Asp Val Glu Gln Val Val Met Tyr Cys Lys Glu
225                 230                 235                 240

Lys Leu Ile Arg Gly Glu Ser Glu Phe Ser Phe Glu Glu Leu Arg Ala
                245                 250                 255

Gln Lys Tyr Asn Gln Arg Arg Lys His Glu Gln Trp Val Asn Glu Asp
            260                 265                 270

Arg His Tyr Met Lys Arg Lys Glu Ala Asn Ala Phe Glu Glu Gln Leu
        275                 280                 285

Leu Lys Gln Lys Met Asp Glu Leu His Lys Lys Leu His Gln Val Val
    290                 295                 300

Glu Thr Ser His Glu Asp Leu Pro Ala Ser Gln Glu Arg Ser Glu Val
305                 310                 315                 320

Asn Pro Ala Arg Met Gly Pro Ser Val Gly Ser Gln Gln Glu Leu Arg
                325                 330                 335

Ala Pro Cys Leu Pro Val Thr Tyr Gln Gln Thr Pro Val Asn Met Glu
            340                 345                 350

Lys Asn Pro Arg Glu Ala Pro Pro Val Val Pro Pro Leu Ala Asn Ala
        355                 360                 365

Ile Ser Ala Ala Leu Val Ser Pro Ala Thr Ser Gln Ser Ile Ala Pro
    370                 375                 380

Pro Val Pro Leu Lys Ala Gln Thr Val Thr Asp Ser Met Phe Ala Val
385                 390                 395                 400

Ala Ser Lys Asp Ala Gly Cys Val Asn Lys Ser Thr His Glu Phe Lys
                405                 410                 415

Pro Gln Ser Gly Ala Glu Ile Lys Glu Gly Cys Glu Thr His Lys Val
            420                 425                 430

Ala Asn Thr Ser Ser Phe His Thr Thr Pro Asn Thr Ser Leu Gly Met
        435                 440                 445

Val Gln Ala Thr Pro Ser Lys Val Gln Pro Ser Pro Thr Val His Thr
    450                 455                 460

Lys Glu Ala Leu Gly Phe Ile Met Asn Met Phe Gln Ala Pro Thr Leu
465                 470                 475                 480

Pro Asp Ile Ser Asp Asp Lys Asp Glu Trp Gln Ser Leu Asp Gln Asn
                485                 490                 495

Glu Asp Ala Phe Glu Ala Gln Phe Gln Lys Asn Val Arg Ser Ser Gly
            500                 505                 510

Ala Trp Gly Val Asn Lys Ile Ile Ser Ser Leu Ser Ser Ala Phe His
        515                 520                 525

Val Phe Glu Asp Gly Asn Lys Glu Asn Tyr Gly Leu Pro Gln Pro Lys
    530                 535                 540

Asn Lys Pro Thr Gly Ala Arg Thr Phe Gly Glu Arg Ser Val Ser Arg
545                 550                 555                 560

Leu Pro Ser Lys Pro Lys Glu Glu Val Pro His Ala Glu Glu Phe Leu
                565                 570                 575

Asp Asp Ser Thr Val Trp Gly Ile Arg Cys Asn Lys Thr Leu Ala Pro
            580                 585                 590

Ser Pro Lys Ser Pro Gly Asp Phe Thr Ser Ala Ala Gln Leu Ala Ser
        595                 600                 605

Thr Pro Phe His Lys Leu Pro Val Glu Ser Val His Ile Leu Glu Asp
    610                 615                 620

Lys Glu Asn Val Val Ala Lys Gln Cys Thr Gln Ala Thr Leu Asp Ser
625                 630                 635                 640

Cys Glu Glu Asn Met Val Val Pro Ser Arg Asp Gly Lys Phe Ser Pro
                645                 650                 655

Ile Gln Glu Lys Ser Pro Lys Gln Ala Leu Ser Ser His Met Tyr Ser
            660                 665                 670

Ala Ser Leu Leu Arg Leu Ser Gln Pro Ala Ala Gly Gly Val Leu Thr
        675                 680                 685

Cys Glu Ala Glu Leu Gly Val Glu Ala Cys Arg Leu Thr Asp Thr Asp
    690                 695                 700

Ala Ala Ile Ala Glu Asp Pro Pro Asp Ala Ile Ala Gly Leu Gln Ala
705                 710                 715                 720

Glu Trp Met Gln Met Ser Ser Leu Gly Thr Val Asp Ala Pro Asn Phe
                725                 730                 735

Ile Val Gly Asn Pro Trp Asp Asp Lys Leu Ile Phe Lys Leu Leu Ser
            740                 745                 750

Gly Leu Ser Lys Pro Val Ser Ser Tyr Pro Asn Thr Phe Glu Trp Gln
        755                 760                 765

Cys Lys Leu Pro Ala Ile Lys Pro Lys Thr Glu Phe Gln Leu Gly Ser
    770                 775                 780

Lys Leu Val Tyr Val His His Leu Leu Gly Glu Gly Ala Phe Ala Gln
785                 790                 795                 800

Val Tyr Glu Ala Thr Gln Gly Asp Leu Asn Asp Ala Lys Asn Lys Gln

                805                 810                 815

Lys Phe Val Leu Lys Val Gln Lys Pro Ala Asn Pro Trp Glu Phe Tyr
            820                 825                 830

Ile Gly Thr Gln Leu Met Glu Arg Leu Lys Pro Ser Met Gln His Met
        835                 840                 845

Phe Met Lys Phe Tyr Ser Ala His Leu Phe Gln Asn Gly Ser Val Leu
    850                 855                 860

Val Gly Glu Leu Tyr Ser Tyr Gly Thr Leu Leu Asn Ala Ile Asn Leu
865                 870                 875                 880

Tyr Lys Asn Thr Pro Glu Lys Val Met Pro Gln Gly Leu Val Ile Ser
                885                 890                 895

Phe Ala Met Arg Met Leu Tyr Met Ile Glu Gln Val His Asp Cys Glu
            900                 905                 910

Ile Ile His Gly Asp Ile Lys Pro Asp Asn Phe Ile Leu Gly Asn Gly
        915                 920                 925

Phe Leu Glu Gln Asp Asp Glu Asp Asp Leu Ser Ala Gly Leu Ala Leu
    930                 935                 940

Ile Asp Leu Gly Gln Ser Ile Asp Met Lys Leu Phe Pro Lys Gly Thr
945                 950                 955                 960

Ile Phe Thr Ala Lys Cys Glu Thr Ser Gly Phe Gln Cys Val Glu Met
                965                 970                 975

Leu Ser Asn Lys Pro Trp Asn Tyr Gln Ile Asp Tyr Phe Gly Val Ala
            980                 985                 990

Ala Thr Val Tyr Cys Met Leu Phe Gly Thr Tyr Met Lys Val Lys Asn
        995                 1000                1005

Glu Gly Gly Glu Cys Lys Pro Glu Gly Leu Phe Arg Arg Leu Pro
    1010                1015                1020

His Leu Asp Met Trp Asn Glu Phe Phe His Val Met Leu Asn Ile
    1025                1030                1035

Pro Asp Cys His His Leu Pro Ser Leu Asp Leu Leu Arg Gln Lys
    1040                1045                1050

Leu Lys Lys Val Phe Gln Gln His Tyr Thr Asn Lys Ile Arg Ala
    1055                1060                1065

Leu Arg Asn Arg Leu Ile Val Leu Leu Leu Glu Cys Lys Arg Ser
    1070                1075                1080

Arg Lys
    1085

<210> 9
<211> 2635
<212> DNA
<213> Homo sapiens

<220>
<221> CDS
<222> (285)..(1679)
<223>

<400> 9
ggccgacgcg agcgccgcgc ttcgcttcag ctgctagctg gcccaaggga ggcgaccgcg 60

gagggtggcg aggggcggcc aggacccgca gccccggggc cgggccggtc cggaccgcca 120

gggagggcag gtcagtgggc agatcgcgtc cgcgggattc aatctctgcc cgctctgata 180

acagtccttt tccctggcgc tcacttcgtg cctggcaccc ggctgggcgc ctcaagaccg 240

ttgtctcttc gatcgcttct ttggacttgg cgaccatttc agag atg tct tcc aga 296
Met Ser Ser Arg
1

agt acc aaa gat tta att aaa agt aag tgg gga tcg aag cct agt aac 344
Ser Thr Lys Asp Leu Ile Lys Ser Lys Trp Gly Ser Lys Pro Ser Asn
5                   10                  15                  20

tcc aaa tcc gaa act aca tta gaa aaa tta aag gga gaa att gca cac 392
Ser Lys Ser Glu Thr Thr Leu Glu Lys Leu Lys Gly Glu Ile Ala His
                25                  30                  35

tta aag aca tca gtg gat gaa atc aca agt ggg aaa gga aag ctg act 440
Leu Lys Thr Ser Val Asp Glu Ile Thr Ser Gly Lys Gly Lys Leu Thr
            40                  45                  50

gat aaa gag aga cac aga ctt ttg gag aaa att cga gtc ctt gag gct 488
Asp Lys Glu Arg His Arg Leu Leu Glu Lys Ile Arg Val Leu Glu Ala
        55                  60                  65

gag aag gag aag aat gct tat caa ctc aca gag aag gac aaa gaa ata 536
Glu Lys Glu Lys Asn Ala Tyr Gln Leu Thr Glu Lys Asp Lys Glu Ile
    70                  75                  80

cag cga ctg aga gac caa ctg aag gcc aga tat agt act acc gca ttg 584
Gln Arg Leu Arg Asp Gln Leu Lys Ala Arg Tyr Ser Thr Thr Ala Leu
85                  90                  95                  100

ctt gaa cag ctg gaa gag aca acg aga gaa gga gaa agg agg gag cag 632
Leu Glu Gln Leu Glu Glu Thr Thr Arg Glu Gly Glu Arg Arg Glu Gln
                105                 110                 115

gtg ttg aaa gcc tta tct gaa gag aaa gac gta ttg aaa caa cag ttg     680
Val Leu Lys Ala Leu Ser Glu Glu Lys Asp Val Leu Lys Gln Gln Leu
            120                 125                 130

tct gct gca acc tca cga att gct gaa ctt gaa agc aaa acc aat aca     728
Ser Ala Ala Thr Ser Arg Ile Ala Glu Leu Glu Ser Lys Thr Asn Thr
        135                 140                 145

ctc cgt tta tca cag act gtg gct cca aac tgc ttc aac tca tca ata     776
Leu Arg Leu Ser Gln Thr Val Ala Pro Asn Cys Phe Asn Ser Ser Ile
    150                 155                 160

aat aat att cat gaa atg gaa ata cag ctg aaa gat gct ctg gag aaa     824
Asn Asn Ile His Glu Met Glu Ile Gln Leu Lys Asp Ala Leu Glu Lys
165                 170                 175                 180

aat cag cag tgg ctc gtg tat gat cag cag cgg gaa gtc tat gta aaa     872
Asn Gln Gln Trp Leu Val Tyr Asp Gln Gln Arg Glu Val Tyr Val Lys
                185                 190                 195

gga ctt tta gca aag atc ttt gag ttg gaa aag aaa acg gaa aca gct     920
Gly Leu Leu Ala Lys Ile Phe Glu Leu Glu Lys Lys Thr Glu Thr Ala
            200                 205                 210

gct cat tca ctc cca cag cag aca aaa aag cct gaa tca gaa ggt tat     968
Ala His Ser Leu Pro Gln Gln Thr Lys Lys Pro Glu Ser Glu Gly Tyr
        215                 220                 225

ctt caa gaa gag aag cag aaa tgt tac aac gat ctc ttg gca agt gca     1016
Leu Gln Glu Glu Lys Gln Lys Cys Tyr Asn Asp Leu Leu Ala Ser Ala
    230                 235                 240

aaa aaa gat ctt gag gtt gaa cga caa acc ata act cag ctg agt ttt     1064
Lys Lys Asp Leu Glu Val Glu Arg Gln Thr Ile Thr Gln Leu Ser Phe
245                 250                 255                 260

gaa ctg agt gaa ttt cga aga aaa tat gaa gaa acc caa aaa gaa gtt     1112
Glu Leu Ser Glu Phe Arg Arg Lys Tyr Glu Glu Thr Gln Lys Glu Val
                265                 270                 275

cac aat tta aat cag ctg ttg tat tca caa aga agg gca gat gtg caa     1160
His Asn Leu Asn Gln Leu Leu Tyr Ser Gln Arg Arg Ala Asp Val Gln
            280                 285                 290

cat ctg gaa gat gat agg cat aaa aca gag aag ata caa aaa ctc agg     1208
His Leu Glu Asp Asp Arg His Lys Thr Glu Lys Ile Gln Lys Leu Arg
        295                 300                 305

gaa gag aat gat att gct agg gga aaa ctt gaa gaa gag aag aag aga     1256
Glu Glu Asn Asp Ile Ala Arg Gly Lys Leu Glu Glu Glu Lys Lys Arg
    310                 315                 320

tcc gaa gag ctc tta tct cag gtc cag ttt ctt tac aca tct ctg cta     1304
Ser Glu Glu Leu Leu Ser Gln Val Gln Phe Leu Tyr Thr Ser Leu Leu
325                 330                 335                 340

aag cag caa gaa gaa caa aca agg gta gct ctg ttg gaa caa cag atg     1352
Lys Gln Gln Glu Glu Gln Thr Arg Val Ala Leu Leu Glu Gln Gln Met
                345                 350                 355

cag gca tgt act tta gac ttt gaa aat gaa aaa ctc gac cgt caa cat     1400
Gln Ala Cys Thr Leu Asp Phe Glu Asn Glu Lys Leu Asp Arg Gln His
            360                 365                 370

gtg cag cat caa ttg ctt gta att ctt aag gag ctc cga aaa gca aga     1448
Val Gln His Gln Leu Leu Val Ile Leu Lys Glu Leu Arg Lys Ala Arg
        375                 380                 385

aat caa ata aca cag ttg gaa tcc ttg aaa cag ctt cat gag ttt gcc     1496
Asn Gln Ile Thr Gln Leu Glu Ser Leu Lys Gln Leu His Glu Phe Ala
    390                 395                 400

atc aca gag cca tta gtc act ttc caa gga gag act gaa aac aga gaa     1544
Ile Thr Glu Pro Leu Val Thr Phe Gln Gly Glu Thr Glu Asn Arg Glu
405                 410                 415                 420

aaa gtt gcc gcc tca cca aaa agt ccc act gct gca ctc aat gaa agc     1592
Lys Val Ala Ala Ser Pro Lys Ser Pro Thr Ala Ala Leu Asn Glu Ser
                425                 430                 435

ctg gtg gaa tgt ccc aag tgc aat ata cag tat cca gcc act gag cat     1640
Leu Val Glu Cys Pro Lys Cys Asn Ile Gln Tyr Pro Ala Thr Glu His
            440                 445                 450

cgc gat ctg ctt gtc cat gtg gaa tac tgt tca aag tag caaaataagt     1689
Arg Asp Leu Leu Val His Val Glu Tyr Cys Ser Lys
        455                 460

atttgttttg atattaaaag attcaatact gtattttctg ttagcttgtg ggcattttga     1749

attatatatt tcacattttg cataaaactg cctatctacc tttgacactc cagcatgcta     1809

gtgaatcatg tatcttttag gctgctgtgc atttctcttg gcagtgatac ctccctgaca     1869

tggttcatca tcaggctgca atgacagaat gtggtgagca gcgtctactg agactactaa     1929

cattttgcac tgtcaaaata cttggtgagg aaaagatagc tcaggttatt gctaatgggt     1989

taatgcacca gcaagcaaaa tattttatgt tttgggggtt tgaaaaatca aagataatta     2049

accaaggatc ttaactgtgt tcgcattttt tatccaagca cttagaaaac ctacaatcct     2109

aattttgatg tccattgtta agaggtggtg atagatacta tttttttttt catattgtat     2169

agcggttatt agaaaagttg gggattttct tgatctttat tgctgcttac cattgaaact     2229

taacccagct gtgttcccca actctgttct gcgcacgaaa cagtatctgt ttgaggcata     2289

atcttaagtg gccacacaca atgttttctc ttatgttatc tggcagtaac tgtaacttga     2349

attacattag cacattctgc ttagctaaaa ttgttaaaat aaactttaat aaacccatgt     2409

agccctctca tttgattgac agtattttag ttatttttgg cattcttaaa gctgggcaat     2469

gtaatgatca gatctttgtt tgtctgaaca ggtattttta tacatgcttt ttgtaaacca     2529

aaaactttta aatttcttca ggttttctaa catgcttacc actgggctac tgtaaatgag     2589

aaaagaataa aattatttaa tgttttaaaa aaaaaaaaaa aaaaaa     2635

<210>  10
<211>  464
<212>  PRT
<213>  Homo sapiens

<400> 10

Met Ser Ser Arg Ser Thr Lys Asp Leu Ile Lys Ser Lys Trp Gly Ser
1               5                   10                  15

Lys Pro Ser Asn Ser Lys Ser Glu Thr Thr Leu Glu Lys Leu Lys Gly
            20                  25                  30

Glu Ile Ala His Leu Lys Thr Ser Val Asp Glu Ile Thr Ser Gly Lys
        35                  40                  45

Gly Lys Leu Thr Asp Lys Glu Arg His Arg Leu Leu Glu Lys Ile Arg
    50                  55                  60

Val Leu Glu Ala Glu Lys Glu Lys Asn Ala Tyr Gln Leu Thr Glu Lys
65                  70                  75                  80

Asp Lys Glu Ile Gln Arg Leu Arg Asp Gln Leu Lys Ala Arg Tyr Ser
                85                  90                  95

Thr Thr Ala Leu Leu Glu Gln Leu Glu Glu Thr Thr Arg Glu Gly Glu
            100                 105                 110

Arg Arg Glu Gln Val Leu Lys Ala Leu Ser Glu Glu Lys Asp Val Leu
        115                 120                 125

Lys Gln Gln Leu Ser Ala Ala Thr Ser Arg Ile Ala Glu Leu Glu Ser
    130                 135                 140

Lys Thr Asn Thr Leu Arg Leu Ser Gln Thr Val Ala Pro Asn Cys Phe
145                 150                 155                 160

Asn Ser Ser Ile Asn Asn Ile His Glu Met Glu Ile Gln Leu Lys Asp
                165                 170                 175

Ala Leu Glu Lys Asn Gln Gln Trp Leu Val Tyr Asp Gln Gln Arg Glu
            180                 185                 190

Val Tyr Val Lys Gly Leu Leu Ala Lys Ile Phe Glu Leu Glu Lys Lys
        195                 200                 205

Thr Glu Thr Ala Ala His Ser Leu Pro Gln Gln Thr Lys Lys Pro Glu
    210                 215                 220

Ser Glu Gly Tyr Leu Gln Glu Glu Lys Gln Lys Cys Tyr Asn Asp Leu
225                 230                 235                 240

Leu Ala Ser Ala Lys Lys Asp Leu Glu Val Glu Arg Gln Thr Ile Thr

245                 250                 255

Gln Leu Ser Phe Glu Leu Ser Glu Phe Arg Arg Lys Tyr Glu Glu Thr
            260                 265                 270

Gln Lys Glu Val His Asn Leu Asn Gln Leu Leu Tyr Ser Gln Arg Arg
        275                 280                 285

Ala Asp Val Gln His Leu Glu Asp Asp Arg His Lys Thr Glu Lys Ile
    290                 295                 300

Gln Lys Leu Arg Glu Glu Asn Asp Ile Ala Arg Gly Lys Leu Glu Glu
305                 310                 315                 320

Glu Lys Lys Arg Ser Glu Glu Leu Leu Ser Gln Val Gln Phe Leu Tyr
                325                 330                 335

Thr Ser Leu Leu Lys Gln Gln Glu Glu Gln Thr Arg Val Ala Leu Leu
            340                 345                 350

Glu Gln Gln Met Gln Ala Cys Thr Leu Asp Phe Glu Asn Glu Lys Leu
        355                 360                 365

Asp Arg Gln His Val Gln His Gln Leu Leu Val Ile Leu Lys Glu Leu
    370                 375                 380

Arg Lys Ala Arg Asn Gln Ile Thr Gln Leu Glu Ser Leu Lys Gln Leu
385                 390                 395                 400

His Glu Phe Ala Ile Thr Glu Pro Leu Val Thr Phe Gln Gly Glu Thr
                405                 410                 415

Glu Asn Arg Glu Lys Val Ala Ala Ser Pro Lys Ser Pro Thr Ala Ala
            420                 425                 430

Leu Asn Glu Ser Leu Val Glu Cys Pro Lys Cys Asn Ile Gln Tyr Pro
        435                 440                 445

Ala Thr Glu His Arg Asp Leu Leu Val His Val Glu Tyr Cys Ser Lys
    450                 455                 460

<210>  11
<211>  2765
<212>  DNA
<213>  Homo sapiens

<220>
<221>  CDS
<222>  (329)..(1048)

<223>

<400> 11
cggagtctgg ccgcagtcgc ggcagtggtg gcttcccatc cccaaaaggc gccctccgac 60

tccttgcgcc gcactgctcg ccgggccagt ccggaaacgg gtcgtggagc tccgcaccac 120

tcccgctggt tcccgaaggc agatcccttc tcccgagagt tgcgagaaac tttcccttgt 180

ccccgacgct gcagcggctc gggtaccgtg gcagccgcag gtttctgaac cccgggccac 240

gctccccgcg cctcggcttc gcgctcgtgt agatcgttcc ctctctggtt gcacgctggg 300

gatcccggac ctcgattctg cgggcgag atg ccc ctg gga cac atc atg agg 352
Met Pro Leu Gly His Ile Met Arg
1               5

ctg gac ctg gag aaa att gcc ctg gag tac atc gtg ccc tgt ctg cac 400
Leu Asp Leu Glu Lys Ile Ala Leu Glu Tyr Ile Val Pro Cys Leu His
    10                  15                  20

gag gtg ggc ttc tgc tac ctg gac aac ttc ctg ggc gag gtg gtg ggc 448
Glu Val Gly Phe Cys Tyr Leu Asp Asn Phe Leu Gly Glu Val Val Gly
25                  30                  35                  40

gac tgc gtc ctg gag cgc gtc aag cag ctg cac tgc acc ggg gcc ctg 496
Asp Cys Val Leu Glu Arg Val Lys Gln Leu His Cys Thr Gly Ala Leu
                45                  50                  55

cgg gac ggc cag ctg gcg ggg ccg cgc gcc ggc gtc tcc aag cga cac 544
Arg Asp Gly Gln Leu Ala Gly Pro Arg Ala Gly Val Ser Lys Arg His
            60                  65                  70

ctg cgg ggc gac cag atc acg tgg atc ggg ggc aac gag gag ggc tgc 592
Leu Arg Gly Asp Gln Ile Thr Trp Ile Gly Gly Asn Glu Glu Gly Cys
        75                  80                  85

gag gcc atc agc ttc ctc ctg tcc ctc atc gac agg ctg gtc ctc tac 640
Glu Ala Ile Ser Phe Leu Leu Ser Leu Ile Asp Arg Leu Val Leu Tyr
    90                  95                  100

tgc ggg agc cgg ctg ggc aaa tac tac gtc aag gag agg tct aag gca 688
Cys Gly Ser Arg Leu Gly Lys Tyr Tyr Val Lys Glu Arg Ser Lys Ala
105                 110                 115                 120

atg gtg gct tgc tat ccg gga aat gga aca ggt tat gtt cgc cac gtg 736
Met Val Ala Cys Tyr Pro Gly Asn Gly Thr Gly Tyr Val Arg His Val
                125                 130                 135

gac aac ccc aac ggt gat ggt cgc tgc atc acc tgc atc tac tat ctg 784
Asp Asn Pro Asn Gly Asp Gly Arg Cys Ile Thr Cys Ile Tyr Tyr Leu
            140                 145                 150

aac aag aat tgg gat gcc aag cta cat ggt ggg atc ctg cgg ata ttt 832
Asn Lys Asn Trp Asp Ala Lys Leu His Gly Gly Ile Leu Arg Ile Phe
        155                 160                 165

cca gag ggg aaa tca ttc ata gca gat gtg gag ccc att ttt gac aga 880
Pro Glu Gly Lys Ser Phe Ile Ala Asp Val Glu Pro Ile Phe Asp Arg
    170                 175                 180

ctc ctg ttc ttc tgg tca gat cgt agg aac cca cac gaa gtg cag ccc 928
Leu Leu Phe Phe Trp Ser Asp Arg Arg Asn Pro His Glu Val Gln Pro

185                 190                 195                 200

tct tac gca acc aga tat gct atg act gtc tgg tac ttt gat gct gaa      976
Ser Tyr Ala Thr Arg Tyr Ala Met Thr Val Trp Tyr Phe Asp Ala Glu
            205                 210                 215

gaa agg gca gaa gcc aaa aag aaa ttc agg aat tta act agg aaa act      1024
Glu Arg Ala Glu Ala Lys Lys Lys Phe Arg Asn Leu Thr Arg Lys Thr
            220                 225                 230

gaa tct gcc ctc act gaa gac tga ccgtgctctg aaatctgctg gccttgttca      1078
Glu Ser Ala Leu Thr Glu Asp
        235

ttttagtaac ggttcctgaa ttctcttaaa ttctttgaga tccaaagatg gcctcttcag      1138

tgacaacaat ctccctgcta cttcttgcat ccttcacatc cctgtcttgt gtgtggtact      1198

tcatgttttc ttgccaagac tgtgttgatc ttcagatact ctctttgcca gatgaagtta      1258

cttgctaact ccagaaattc ctgcagacat cctactcggc cagcggttta cctgatagat      1318

tcggtaatac tatcaagaga agagcctagg agcacagcga gggaatgaac cttacttgca      1378

ctttatgtat acttcctgat ttgaaaggag gaggtttgaa aagaaaaaaa tggaggtggt      1438

agatgccaca gagaggcatc acggaagcct taacagcagg aaacagagaa atttgtgtca      1498

tctgaacaat ttccagatgt tcttaatcca gggctgttgg ggtttctgga gaattatcac      1558

aacctaatga cattaatacc tctagaaagg gctgctgtca tagtgaacaa tttataagtg      1618

tcccatgggg cagacactcc ttttttccca gtcctgcaac ctggattttc tgcctcagcc      1678

ccattttgct gaaaataatg actttctgaa taaagatggc aacacaattt tttctccatt      1738

ttcagttctt acctgggaac ctaattcccc agaagctaaa aaactagaca ttagttgttt      1798

tggttgcttt gttggaatgg aatttaaatt taaatgaaag gaaaaatata tccctggtag      1858

ttttgtgtta accactgata actgtggaaa gagctaggtc tactgatata caataaacat      1918

gtgtgcatct tgaacaattt gagaggggag gtggagttgg aaatgtgggt gttcctgttt      1978

tttttttttt ttttttttta gttttccttt ttaatgagct caccctttaa cacaaaaaaa      2038

gcagggtgat gtattttaaa aaaggaagtg gaaataaaaa aatctcaaag ctatttgagt      2098

tctcgtctgt ccctagcagt ctttcttcag ctcacttggc tctctagatc cactgtggtt      2158

ggcagtatga ccagaatcat ggaatttgct agaactgtgg aagcttctac tcctgcagta      2218

agcacagatc gcactgcctc aataacttgg tattgagcac gtattttgca aaagctactt      2278

ttcctagttt tcagtattac tttcatgttt taaaaatccc tttaatttct tgcttgaaaa      2338

tcccatgaac attaaagagc cagaaatatt ttcctttgtt atgtacggat atatatatat      2398

atagtcttcc aagatagaag tttacttttt cctcttctgg ttttggaaaa tttccagata      2458

agacatgtca ccattaattc tcaacgactg ctctattttg ttgtacggta atagttatca      2518

ccttctaaat tactatgtaa tttactcact tattatgttt attgtcttgt atcctttctc      2578

tggagtgtaa gcacaatgaa gacaggaatt ttgtatattt ttaaccaatg caacatactc 2638

tcagcaccta aaatagtgcc gggaacatag taagggctca gtaaatactt gttgaataaa 2698

ctcagtctcc tacattagca ttctaaaaaa aaaaaaaaaa aaaaaaaaaa aaaaaaaaaa 2758

aaaaaag 2765

<210> 12
<211> 239
<212> PRT
<213> Homo sapiens

<400> 12

Met Pro Leu Gly His Ile Met Arg Leu Asp Leu Glu Lys Ile Ala Leu
1               5                   10                  15

Glu Tyr Ile Val Pro Cys Leu His Glu Val Gly Phe Cys Tyr Leu Asp
            20                  25                  30

Asn Phe Leu Gly Glu Val Val Gly Asp Cys Val Leu Glu Arg Val Lys
        35                  40                  45

Gln Leu His Cys Thr Gly Ala Leu Arg Asp Gly Gln Leu Ala Gly Pro
    50                  55                  60

Arg Ala Gly Val Ser Lys Arg His Leu Arg Gly Asp Gln Ile Thr Trp
65                  70                  75                  80

Ile Gly Gly Asn Glu Glu Gly Cys Glu Ala Ile Ser Phe Leu Leu Ser
                85                  90                  95

Leu Ile Asp Arg Leu Val Leu Tyr Cys Gly Ser Arg Leu Gly Lys Tyr
            100                 105                 110

Tyr Val Lys Glu Arg Ser Lys Ala Met Val Ala Cys Tyr Pro Gly Asn
        115                 120                 125

Gly Thr Gly Tyr Val Arg His Val Asp Asn Pro Asn Gly Asp Gly Arg
    130                 135                 140

Cys Ile Thr Cys Ile Tyr Tyr Leu Asn Lys Asn Trp Asp Ala Lys Leu
145                 150                 155                 160

His Gly Gly Ile Leu Arg Ile Phe Pro Glu Gly Lys Ser Phe Ile Ala
                165                 170                 175

Asp Val Glu Pro Ile Phe Asp Arg Leu Leu Phe Phe Trp Ser Asp Arg
            180                 185                 190

Arg Asn Pro His Glu Val Gln Pro Ser Tyr Ala Thr Arg Tyr Ala Met
        195                 200                 205

Thr Val Trp Tyr Phe Asp Ala Glu Glu Arg Ala Glu Ala Lys Lys Lys
    210                 215                 220

Phe Arg Asn Leu Thr Arg Lys Thr Glu Ser Ala Leu Thr Glu Asp
225                 230                 235

<210>  13
<211>  10
<212>  PRT
<213>  artificial

<220>
<223>  Synthetic peptide

<400>  13

Lys Tyr Asn Tyr Ile Ala Ser Phe Phe Ile
1               5                   10

<210>  14
<211>  9
<212>  PRT
<213>  artificial

<220>
<223>  Synthetic peptide

<400>  14

Arg Tyr Ser Lys Asp Arg Lys Asp Leu
1               5

<210>  15
<211>  9
<212>  PRT
<213>  artificial

<220>
<223>  Synthetic peptide

<400>  15

Phe Tyr Gln Val Glu Pro Ser Glu Ile
1               5

<210>  16
<211>  9
<212>  PRT
<213>  artificial

<220>
<223>  Synthetic peptide

<400>  16

Lys Tyr Glu Leu Glu Asn Glu Glu Ile
1               5


<210>  17
<211>  9
<212>  PRT
<213>  artificial

<220>
<223>  Synthetic peptide

<400>  17

Asp Met Ser Asn Ser Asn Asp Cys Met
1               5


<210>  18
<211>  9
<212>  PRT
<213>  artificial

<220>
<223>  Synthetic peptide

<400>  18

Cys Met Arg Asp Ser Ser Gln Ile Leu
1               5


<210>  19
<211>  9
<212>  PRT
<213>  artificial

<220>
<223>  Synthetic peptide

<400>  19

Arg Met Lys His Ile Arg Gln Ala Met
1               5


<210>  20
<211>  10
<212>  PRT
<213>  artificial

<220>
<223>  Synthetic peptide

<400>  20

Gln Phe Val Arg Phe Glu Ala Thr Asp Leu
1               5                   10

<210>  21
<211>  10
<212>  PRT
<213>  artificial

<220>
<223>  Synthetic peptide

<400>  21

Cys Met Pro Arg Gly Tyr Leu Glu Val Ile
1               5                   10


<210>  22
<211>  9
<212>  PRT
<213>  artificial

<220>
<223>  Synthetic peptide

<400>  22

Cys Phe Asn Ser Asp Ile Val Leu Met
1               5


<210>  23
<211>  10
<212>  PRT
<213>  artificial

<220>
<223>  Synthetic peptide

<400>  23

Pro Trp Ala Asp Arg Thr Ala Arg Val Ile
1               5                   10


<210>  24
<211>  10
<212>  PRT
<213>  artificial

<220>
<223>  Synthetic peptide

<400>  24

Thr Phe Thr Val Thr Gly Glu Pro Leu Leu
1               5                   10


<210>  25
<211>  9
<212>  PRT
<213>  artificial

<220>
<223>  Synthetic peptide

<400>  25

Gly Phe Ser Leu Leu Ser Ala Phe Ile
1               5


<210>  26
<211>  10
<212>  PRT
<213>  artificial

<220>
<223>  Synthetic peptide

<400>  26

Pro Phe Met Gln Glu Leu Val Asp Gly Leu
1               5                   10


<210>  27
<211>  10
<212>  PRT
<213>  artificial

<220>
<223>  Synthetic peptide

<400>  27

Gln Met Glu Ile Ser Phe Leu Pro Glu Phe
1               5                   10


<210>  28
<211>  10
<212>  PRT
<213>  artificial

<220>
<223>  Synthetic peptide

<400>  28

Leu Trp Asp Val Asp Arg Lys Lys Asn Met
1               5                   10


<210>  29
<211>  10
<212>  PRT
<213>  artificial

<220>
<223>  Synthetic peptide

<400>  29

Thr Trp Gly Tyr Asn Asp Asn Ser Cys Ile
1               5                   10

<210>  30
<211>  9
<212>  PRT
<213>  artificial

<220>
<223>  Synthetic peptide

<400>  30

Gly Tyr Asn Asp Asn Ser Cys Ile Phe
1               5


<210>  31
<211>  9
<212>  PRT
<213>  artificial

<220>
<223>  Synthetic peptide

<400>  31

Thr Phe Ile Arg Tyr Phe Val Ala Met
1               5


<210>  32
<211>  9
<212>  PRT
<213>  artificial

<220>
<223>  Synthetic peptide

<400>  32

Ile Leu Ser His Ser Leu Trp Asp Val
1               5


<210>  33
<211>  9
<212>  PRT
<213>  artificial

<220>
<223>  Synthetic peptide

<400>  33

Phe Met Gln Glu Leu Val Asp Gly Leu
1               5


<210>  34
<211>  9
<212>  PRT
<213>  artificial

<220>
<223>  Synthetic peptide

<400>  34

Gly Leu Tyr His Thr Gly Ala Asn Val
1               5


<210>  35
<211>  10
<212>  PRT
<213>  artificial

<220>
<223>  Synthetic peptide

<400>  35

Phe Ile Tyr Asp Phe Cys Ile Phe Gly Val
1               5                   10


<210>  36
<211>  10
<212>  PRT
<213>  artificial

<220>
<223>  Synthetic peptide

<400>  36

Leu Met Pro Glu Leu Ser Thr Phe Arg Val
1               5                   10


<210>  37
<211>  10
<212>  PRT
<213>  artificial

<220>
<223>  Synthetic peptide

<400>  37

Ala Met Ala Lys Asn Arg Leu Gln Phe Val
1               5                   10


<210>  38
<211>  10
<212>  PRT
<213>  artificial

<220>
<223>  Synthetic peptide

<400>  38

Gly Ile Leu Ser His Ser Leu Trp Asp Val
1               5                   10

<210>  39
<211>  10
<212>  PRT
<213>  artificial

<220>
<223>  Synthetic peptide

<400>  39

Phe Leu Pro Glu Phe Gly Ile Ser Ser Ala
1               5                   10


<210>  40
<211>  10
<212>  PRT
<213>  artificial

<220>
<223>  Synthetic peptide

<400>  40

Phe Leu Asn Asn His Asp Gln Pro Phe Met
1               5                   10


<210>  41
<211>  10
<212>  PRT
<213>  artificial

<220>
<223>  Synthetic peptide

<400>  41

Gln Leu Thr Ile Thr Gly Lys Lys Trp Leu
1               5                   10


<210>  42
<211>  10
<212>  PRT
<213>  artificial

<220>
<223>  Synthetic peptide

<400>  42

Gln Trp Val Arg Pro Ala Arg Ala Asp Phe
1               5                   10


<210>  43
<211>  10
<212>  PRT
<213>  artificial

<220>
<223>  Synthetic peptide

<400>  43

Ser Trp Ala Ser Ala Glu Lys Pro Tyr Leu
1               5                   10


<210>  44
<211>  10
<212>  PRT
<213>  artificial

<220>
<223>  Synthetic peptide

<400>  44

Tyr Phe Gln Thr Val Lys His Asn Asn Ile
1               5                   10


<210>  45
<211>  10
<212>  PRT
<213>  artificial

<220>
<223>  Synthetic peptide

<400>  45

Leu Met Asp Val Phe Thr Asp Val Glu Ile
1               5                   10


<210>  46
<211>  10
<212>  PRT
<213>  artificial

<220>
<223>  Synthetic peptide

<400>  46

Ile Tyr Cys Ala Lys Ser Gly Arg Lys Phe
1               5                   10


<210>  47
<211>  9
<212>  PRT
<213>  artificial

<220>
<223>  Synthetic peptide

<400>  47

Lys Phe Thr Gly Gln Ala Val Glu Leu
1               5

<210> 48
<211> 9
<212> PRT
<213> artificial

<220>
<223> Synthetic peptide

<400> 48

Leu Phe Asp Glu Glu Phe Arg His Leu
1               5


<210> 49
<211> 9
<212> PRT
<213> artificial

<220>
<223> Synthetic peptide

<400> 49

Leu Tyr Ala Ser Ser Lys Pro Val Met
1               5


<210> 50
<211> 9
<212> PRT
<213> artificial

<220>
<223> Synthetic peptide

<400> 50

Ile Leu Met Asp Val Phe Thr Asp Val
1               5


<210> 51
<211> 9
<212> PRT
<213> artificial

<220>
<223> Synthetic peptide

<400> 51

Val Leu Ser Gly Ser Tyr Ser Phe Thr
1               5


<210> 52
<211> 9
<212> PRT
<213> artificial

<220>
<223> Synthetic peptide

<400>  52

Leu Gln Ser Gly Thr Tyr Phe Pro Val
1               5


<210>  53
<211>  10
<212>  PRT
<213>  artificial

<220>
<223>  Synthetic peptide

<400>  53

Ser Leu Gln Ser Gly Thr Tyr Phe Pro Val
1               5                   10


<210>  54
<211>  10
<212>  PRT
<213>  artificial

<220>
<223>  Synthetic peptide

<400>  54

Val Leu Leu Asp Gln Gly Gly Val Lys Leu
1               5                   10


<210>  55
<211>  10
<212>  PRT
<213>  artificial

<220>
<223>  Synthetic peptide

<400>  55

Lys Leu Phe Gln Glu Met Cys Asp Lys Val
1               5                   10


<210>  56
<211>  10
<212>  PRT
<213>  artificial

<220>
<223>  Synthetic peptide

<400>  56

Phe Leu Ser Ser Val Glu Ala Gln Tyr Ile
1               5                   10

<210>  57
<211>  10
<212>  PRT
<213>  artificial

<220>
<223>  Synthetic peptide

<400>  57

Ile Leu Ser Lys Phe Thr Gly Gln Ala Val
1               5                   10


<210>  58
<211>  10
<212>  PRT
<213>  artificial

<220>
<223>  Synthetic peptide

<400>  58

Val Ile Leu Met Asp Val Phe Thr Asp Val
1               5                   10


<210>  59
<211>  10
<212>  PRT
<213>  artificial

<220>
<223>  Synthetic peptide

<400>  59

Lys Tyr Gly Val Gln Ala Asp Ala Lys Leu
1               5                   10


<210>  60
<211>  9
<212>  PRT
<213>  artificial

<220>
<223>  Synthetic peptide

<400>  60

Leu Tyr Ser Lys Thr Met Thr Pro Ile
1               5


<210>  61
<211>  10
<212>  PRT
<213>  artificial

<220>
<223>  Synthetic peptide

<400>  61

Lys Tyr Asp Ala Val Arg Ile Asn Gln Leu
1               5                   10


<210>  62
<211>  10
<212>  PRT
<213>  artificial

<220>
<223>  Synthetic peptide

<400>  62

Leu Tyr Glu Gln Ala Arg Trp Ala Ile Leu
1               5                   10


<210>  63
<211>  9
<212>  PRT
<213>  artificial

<220>
<223>  Synthetic peptide

<400>  63

Gln Tyr His Ile Ser Lys Leu Ser Leu
1               5


<210>  64
<211>  10
<212>  PRT
<213>  artificial

<220>
<223>  Synthetic peptide

<400>  64

Ser Tyr Gln Pro Glu Val Leu Asn Ile Leu
1               5                   10


<210>  65
<211>  9
<212>  PRT
<213>  artificial

<220>
<223>  Synthetic peptide

<400>  65

Glu Tyr Ile Gly Gly Tyr Ile Phe Leu
1               5

<210>  66
<211>  9
<212>  PRT
<213>  artificial

<220>
<223>  Synthetic peptide

<400>  66

Val Met Leu Lys Leu Val Glu Gln Ile
1               5


<210>  67
<211>  10
<212>  PRT
<213>  artificial

<220>
<223>  Synthetic peptide

<400>  67

Cys Trp Leu Leu Lys Thr His Trp Thr Leu
1               5                   10


<210>  68
<211>  9
<212>  PRT
<213>  artificial

<220>
<223>  Synthetic peptide

<400>  68

Leu Phe Thr Pro Gln His Lys Met Leu
1               5


<210>  69
<211>  10
<212>  PRT
<213>  artificial

<220>
<223>  Synthetic peptide

<400>  69

Thr Met Thr Trp Phe Ser Asp Ser Pro Leu
1               5                   10


<210>  70
<211>  9
<212>  PRT
<213>  artificial

<220>
<223>  Synthetic peptide

<400>  70

Gly Trp Leu Asp Ser Ser Arg Ser Leu
1               5


<210>  71
<211>  10
<212>  PRT
<213>  artificial

<220>
<223>  Synthetic peptide

<400>  71

Arg Phe Lys Tyr Tyr Ser Phe Phe Asp Leu
1               5                   10


<210>  72
<211>  10
<212>  PRT
<213>  artificial

<220>
<223>  Synthetic peptide

<400>  72

Tyr Trp Phe Ile Phe Lys Asp Thr Ser Ile
1               5                   10


<210>  73
<211>  10
<212>  PRT
<213>  artificial

<220>
<223>  Synthetic peptide

<400>  73

Gln Tyr Ala Gln Trp Met Ala Ala Cys Met
1               5                   10


<210>  74
<211>  9
<212>  PRT
<213>  artificial

<220>
<223>  Synthetic peptide

<400>  74

Asn Met Asp Met Asn Pro Glu Cys Phe
1               5

<210>  75
<211>  9
<212>  PRT
<213>  artificial

<220>
<223>  Synthetic peptide

<400>  75

Gln Met Pro Leu Val Glu Ala Lys Leu
1               5


<210>  76
<211>  9
<212>  PRT
<213>  artificial

<220>
<223>  Synthetic peptide

<400>  76

Arg Phe Ile Gln Ala Trp Gln Ser Leu
1               5


<210>  77
<211>  10
<212>  PRT
<213>  artificial

<220>
<223>  Synthetic peptide

<400>  77

Ala Trp Gln Ser Leu Pro Glu Phe Gly Leu
1               5                   10


<210>  78
<211>  10
<212>  PRT
<213>  artificial

<220>
<223>  Synthetic peptide

<400>  78

Thr Trp Arg Phe Thr Asn Ile Lys Gln Trp
1               5                   10


<210>  79
<211>  9
<212>  PRT
<213>  artificial

<220>
<223>  Synthetic peptide

<400>  79

Trp Leu Leu Lys Thr His Trp Thr Leu
1               5


<210>  80
<211>  9
<212>  PRT
<213>  artificial

<220>
<223>  Synthetic peptide

<400>  80

Ser Leu Leu Glu Asp Ile Thr Asp Ile
1               5


<210>  81
<211>  9
<212>  PRT
<213>  artificial

<220>
<223>  Synthetic peptide

<400>  81

Lys Leu Phe Arg Pro Lys Lys Leu Leu
1               5


<210>  82
<211>  9
<212>  PRT
<213>  artificial

<220>
<223>  Synthetic peptide

<400>  82

Lys Leu Asn Leu Arg Gly Cys Glu Val
1               5


<210>  83
<211>  9
<212>  PRT
<213>  artificial

<220>
<223>  Synthetic peptide

<400>  83

Ile Leu Leu Glu Glu Ile Asp Cys Thr
1               5

<210>  84
<211>  9
<212>  PRT
<213>  artificial

<220>
<223>  Synthetic peptide

<400>  84

Lys Gln Trp Asn Val Asn Trp Glu Thr
1               5


<210>  85
<211>  9
<212>  PRT
<213>  artificial

<220>
<223>  Synthetic peptide

<400>  85

Val Val Ile Glu Phe Asp Gln Asn Val
1               5


<210>  86
<211>  10
<212>  PRT
<213>  artificial

<220>
<223>  Synthetic peptide

<400>  86

Gln Leu Tyr Glu Gln Ala Arg Trp Ala Ile
1               5                   10


<210>  87
<211>  10
<212>  PRT
<213>  artificial

<220>
<223>  Synthetic peptide

<400>  87

Lys Met Leu Arg Leu Arg Leu Pro Asn Leu
1               5                   10


<210>  88
<211>  10
<212>  PRT
<213>  artificial

<220>
<223>  Synthetic peptide

<400>  88

Leu Leu Phe Thr Pro Gln His Lys Met Leu
1               5                   10


<210>  89
<211>  10
<212>  PRT
<213>  artificial

<220>
<223>  Synthetic peptide

<400>  89

Glu Tyr Leu Ile Thr Leu Leu Glu His Leu
1               5                   10


<210>  90
<211>  9
<212>  PRT
<213>  artificial

<220>
<223>  Synthetic peptide

<400>  90

Lys Tyr His Asn Asp Pro Arg Phe Ile
1               5


<210>  91
<211>  9
<212>  PRT
<213>  artificial

<220>
<223>  Synthetic peptide

<400>  91

Leu Tyr Asn His Gly Ile Gly Thr Leu
1               5


<210>  92
<211>  9
<212>  PRT
<213>  artificial

<220>
<223>  Synthetic peptide

<400>  92

Leu Tyr Ile Ala Trp Ala Gly His Leu
1               5

<210>  93
<211>  9
<212>  PRT
<213>  artificial

<220>
<223>  Synthetic peptide

<400>  93

Gln Tyr Arg Leu Phe Gln Thr Arg Leu
1               5


<210>  94
<211>  9
<212>  PRT
<213>  artificial

<220>
<223>  Synthetic peptide

<400>  94

Met Tyr Ser Ala Ser Leu Leu Arg Leu
1               5


<210>  95
<211>  9
<212>  PRT
<213>  artificial

<220>
<223>  Synthetic peptide

<400>  95

Val Tyr Glu Ala Thr Gln Gly Asp Leu
1               5


<210>  96
<211>  9
<212>  PRT
<213>  artificial

<220>
<223>  Synthetic peptide

<400>  96

Ser Tyr Gly Thr Leu Leu Asn Ala Ile
1               5


<210>  97
<211>  9
<212>  PRT
<213>  artificial

<220>
<223>  Synthetic peptide

<400>  97

His Tyr Thr Asn Lys Ile Arg Ala Leu
1               5


<210>  98
<211>  10
<212>  PRT
<213>  artificial

<220>
<223>  Synthetic peptide

<400>  98

Arg Tyr Ile Gln Trp Val Glu Glu Asn Phe
1               5                   10


<210>  99
<211>  10
<212>  PRT
<213>  artificial

<220>
<223>  Synthetic peptide

<400>  99

Glu Tyr Asn Ser Asp Leu His Gln Phe Phe
1               5                   10


<210>  100
<211>  9
<212>  PRT
<213>  artificial

<220>
<223>  Synthetic peptide

<400>  100

Asn Met Glu Arg Arg Val Ile Thr Ile
1               5


<210>  101
<211>  9
<212>  PRT
<213>  artificial

<220>
<223>  Synthetic peptide

<400>  101

Val Met Tyr Cys Lys Glu Lys Leu Ile
1               5

<210>  102
<211>  9
<212>  PRT
<213>  artificial

<220>
<223>  Synthetic peptide

<400>  102

Lys Met Asp Glu Leu His Lys Lys Leu
1               5


<210>  103
<211>  9
<212>  PRT
<213>  artificial

<220>
<223>  Synthetic peptide

<400>  103

Thr Tyr Gln Gln Thr Pro Val Asn Met
1               5


<210>  104
<211>  10
<212>  PRT
<213>  artificial

<220>
<223>  Synthetic peptide

<400>  104

Glu Phe Lys Pro Gln Ser Gly Ala Glu Ile
1               5                   10


<210>  105
<211>  10
<212>  PRT
<213>  artificial

<220>
<223>  Synthetic peptide

<400>  105

Met Phe Gln Ala Pro Thr Leu Pro Asp Ile
1               5                   10


<210>  106
<211>  10
<212>  PRT
<213>  artificial

<220>
<223>  Synthetic peptide

<400>  106

Thr Phe Gly Glu Arg Ser Val Ser Arg Leu
1               5                   10


<210>  107
<211>  9
<212>  PRT
<213>  artificial

<220>
<223>  Synthetic peptide

<400>  107

Glu Phe Leu Asp Asp Ser Thr Val Trp
1               5


<210>  108
<211>  10
<212>  PRT
<213>  artificial

<220>
<223>  Synthetic peptide

<400>  108

Val Trp Gly Ile Arg Cys Asn Lys Thr Leu
1               5                   10


<210>  109
<211>  10
<212>  PRT
<213>  artificial

<220>
<223>  Synthetic peptide

<400>  109

Pro Trp Asp Asp Lys Leu Ile Phe Lys Leu
1               5                   10


<210>  110
<211>  9
<212>  PRT
<213>  artificial

<220>
<223>  Synthetic peptide

<400>  110

Leu Met Glu Arg Leu Lys Pro Ser Met
1               5

<210>  111
<211>  10
<212>  PRT
<213>  artificial

<220>
<223>  Synthetic peptide

<400>  111

Leu Tyr Lys Asn Thr Pro Glu Lys Val Met
1               5                   10


<210>  112
<211>  9
<212>  PRT
<213>  artificial

<220>
<223>  Synthetic peptide

<400>  112

Asn Phe Ile Leu Gly Asn Gly Phe Leu
1               5


<210>  113
<211>  10
<212>  PRT
<213>  artificial

<220>
<223>  Synthetic peptide

<400>  113

Asp Met Lys Leu Phe Pro Lys Gly Thr Ile
1               5                   10


<210>  114
<211>  10
<212>  PRT
<213>  artificial

<220>
<223>  Synthetic peptide

<400>  114

Val Tyr Cys Met Leu Phe Gly Thr Tyr Met
1               5                   10


<210>  115
<211>  10
<212>  PRT
<213>  artificial

<220>
<223>  Synthetic peptide

<400>  115

Leu Phe Arg Arg Leu Pro His Leu Asp Met
1               5                   10


<210>  116
<211>  10
<212>  PRT
<213>  artificial

<220>
<223>  Synthetic peptide

<400>  116

Met Trp Asn Glu Phe Phe His Val Met Leu
1               5                   10


<210>  117
<211>  10
<212>  PRT
<213>  artificial

<220>
<223>  Synthetic peptide

<400>  117

Val Phe Gln Gln His Tyr Thr Asn Lys Ile
1               5                   10


<210>  118
<211>  9
<212>  PRT
<213>  artificial

<220>
<223>  Synthetic peptide

<400>  118

Gln Leu Gly Ser Lys Leu Val Tyr Val
1               5


<210>  119
<211>  9
<212>  PRT
<213>  artificial

<220>
<223>  Synthetic peptide

<400>  119

Met Leu Phe Gly Thr Tyr Met Lys Val
1               5

<210>  120
<211>  9
<212>  PRT
<213>  artificial

<220>
<223>  Synthetic peptide

<400>  120

Asp Met Trp Asn Glu Phe Phe His Val
1               5


<210>  121
<211>  9
<212>  PRT
<213>  artificial

<220>
<223>  Synthetic peptide

<400>  121

Thr Val Thr Asp Ser Met Phe Ala Val
1               5


<210>  122
<211>  9
<212>  PRT
<213>  artificial

<220>
<223>  Synthetic peptide

<400>  122

Arg Met Leu Tyr Met Ile Glu Gln Val
1               5


<210>  123
<211>  9
<212>  PRT
<213>  artificial

<220>
<223>  Synthetic peptide

<400>  123

Gly Leu Phe Arg Arg Leu Pro His Leu
1               5


<210>  124
<211>  9
<212>  PRT
<213>  artificial

<220>
<223>  Synthetic peptide

<400>  124

Tyr Met Ile Glu Gln Val His Asp Cys
1               5


<210>  125
<211>  9
<212>  PRT
<213>  artificial

<220>
<223>  Synthetic peptide

<400>  125

Leu Leu Ser Gly Leu Ser Lys Pro Val
1               5


<210>  126
<211>  9
<212>  PRT
<213>  artificial

<220>
<223>  Synthetic peptide

<400>  126

Tyr Leu Ile Thr Leu Leu Glu His Leu
1               5


<210>  127
<211>  9
<212>  PRT
<213>  artificial

<220>
<223>  Synthetic peptide

<400>  127

Phe Leu Tyr Asn His Gly Ile Gly Thr
1               5


<210>  128
<211>  9
<212>  PRT
<213>  artificial

<220>
<223>  Synthetic peptide

<400>  128

Lys Met Asp Glu Leu His Lys Lys Leu
1               5

<210>  129
<211>  9
<212>  PRT
<213>  artificial

<220>
<223>  Synthetic peptide

<400>  129

Lys Leu Val Tyr Val His His Leu Leu
1               5


<210>  130
<211>  9
<212>  PRT
<213>  artificial

<220>
<223>  Synthetic peptide

<400>  130

Trp Val Asn Glu Asp Arg His Tyr Met
1               5


<210>  131
<211>  9
<212>  PRT
<213>  artificial

<220>
<223>  Synthetic peptide

<400>  131

Arg Leu Thr Asp Thr Asp Ala Ala Ile
1               5


<210>  132
<211>  9
<212>  PRT
<213>  artificial

<220>
<223>  Synthetic peptide

<400>  132

Ala Leu Ile Asp Leu Gly Gln Ser Ile
1               5


<210>  133
<211>  9
<212>  PRT
<213>  artificial

<220>
<223>  Synthetic peptide

<400>  133

Leu Ile Phe Lys Leu Leu Ser Gly Leu
1               5


<210>  134
<211>  9
<212>  PRT
<213>  artificial

<220>
<223>  Synthetic peptide

<400>  134

Val Met Tyr Cys Lys Glu Lys Leu Ile
1               5


<210>  135
<211>  9
<212>  PRT
<213>  artificial

<220>
<223>  Synthetic peptide

<400>  135

Phe Ile Thr Tyr Cys Leu Lys Phe Ala
1               5


<210>  136
<211>  9
<212>  PRT
<213>  artificial

<220>
<223>  Synthetic peptide

<400>  136

Lys Leu Pro Val Glu Ser Val His Ile
1               5


<210>  137
<211>  9
<212>  PRT
<213>  artificial

<220>
<223>  Synthetic peptide

<400>  137

Ile Met Asn Met Phe Gln Ala Pro Thr
1               5

<210>  138
<211>  10
<212>  PRT
<213>  artificial

<220>
<223>  Synthetic peptide

<400>  138

Phe Gln Leu Gly Ser Lys Leu Val Tyr Val
1               5                   10


<210>  139
<211>  10
<212>  PRT
<213>  artificial

<220>
<223>  Synthetic peptide

<400>  139

Phe Leu Asp Asp Ser Thr Val Trp Gly Ile
1               5                   10


<210>  140
<211>  10
<212>  PRT
<213>  artificial

<220>
<223>  Synthetic peptide

<400>  140

Leu Leu Gly Glu Gly Ala Phe Ala Gln Val
1               5                   10


<210>  141
<211>  10
<212>  PRT
<213>  artificial

<220>
<223>  Synthetic peptide

<400>  141

Lys Leu Leu Ser Gly Leu Ser Lys Pro Val
1               5                   10


<210>  142
<211>  10
<212>  PRT
<213>  artificial

<220>
<223>  Synthetic peptide

<400>  142

Gly Glu Trp Glu Arg Tyr Ile Gln Trp Val
1               5                   10


<210>  143
<211>  10
<212>  PRT
<213>  artificial

<220>
<223>  Synthetic peptide

<400>  143

Lys Leu Phe Pro Lys Gly Thr Ile Phe Thr
1               5                   10


<210>  144
<211>  10
<212>  PRT
<213>  artificial

<220>
<223>  Synthetic peptide

<400>  144

Cys Met Leu Phe Gly Thr Tyr Met Lys Val
1               5                   10


<210>  145
<211>  10
<212>  PRT
<213>  artificial

<220>
<223>  Synthetic peptide

<400>  145

Lys Leu Ile Phe Lys Leu Leu Ser Gly Leu
1               5                   10


<210>  146
<211>  10
<212>  PRT
<213>  artificial

<220>
<223>  Synthetic peptide

<400>  146

Val Leu Thr Cys Glu Ala Glu Leu Gly Val
1               5                   10

<210>  147
<211>  10
<212>  PRT
<213>  artificial

<220>
<223>  Synthetic peptide

<400>  147

Asn Leu Tyr Lys Asn Thr Pro Glu Lys Val
1               5                   10


<210>  148
<211>  10
<212>  PRT
<213>  artificial

<220>
<223>  Synthetic peptide

<400>  148

Phe Leu Tyr Asn His Gly Ile Gly Thr Leu
1               5                   10


<210>  149
<211>  10
<212>  PRT
<213>  artificial

<220>
<223>  Synthetic peptide

<400>  149

Gln Leu Leu Lys Gln Lys Met Asp Glu Leu
1               5                   10


<210>  150
<211>  10
<212>  PRT
<213>  artificial

<220>
<223>  Synthetic peptide

<400>  150

Gln Leu Met Glu Arg Leu Lys Pro Ser Met
1               5                   10


<210>  151
<211>  10
<212>  PRT
<213>  artificial

<220>
<223>  Synthetic peptide

<400>  151

Lys Leu Pro Val Glu Ser Val His Ile Leu
1               5                   10


<210>  152
<211>  10
<212>  PRT
<213>  artificial

<220>
<223>  Synthetic peptide

<400>  152

Trp Met Gln Met Ser Ser Leu Gly Thr Val
1               5                   10


<210>  153
<211>  10
<212>  PRT
<213>  artificial

<220>
<223>  Synthetic peptide

<400>  153

Phe Ile Met Asn Met Phe Gln Ala Pro Thr
1               5                   10


<210>  154
<211>  10
<212>  PRT
<213>  artificial

<220>
<223>  Synthetic peptide

<400>  154

Arg Leu Phe Gln Thr Arg Leu Thr Glu Thr
1               5                   10


<210>  155
<211>  10
<212>  PRT
<213>  artificial

<220>
<223>  Synthetic peptide

<400>  155

Trp Asn Tyr Gln Ile Asp Tyr Phe Gly Val
1               5                   10

<210>  156
<211>  10
<212>  PRT
<213>  artificial

<220>
<223>  Synthetic peptide

<400>  156

Gly Met Val Gln Ala Thr Pro Ser Lys Val
1               5                   10


<210>  157
<211>  10
<212>  PRT
<213>  artificial

<220>
<223>  Synthetic peptide

<400>  157

Asp Leu Leu Arg Gln Lys Leu Lys Lys Val
1               5                   10


<210>  158
<211>  10
<212>  PRT
<213>  artificial

<220>
<223>  Synthetic peptide

<400>  158

Val Tyr Val Lys Gly Leu Leu Ala Lys Ile
1               5                   10


<210>  159
<211>  10
<212>  PRT
<213>  artificial

<220>
<223>  Synthetic peptide

<400>  159

Gln Tyr Pro Ala Thr Glu His Arg Asp Leu
1               5                   10


<210>  160
<211>  10
<212>  PRT
<213>  artificial

<220>
<223>  Synthetic peptide

<400>  160

Glu Met Glu Ile Gln Leu Lys Asp Ala Leu
1               5                   10


<210>  161
<211>  9
<212>  PRT
<213>  artificial

<220>
<223>  Synthetic peptide

<400>  161

Gln Met Gln Ala Cys Thr Leu Asp Phe
1               5


<210>  162
<211>  10
<212>  PRT
<213>  artificial

<220>
<223>  Synthetic peptide

<400>  162

Val Tyr Val Lys Gly Leu Leu Ala Lys Ile
1               5                   10


<210>  163
<211>  10
<212>  PRT
<213>  artificial

<220>
<223>  Synthetic peptide

<400>  163

Gln Tyr Pro Ala Thr Glu His Arg Asp Leu
1               5                   10


<210>  164
<211>  10
<212>  PRT
<213>  artificial

<220>
<223>  Synthetic peptide

<400>  164

Glu Met Glu Ile Gln Leu Lys Asp Ala Leu
1               5                   10

<210>  165
<211>  9
<212>  PRT
<213>  artificial

<220>
<223>  Synthetic peptide

<400>  165

Gln Met Gln Ala Cys Thr Leu Asp Phe
1               5


<210>  166
<211>  9
<212>  PRT
<213>  artificial

<220>
<223>  Synthetic peptide

<400>  166

Gly Leu Leu Ala Lys Ile Phe Glu Leu
1               5


<210>  167
<211>  9
<212>  PRT
<213>  artificial

<220>
<223>  Synthetic peptide

<400>  167

Gln Leu Leu Val Ile Leu Lys Glu Leu
1               5


<210>  168
<211>  9
<212>  PRT
<213>  artificial

<220>
<223>  Synthetic peptide

<400>  168

Ala Leu Leu Glu Gln Leu Glu Glu Thr
1               5


<210>  169
<211>  9
<212>  PRT
<213>  artificial

<220>
<223>  Synthetic peptide

<400>  169

Lys Gln Gln Glu Glu Gln Thr Arg Val
1               5


<210>  170
<211>  9
<212>  PRT
<213>  artificial

<220>
<223>  Synthetic peptide

<400>  170

Tyr Leu Gln Glu Glu Lys Gln Lys Cys
1               5


<210>  171
<211>  9
<212>  PRT
<213>  artificial

<220>
<223>  Synthetic peptide

<400>  171

Thr Gln Leu Glu Ser Leu Lys Gln Leu
1               5


<210>  172
<211>  10
<212>  PRT
<213>  artificial

<220>
<223>  Synthetic peptide

<400>  172

Leu Leu Tyr Ser Gln Arg Arg Ala Asp Val
1               5                   10


<210>  173
<211>  10
<212>  PRT
<213>  artificial

<220>
<223>  Synthetic peptide

<400>  173

Lys Leu Thr Asp Lys Glu Arg His Arg Leu
1               5                   10

<210>  174
<211>  10
<212>  PRT
<213>  artificial

<220>
<223>  Synthetic peptide

<400>  174

Ala Leu Leu Glu Gln Gln Met Gln Ala Cys
1               5                   10


<210>  175
<211>  10
<212>  PRT
<213>  artificial

<220>
<223>  Synthetic peptide

<400>  175

Leu Leu Ser Gln Val Gln Phe Leu Tyr Thr
1               5                   10


<210>  176
<211>  9
<212>  PRT
<213>  artificial

<220>
<223>  Synthetic peptide

<400>  176

Ile Met Arg Leu Asp Leu Glu Lys Ile
1               5


<210>  177
<211>  9
<212>  PRT
<213>  artificial

<220>
<223>  Synthetic peptide

<400>  177

Gly Phe Cys Tyr Leu Asp Asn Phe Leu
1               5


<210>  178
<211>  10
<212>  PRT
<213>  artificial

<220>
<223>  Synthetic peptide

<400>  178

Ser Phe Leu Leu Ser Leu Ile Asp Arg Leu
1               5                   10


<210>  179
<211>  10
<212>  PRT
<213>  artificial

<220>
<223>  Synthetic peptide

<400>  179

Tyr Tyr Val Lys Glu Arg Ser Lys Ala Met
1               5                   10


<210>  180
<211>  10
<212>  PRT
<213>  artificial

<220>
<223>  Synthetic peptide

<400>  180

Asn Trp Asp Ala Lys Leu His Gly Gly Ile
1               5                   10


<210>  181
<211>  9
<212>  PRT
<213>  artificial

<220>
<223>  Synthetic peptide

<400>  181

Ile Phe Pro Glu Gly Lys Ser Phe Ile
1               5


<210>  182
<211>  9
<212>  PRT
<213>  artificial

<220>
<223>  Synthetic peptide

<400>  182

Ser Phe Ile Ala Asp Val Glu Pro Ile
1               5

<210>  183
<211>  9
<212>  PRT
<213>  artificial

<220>
<223>  Synthetic peptide

<400>  183

Arg Tyr Ala Met Thr Val Trp Tyr Phe
1               5


<210>  184
<211>  9
<212>  PRT
<213>  artificial

<220>
<223>  Synthetic peptide

<400>  184

Phe Leu Leu Ser Leu Ile Asp Arg Leu
1               5


<210>  185
<211>  9
<212>  PRT
<213>  artificial

<220>
<223>  Synthetic peptide

<400>  185

Tyr Leu Asp Asn Phe Leu Gly Glu Val
1               5


<210>  186
<211>  9
<212>  PRT
<213>  artificial

<220>
<223>  Synthetic peptide

<400>  186

Tyr Ile Val Pro Cys Leu His Glu Val
1               5


<210>  187
<211>  9
<212>  PRT
<213>  artificial

<220>
<223>  Synthetic peptide

73

<400>  187

Lys Leu His Gly Gly Ile Leu Arg Ile
1               5


<210>  188
<211>  9
<212>  PRT
<213>  artificial

<220>
<223>  Synthetic peptide

<400>  188

Gln Leu Ala Gly Pro Arg Ala Gly Val
1               5


<210>  189
<211>  9
<212>  PRT
<213>  artificial

<220>
<223>  Synthetic peptide

<400>  189

Cys Val Leu Glu Arg Val Lys Gln Leu
1               5


<210>  190
<211>  10
<212>  PRT
<213>  artificial

<220>
<223>  Synthetic peptide

<400>  190

Cys Leu His Glu Val Gly Phe Cys Tyr Leu
1               5                   10


<210>  191
<211>  10
<212>  PRT
<213>  artificial

<220>
<223>  Synthetic peptide

<400>  191

Phe Leu Gly Glu Val Val Gly Asp Cys Val
1               5                   10

74

<210>  192
<211>  10
<212>  PRT
<213>  artificial

<220>
<223>  Synthetic peptide

<400>  192

Ser Leu Ile Asp Arg Leu Val Leu Tyr Cys
1               5                   10


<210>  193
<211>  10
<212>  PRT
<213>  artificial

<220>
<223>  Synthetic peptide

<400>  193

Phe Leu Leu Ser Leu Ile Asp Arg Leu Val
1               5                   10


<210>  194
<211>  10
<212>  PRT
<213>  artificial

<220>
<223>  Synthetic peptide

<400>  194

Tyr Leu Asn Lys Asn Trp Asp Ala Lys Leu
1               5                   10


<210>  195
<211>  11
<212>  PRT
<213>  artificial

<220>
<223>  Synthetic peptide

<400>  195

Leu Met Pro Glu Leu Ser Thr Phe Arg Val Leu
1               5                   10


<210>  196
<211>  12
<212>  PRT
<213>  artificial

<220>
<223>  Synthetic peptide

<400>  196

Asn Met Phe Cys Ser Thr Ser Gly Thr Glu Gln Leu
1               5                   10


<210>  197
<211>  12
<212>  PRT
<213>  artificial

<220>
<223>  Synthetic peptide

<400>  197

Ala Met Lys Lys Tyr Glu Leu Glu Asn Glu Glu Ile
1               5                   10


<210>  198
<211>  11
<212>  PRT
<213>  artificial

<220>
<223>  Synthetic peptide

<400>  198

Arg Tyr Ile Ala Lys Arg Gln Leu Ser His Leu
1               5                   10


<210>  199
<211>  11
<212>  PRT
<213>  artificial

<220>
<223>  Synthetic peptide

<400>  199

Leu Tyr His Thr Gly Ala Asn Val Glu Ser Phe
1               5                   10


<210>  200
<211>  11
<212>  PRT
<213>  artificial

<220>
<223>  Synthetic peptide

<400>  200

Arg Tyr Phe Val Ala Met Lys Lys Tyr Glu Leu
1               5                   10

<210>  201
<211>  12
<212>  PRT
<213>  artificial

<220>
<223>  Synthetic peptide

<400>  201

Ile Tyr Asp Phe Cys Ile Phe Gly Val Pro Glu Ile
1               5                   10


<210>  202
<211>  20
<212>  DNA
<213>  Artificial

<220>
<223>  primer

<400>  202
tggcactgga agaagatcaa                                                   20


<210>  203
<211>  23
<212>  DNA
<213>  Artificial

<220>
<223>  primer

<400>  203
tcaccacaac tttgttgttt gtt                                               23


<210>  204
<211>  20
<212>  DNA
<213>  Artificial

<220>
<223>  primer

<400>  204
cagatccagg tgcctctgac                                                   20


<210>  205
<211>  21
<212>  DNA
<213>  Artificial

<220>
<223>  primer

<400>  205
tcaggtaatc caaccacctt g                                                 21


<210>  206

<211>  20
<212>  DNA
<213>  Artificial

<220>
<223>  primer

<400>  206
tgaatttctg aggccttgct                                                    20


<210>  207
<211>  20
<212>  DNA
<213>  Artificial

<220>
<223>  primer

<400>  207
tgttctcagc agcaaacagg                                                    20


<210>  208
<211>  20
<212>  DNA
<213>  Artificial

<220>
<223>  primer

<400>  208
ttatctgctg gcttggcact                                                    20


<210>  209
<211>  21
<212>  DNA
<213>  Artificial

<220>
<223>  primer

<400>  209
gcttttgcct taacaaatcc a                                                  21


<210>  210
<211>  22
<212>  DNA
<213>  Artificial

<220>
<223>  primer

<400>  210
tgtccattgt taagaggtgg tg                                                 22


<210>  211
<211>  20
<212>  DNA
<213>  Artificial

<220>
<223>  primer

<400>  211
tgagagggct acatgggttt                                                    20


<210>  212
<211>  20
<212>  DNA
<213>  Artificial

<220>
<223>  primer

<400>  212
catccctgtc ttgtgtgtgg                                                    20


<210>  213
<211>  20
<212>  DNA
<213>  Artificial

<220>
<223>  primer

<400>  213
ccaacagccc tggattaaga                                                    20

## Description

### Technical Field

The invention relates to novel tumor antigen proteins and peptides derived therefrom and use of the same in the field of cancer immunity.

### Background Art

It is known that the immune system, particularly T cells, plays an important role in the elimination of cancer (tumor) by a living body. Indeed, infiltration of lymphocytes exhibiting cytotoxic activity on cancer cells in human cancer foci has been observed (Non-patent literature 1), and cytotoxic T lymphocytes (CTLs) recognizing autologous tumor cells have been isolated from melanomas without great difficulties ((Non-patent literature 2, 3, 4). In addition, the results of clinical treatment of melanomas by transfer of the CTLs also suggested the importance of T cells in cancer elimination (Non-patent literature 5).

Although the target molecules of CTLs attacking autologous tumor cells had long been unclear, such molecules have become clearer gradually along the advance in immunology and molecular biology in recent years. Specifically, it has been revealed that CTLs recognize a complex between a peptide, called cancer antigen peptide, and a major histocompatibility complex class I antigen (MHC class I antigen, also referred to as HLA antigen) through the T cell receptors (TCRs), and thereby attacking autologous tumor cells.

Cancer antigen peptides are generated by intracellular proteasomal degradation of cancer-specific antigen proteins after synthesis in cells. The cancer antigen peptides thus generated bind to MHC class I antigens (HLA antigens) in endoplasmic reticulum to form complexes, and the complexes are transported to the cell surface to be presented as an antigen. Antigen-specific CTLs recognize the complex presented as an antigen, and exhibits anti-cancer effects through the cytotoxic action or production of lymphokines. As a consequence of elucidation of a series of the actions, it has become possible to treat cancer by using cancer antigen protein or a cancer antigen peptide as a so-called cancer vaccine to enhance cancer-specific CTLs in the body of a cancer patient.

As a tumor antigen protein, T. Boon et al. identified a protein named MAGE from human melanoma cells for the first time in 1991 (Non-patent literature 6). Subsequently, several additional tumor antigen proteins have been identified mainly from melanoma cells.

In order to apply tumor antigen proteins and peptides to tumor therapy or diagnostics, it is necessary to identify novel ones which can be applied widely to adenocarcinoma, such as lung cancer, which occurs much more frequently than melanoma.

Lengsin (Glutamate-ammonia ligase (glutamine synthase) domain containing 1 (GLULD1) UniGene Hs.149585) was identified as a novel gene highly expressing in human lens and has been reported to be a possible member of a glutamine synthase family from the similarity of the amino acid sequence. The physiological function of the gene, however, is still unknown (Non-patent literature 7).

BJ-TSA-9 (Hypothetical protein MGC14128) (UniGene Hs.379821) is a novel gene cloned in The National Institutes of Health Mammalian Gene Collection (MGC) Program, and its physiological function is still unknown (Non-patent literature 8).

C20orf42 (or URP1, Kindlerin) was cloned as a gene highly expressing in lung cancer and colon cancer (Non-patent literature 9), and has a high similarity to C. elegans UNC-112. It has been reported that C20orf42 interacts with Integrin as UNC-112 does and the expression of C20orf42 is induced by TGF-β (Non-patent literature 10). C20orf42 was also identified as Kindlerin gene which is mutated in Kindler syndrome, a rare autosomal recessive dermatosis (Non-patent literature 11, 12).

BUB1 is Serine/threonine Kinase involved in spindle checkpoint of cell cycle M phase and lack of functional BUB1 results in abnormal chromosome segregation. In addition, BUB1 phosphorylates Cdc2 and suppresses an ubiquitin ligase activity of APC/C^{cdc20}, resulting in prevention of abnormal chromosome segregation (Non-patent literature 13). Decrease of expression and mutation of BUB1 have been reported in cancer tissue (Non-patent literature 14, 15).

C10orf3 is a novel gene cloned in The National Institutes of Health Mammalian Gene Collection (MGC) Program and its physiological function is still unknown (Non-patent literature 16).

HIFPH3 (egl nine homolog3,EGLN3) was cloned as one of three molecules having a similar activity to egl9, which was identified as a dioxygenase regulating the activity of Hypoxia-inducible factor1(HIF1) in C.elegans (Non-patent literature 17). It is considered to regulate activities under hypoxic condition through HIF1, but differences between the three egl nine homologs is not known.

Non-patent literature 1: Arch.Surg., 126:200 (1990)
Non-patent literature 2: Immunol.Today, 8:385 (1987)
Non-patent literature 3: J.Immunol., 138:989 (1987)
Non-patent literature 4: Int.J.Cancer, 52:52 (1992)
Non-patent literature 5: J.Natl.Cancer.Inst., 86:1159 (1994)
Non-patent literature 6: Science, 254, 1643-1647 (1991)
Non-patent literature 7: Mol. Vis. Jun. 15;8:185-95 (2002)
Non-patent literature 8: Proc. Natl. Acad. Sci. U S A., 99(26):16899-903 (2002)
Non-patent literature 9: Biochim. Biophys. Acta 1637: 207-216 (2003)
Non-patent literature 10: J. Biol. Chem., Feb 20; 279 (8) : 6824-33 (2004)
Non-patent literature 11: Hum. Molec. Genet., 12: 925-935 (2003)
Non-patent literature 12: Am. J. Hum. Genet., 73: 174-187 (2003)
Non-patent literature 13: Mol. Cell., Nov 5;16(3):387-97 (2004)
Non-patent literature 14: Oncogene Jul 11;21(30):4673-9 (2002)
Non-patent literature 15: Leuk. Lymphoma. Feb;43(2):385-91 (2002)
Non-patent literature 16: Proc. Natl. Acad. Sci. U S A., Dec 24;99(26):16899-903 (2002)
Non-patent literature 17: Cell 107: 43-54 (2001)

### Disclosure of Invention

### Problem to be Solved by the Invention

An object of the invention is to provide a novel tumor antigen protein and peptide as well as use of the same in the field of cancer immunity.

### Means for Solving the Problem

The inventers intensively tried to find genes showing increase of expression level or frequency in cancer tissues compared to normal tissues and selected six genes, Lengsin, BJ-TSA-9, C20orf42, BUB1, C10orf3 and HIFPH3 genes, and thus those genes and products expressed therefrom (i. e., proteins) were found to be useful as a disease marker for cancer.

Then, the inventors selected partial peptides possible to bind to an HLA molecule from the amino acid sequence of the proteins encoded by Lengsin, BJ-TSA-9, C20orf42, BUB1, C10orf3 and HIFPH3 genes (Lengsin, BJ-TSA-9, C20orf42, BUB1, C10orf3 and HIFPH3) and examined their biding affinities to an HLA molecule and CTL-inducing activities. As a result, it was found that those six proteins (especially BJ-TSA-9, C20orf42 and C10orf3) were tumor antigen proteins and the partial peptides derived therefrom were tumor antigen peptides. Accordingly, Lengsin, BJ-TSA-9, C20orf42, BUB1, C10orf3 and HIFPH3 and the peptides derived therefrom are considered to be useful as cancer vaccines for various cancers showing expression (particularly increase of expression) of those proteins.
The present invention was accomplished from the findings as described above.

Accordingly, the present invention relates to the followings:
(1) A peptide which comprises a partial peptide derived from Lengsin, BJ-TSA-9, C20orf42, BUB1, C10orf3 or HIFPH3 and is capable of binding to an HLA antigen and is recognized by a CTL;
(2) The peptide of (1) above, wherein the HLA antigen is HLA-A24 or HLA-A2 antigen;
(3) The peptide of (2) above, which comprises the amino acid sequence of any one of SEQ ID NOS: 13 to 201;
(4) A peptide which comprises an amino acid sequence which is the same as the amino acid sequence of any one of SEQ ID NOS: 13 to 31, 42 to 49, 59 to 78, 89 to 117, 158 to 165, 176 to 183, and 195 to 201 except that the amino acid at position 2 is substituted by tyrosine, phenylalanine, methionine or tryptophan, and/or the C terminal amino acid by phenylalanine, leucine, isoleucine, tryptophan or methionine, and is capable of binding to HLA-A24 antigen and is recognized by a CTL;
(5) A peptide which comprises an amino acid sequence which is the same as the amino acid sequence of any one of SEQ ID NOS:32 to 41, 50 to 58, 79 to 88, 118 to 157, 166 to 175, and 184 to 194 except that the amino acid at position 2 is substituted by leucine, methionine, valine, isoleucine or glutamine and/or the C terminal amino acid by valine or leucine, and is capable of binding to HLA-A2 antigen and is recognized by a CTL;

(6) An epitope peptide which comprises the peptide of any one of (1) to (5) above;
(7) A pharmaceutical composition which comprises the peptide of any one of (1) to (6) above and a pharmaceutically acceptable carrier;
(8) A nucleic acid which comprises a polynucleotide encoding the peptide of any one of (1) to (6) above;
(9) A pharmaceutical composition which comprises the nucleic acid of (8) above and a pharmaceutically acceptable carrier;
(10) A pharmaceutical composition which comprises Lengsin, BJ-TSA-9, C20orf42, BUB1, C10orf3 or HIFPH3 and a pharmaceutically acceptable carrier;

(11) The pharmaceutical composition of (10) above, wherein Lengsin comprises the amino acid sequence of SEQ ID NO: 2;
(12) The pharmaceutical composition of (10) above, wherein BJ-TSA-9 comprises the amino acid sequence of SEQ ID NO: 4;
(13) The pharmaceutical composition of (10) above, wherein C20orf42 comprises the amino acid sequence of SEQ ID NO: 6;
(14) The pharmaceutical composition of (10) above, wherein BUB1 comprises the amino acid sequence of SEQ ID NO: 8;
(15) The pharmaceutical composition of (10) above, wherein C10orf3 comprises the amino acid sequence of SEQ ID NO: 10;

(16) The pharmaceutical composition of (10) above, wherein HIFPH3 comprises the amino acid sequence of SEQ ID NO: 12;
(17) A pharmaceutical composition which comprises a nucleic acid comprising a polynucleotide encoding Lengsin, BJ-TSA-9, C20orf42, BUB1, C10orf3 or HIFPH3 and a pharmaceutically acceptable carrier;
(18) The pharmaceutical composition of (17) above, wherein the polynucleotide encoding Lengsin comprises the base sequence of SEQ ID NO: 1, or encodes the amino acid sequence of SEQ ID NO: 2;
(19) The pharmaceutical composition of (17) above, wherein the polynucleotide encoding BJ-TSA-9 comprises the base sequence of SEQ ID NO: 3, or encodes the amino acid sequence of SEQ ID NO: 4;
(20) The pharmaceutical composition of (17) above, wherein the polynucleotide encoding C20orf42 comprises the base sequence of SEQ ID NO: 5, or encodes the amino acid sequence of SEQ ID NO: 6;

(21) The pharmaceutical composition of (17) above, wherein the polynucleotide encoding BUB1 comprises the base sequence of SEQ ID NO: 7, or encodes the amino acid sequence of SEQ ID NO: 8;
(22) The pharmaceutical composition of (17) above, wherein the polynucleotide encoding C10orf3 comprises the base sequence of SEQ ID NO: 9, or encodes the amino acid sequence of SEQ ID NO: 10;
(23) The pharmaceutical composition of (17) above, wherein the polynucleotide encoding HIFPH3 comprises the base sequence of SEQ ID NO: 11, or encodes the amino acid sequence of SEQ ID NO: 12;
(24) A method of preparing an antigen presenting cell, wherein a cell having an antigen-presenting ability is brought into contact in vitro with any one of:
   (a) the peptide of any one of (1) to (6) above,
   (b) the nucleic acid of (8) above,
   (c) Lengsin, BJ-TSA-9, C20orf42, BUB1, C10orf3 or HIFPH3, and
   (d) a nucleic acid comprising a polynucleotide encoding Lengsin, BJ-TSA-9, C20orf42, BUB1, C10orf3 or HIFPH3;
(25) An antigen presenting cell prepared by the method of (24) above;

(26) A pharmaceutical composition which comprises the antigen presenting cell of (25) above and a pharmaceutically acceptable carrier;
(27) A method of inducing a CTL, wherein peripheral blood lymphocytes are brought into contact in vitro with any one of:
   (a) the peptide of any one of (1) to (6) above,
   (b) the nucleic acid of (8) above,
   (c) Lengsin, BJ-TSA-9, C20orf42, BUB1, C10orf3 or HIFPH3, and
   (d) a nucleic acid comprising a polynucleotide encoding Lengsin, BJ-TSA-9, C20orf42, BUB1, C10orf3 or HIFPH3;
(28) A CTL induced by the method of (27) above;
(29) A pharmaceutical composition which comprises the CTL of (28) above and a pharmaceutically acceptable carrier;
(30) The pharmaceutical composition of (7), (9) to (23), (26), or (29) above, which is used as an inducer of CTL;

(31) The pharmaceutical composition of (7), (9) to (23), (26), or (29) above, which is used as a cancer vaccine;
(32) An antibody which specifically binds to the peptide of any one of (1) to (5) above;
(33) An HLA monomer, HLA dimer, HLA tetramer or HLA pentamer which comprises the peptide of any one of (1) to (5) above and an HLA antigen;
(34) A reagent for detecting a CTL specific to a tumor antigen peptide derived from Lengsin, BJ-TSA-9, C20orf42, BUB1, C10orf3 or HIFPH3, which comprises as a component the HLA monomer, HLA dimer, HLA tetramer or HLA pentamer of
(33) above;
(35) A disease marker consisting of a polynucleotide and/or a complementary polynucleotide thereof, wherein the polynucleotide comprises at least 15 contiguous nucleotides from the base sequence of Lengsin, BJ-TSA-9, C20orf42, BUB1, C10orf3 or HIFPH3 gene;

(36) The disease marker of (35) above, which is used as a probe or primer for detecting cancer;
(37) The disease marker of (35) or (36) above, wherein the disease marker derived from Lengsin gene is used for lung adenocarcinoma, lung squamous cell carcinoma or gastric cancer;
(38) The disease marker of (35) or (36) above, wherein the disease marker derived from BJ-TSA-9 gene is used for leukemia, lung adenocarcinoma, lung squamous cell carcinoma, small cell lung cancer, oral cancer, gastric cancer, pancreas cancer or lymphoma;
(39) The disease marker of (35) or (36) above, wherein the disease marker derived from C20orf42 gene is used for lung squamous cell carcinoma, lung adenocarcinoma, liver cancer, gastric cancer, leukemia, malignant lymphoma tissues, rectal cancer, colon cancer or pancreas cancer;
(40) The disease marker of (35) or (36) above, wherein the disease marker derived from BUB1 gene is used for breast cancer, lung adenocarcinoma, lung squamous cell carcinoma, ovarian cancer, oral squamous cell carcinoma, renal cancer, large bowel cancer (colon cancer, rectal cancer), gastric cancer, pancreas cancer, liver cancer, leukemia, lymphoma or melanoma;

(41) The disease marker of (35) or (36) above, wherein the disease marker derived from C10orf3 gene is used for breast cancer, colon cancer, rectal cancer, renal cancer, gastric cancer, ovarian cancer, liver cancer, pancreas cancer, lung squamous cell carcinoma, lung adenocarcinoma, small cell lung cancer or melanoma;
(42) The disease marker of (35) or (36) above, wherein the disease marker derived from HIFPH3 gene is used for breast cancer, colon cancer, gastric cancer, renal cancer, pancreas cancer, liver cancer, lung adenocarcinoma or lung squamous cell carcinoma;
(43) A method for detecting cancer which comprises the following steps (a), (b) and (c):
   (a) allowing RNA prepared from a biological sample of a test subject or complementary polynucleotides transcribed therefrom to hybridize with the disease marker of any one of (35) to (42) above;
   b) detecting RNA prepared from the biological sample or complementary polynucleotides transcribed therefrom hybridized with the disease marker by using the disease marker as an indicator; and
   (c) determining whether or not the test subject has cancer based on the result of the detection in (b);
(44) The method of (43) above, wherein the test subject is determined to have cancer in the step (C) when the result of the detection from the test subject is compared with that from a healthy subject and the level of hybridization to the disease marker observed in the test subject is higher than that observed in the healthy subject;
(45) A disease marker for cancer which comprises an antibody specifically recognizing Lengsin, BJ-TSA-9, C20orf42, BUB1, C10orf3 or HIFPH3;

(46) The disease marker of (45) above, which is used as a probe for detecting cancer;
(47) A method for detecting cancer which comprises the following steps (a), (b) and (c):
   (a) allowing proteins prepared from a biological sample of a test subject to bind to the disease marker of (45) or (46) above;
   b) detecting proteins prepared from the biological sample or partial peptides derived therefrom bound to the disease marker by using the disease marker as an indicator; and
   (c) determining whether or not the test subject has cancer based on the result of the detection in (b);
(48) The method of (47) above, wherein the test subject is determined to have cancer in the step (C) when the result of the detection from the test subject is compared with that from a healthy subject and the level of binding to the disease marker observed in the test subject is higher than that observed in the healthy subject.

### Effects of the invention

The novel tumor antigen peptides, the nucleic acids encoding the same, and the like of the present invention can be useful as cancer vaccines. Further, the tumor antigen peptides are also useful as components of HLA tetramers and the like to detect CTLs.

### Best Mode for Carrying Out the Invention

Abbreviations for amino acids, (poly)peptides, (poly)nucleotides and the like used herein, follow rules of IUPAC-IUB (IUPAC-IUB Communication on Biological Nomenclature, Eur. J. Biochem., 138: 9 (1984)), "Guidelines for preparing the specification containing a base sequence or an amino acid sequence" (Japan Patent Office), and symbols commonly used in this field.

### 1) Protein of the present invention

The proteins of the present invention are Lengsin, BJ-TSA-9, C20orf42, BUB1, C10orf3 and HIFPH3.
The protein of the present invention Lengsin comprises the amino acid sequence of SEQ ID NO: 2 or an amino acid sequence similar to the aforementioned amino acid sequence. The protein Lengsin of the invention may be a protein originated from natural source (e.g., a lung adenocarcinoma cell line 1-87) or a recombinant protein. Here, the amino acid sequence of SEQ ID NO: 2 is registered with the GenBank database under Accession No. NM_016571, Accession No. NP_057655, and represents human Lengsin (Glutamate-ammonia ligase (glutamine synthase) domain containing 1, also reffered to as GLULD1). The human Lengsin was discloed in Non-patent literature 7 (Mol. Vis. Jun. 15;8:185-95 (2002)).

The protein of the present invention BJ-TSA-9 comprises the amino acid sequence of SEQ ID NO: 4 or an amino acid sequence similar to the aforementioned amino acid sequence. The protein BJ-TSA-9 of the invention may be a protein originated from natural source (e.g., a lung adenocarcinoma cell line 1-87) or a recombinant protein. Here, the amino acid sequence of SEQ ID NO: 4 is registered with the GenBank database under Accession No. NM_032899, Accession No. NP_116288, and represents human BJ-TSA-9 (Hypothetical protein MGC14128). The human BJ-TSA-9 was discloed in Non-patent literature 8 (Proc. Natl. Acad. Sci. U S A., 99(26):16899-903 (2002)).

The protein of the present invention C20orf42 comprises the amino acid sequence of SEQ ID NO: 6 or an amino acid sequence similar to the aforementioned amino acid sequence. The protein C20orf42 of the invention may be a protein originated from natural source (e.g., a colon cancer cell line SW480) or a recombinant protein. Here, the amino acid sequence of SEQ ID NO: 6 is registered with the GenBank database under Accession No. NM_017671, Accession No. NP_060141, and represents human C20orf42 (URP1, also referred to as Kindlerin). The human C20orf42 was discloed in Non-patent literature 9 (Biochim. Biophys. Acta 1637: 207-216 (2003)).

The protein of the present invention BUB1 comprises the amino acid sequence of SEQ ID NO: 8 or an amino acid sequence similar to the aforementioned amino acid sequence. The protein BUB1 of the invention may be a protein originated from natural source (e.g., a pancreas cancer cell line PUN) or a recombinant protein. Here, the amino acid sequence of SEQ ID NO: 8 is registered with the GenBank database under Accession No. NM_004336, Accession No. NP_004327, and represents human BUB1. The human BUB1 was discloed in a literature (Genomics 46:379-388(1997)).

The protein of the present invention C10orf3 comprises the amino acid sequence of SEQ ID NO: 10 or an amino acid sequence similar to the aforementioned amino acid sequence. The protein C10orf3 of the invention may be a protein originated from natural source (e.g., a lung adenocarcinoma cell line 1-87) or a recombinant protein. Here, the amino acid sequence of SEQ ID NO: 10 is registered with the GenBank database under Accession No. NM_018131, Accession No. NP_060601, and represents human C10orf3. The human C10orf3 was discloed in Non-patent literature 16 (Proc. Natl. Acad. Sci. U S A., Dec 24;99(26):16899-903 (2002)).

The protein of the present invention HIFPH3 comprises the amino acid sequence of SEQ ID NO: 12 or an amino acid sequence similar to the aforementioned amino acid sequence. The protein HIFPH3 of the invention may be a protein originated from natural source (e.g., a renal cancer cell line SMKTR-1) or a recombinant protein. Here, the amino acid sequence of SEQ ID NO: 12 is registered with the GenBank database under Accession No. NM_022073, Accession No. NP_071356, and represents human HIFPH3 (egl nine homolog 3, also referred to as EGLN3). The human HIFPH3 was discloed in Non-patent literature 17 (Cell 107: 43-54 (2001)).

Each of Lengsin, BJ-TSA-9, C20orf42, BUB1, C10orf3, and HIFPH3 is also referred herein as "the protein of the invention".

The "protein comprising the amino acid sequence of SEQ ID NO: 2, 4, 6, 8, 10 or 12" specifically includes a protein consisting of the amino acid sequence of SEQ ID NO: 2, 4, 6, 8, 10 or 12, and a protein consisting of an amino acid sequence which comprises the amino acid sequence of SEQ ID NO: 2, 4, 6, 8, 10 or 12 having an additional amino acid sequence(s) attached to the N and/or C terminus. "Additional amino acid sequence" may be the amino acid sequence derived from other structual genes than the proteins of the invention.

The "protein comprising an amino acid sequence similar to the amino acid sequence of SEQ ID NO: 2, 4, 6, 8, 10 or 12" specifically includes the following proteins (a) to (c) :
(a) a protein comprising an amino acid sequence which is the same as the amino acid sequence of SEQ ID NO: 2, 4, 6, 8, 10 or 12 except that one or more amino acids are deleted, substituted and/or added, and having an activity as a tumor antigen protein;
(b) a protein comprising an amino acid sequence having at least 70% sequence identity with the amino acid sequence of SEQ ID NO: 2, 4, 6, 8, 10 or 12, and having an activity as a tumor antigen protein;
(c) a protein being encoded by a polynucleotide capable of hybridizing to a complementary strand of a polynucleotide encoding the amino acid sequence of SEQ ID NO: 2, 4, 6, 8, 10 or 12 under stringent conditions, and having an activity as a tumor antigen protein.

Preferred examples include a protein consisting of an amino acid sequence similar to the amino acid sequence of SEQ ID NO: 2, 4, 6, 8, 10 or 12. Examples of such a protein include the proteins (a') to (c') below:
(a') a protein consisting of an amino acid sequence which is the same as the amino acid sequence of SEQ ID NO: 2, 4, 6, 8, 10 or 12 except that one or more amino acids are deleted, substituted and/or added, and having an activity as a tumor antigen protein;
(b') a protein consisting of an amino acid sequence having at least 70% sequence identity with the amino acid sequence of SEQ ID NO: 2, 4, 6, 8, 10 or 12, and having an activity as a tumor antigen protein;
(c') a protein being encoded by a polynucleotide capable of hybridizing to a complementary strand of a polynucleotide encoding the amino acid sequence of SEQ ID NO: 2, 4, 6, 8, 10 or 12 under stringent conditions, and having an activity as a tumor antigen protein.

The "protein comprising an amino acid sequence which is the same as the amino acid sequence of SEQ ID NO: 2, 4, 6, 8, 10 or 12 except that one or more amino acids are deleted, substituted and/or added" in (a) above refers to a protein produced artificially, that is, a modified (variant) protein, or an allele variant present in a living body, for example.
In this respect, there is no limitation regarding the number or position of modification (mutation) in the protein as far as the activity of the protein of the invention is maintained. Criteria based on which one can determine the number or position of the amino acid residue to be deleted, substituted and/or added without reducing the activity can be obtained using a computer program well known in the art, such as DNA Star software. For example, the number of mutation would typically be within 10%, preferably 5% of the total amino acid residues. Furthermore, the amino acid introduced by substitution preferably has similar characteristics to that to be substituted in view of retention of structure, which characteristics include polarity, charge, solubility, hydrophobicity, hydrophilicity, amphipathicity, and the like. For instance, Ala, Val, Leu, Ile, Pro, Met, Phe and Trp are classified into nonpolar amino acids; Gly, Ser, Thr, Cys, Tyr, Asn and Gln into non-charged amino acids; Asp and Glu into acidic amino acids; and Lys, Arg and His into basic amino acids. One of ordinary skill in the art can select an appropriate amino acid(s) falling within the same group on the basis of these criteria.

The "protein comprising an amino acid sequence having at least 70% sequence identity with the amino acid sequence of SEQ ID NO: 2, 4, 6, 8, 10 or 12" in (b) above includes a protein comprising an amino acid sequence having at least about 70%, preferably about 80%, more preferably about 90%, and further more preferably about 95% sequence identity with the amino acid sequence of SEQ ID NO: 2, 4, 6, 8, 10 or 12, and specifically, a protein consisting of a partial amino acid sequence of SEQ ID NO: 2, 4, 6, 8, 10 or 12.

The term "sequence identity" herein used refers to the identity and homology between two proteins. The "sequence identity" is determined by comparing two sequences aligned optimally over the sequence region to be compared. In this context, the optimum alignment of the proteins to be compared may have an addition or deletion (e.g., "gap"). The sequence identity can be calculated by preparing an alignment using, for example, Vector NTI, ClustalW algorithm (Nucleic Acid Res., 22 (22): 4673-4680(1994)). The sequence identity can be determined using software for sequence analysis, specifically, Vector NTI or GENETYX-MAC, or a sequencing tool provided by a public database. Such a public database is commonly available at Web site (http: / /www.ddbj.nig.ac.ip).

The "polynucleotide capable of hybridizing to a complementary strand of a polynucleotide encoding the amino acid sequence of SEQ ID NO: 2, 4, 6, 8, 10 or 12 under stringent conditions" in (c) above includes a polynucleotide comprising a base sequences having at least about 40%, preferably about 60%, more preferably about 70%, still more preferably about 80%, further more preferably about 90%, and most preferably about 95% sequence identity with a polynucleotide encoding the amino acid sequence of SEQ ID NO: 2, 4, 6, 8, 10 or 12. Specifically, a polynucleotide comprising a base sequence having at least about 40%, preferably about 60%, more preferably about 70%, still more preferably about 80%, further more preferably about 90%, and most preferably about 95% sequence identity with the base sequence of SEQ ID NO: 1, 3, 5, 7, 9 or 11 is exemplified. More specifically, a polynucleotide consisting of a partial sequence of the base sequence of SEQ ID NO: 1, 3, 5, 7, 9 or 11 is exemplified.

Hybridization can be conducted according to a method known per se or a method equivalent thereto, for example, that described in a fundamental text book such as "Molecular Cloning 2nd Edt. Cold Spring Harbor Laboratory Press (1989)", and the like. Also, it can be performed using a commercially available library according to the instructions attached thereto.
The "stringent conditions" herein used can be determined on the basis of the melting temperature (Tm) of nucleic acids forming a complex or a nucleic acid binding to a probe as described in literatures (Berger and Kimmel, 1987, "Guide to Molecular Cloning Techniques Methods in Enzymology", Vol. 152, Academic Press, San Diego Calif.; or "Molecular Cloning" 2nd Edt. Cold Spring Harbor Laboratory Press (1989), ibid.).

For example, hybridization can be carried out in a solution containing 6xSSC (20xSSC means 333 mM sodium citrate, 333 mM NaCl), 0.5% SDS and 50% formamide at 42°C, or in a solution containing 6xSSC (without 50% formamide) at 65°C.
Washing after the hybridization can be conducted under a condition around "1xSSC, 0.1% SDS, 37°C". The complementary strand preferably remains to be bound to the target forward strand when washed under such washing conditions. More stringent hybridization conditions may involve washing conditions of around "0.5xSSC, 0.1% SDS, 42°C" and still more stringent hybridization conditions around "0.1xSSC, 0.1% SDS, 65°C", although it is not limited thereto.

The protein of the invention has an activity as a tumor antigen protein. The term "activity as a tumor antigen protein" refers to an activity detected by a conventional assay for the activity of a tumor antigen protein. Specifically, it refers to the characteristics that a cell expressing Lengsin, BJ-TSA-9, C20orf42, BUB1, C10orf3 or HIFPH3 is recognized by a CTL, that is, the cell exhibits reactivity to a CTL, in other words, the protein of the present invention or antigen peptides derived therefrom activates or induces a CTL.

In this respect, the "cell" preferably expresses an HLA antigen. Accordingly, the "activity as a tumor antigen protein " more specifically refers to the characteristics that, when the protein of the present invention is expressed in a cell expressing an HLA antigen such as HLA-A24 or HLA-A2, a complex between a tumor antigen peptide originated from the protein of the invention and the HLA antigen is presented on the cell surface and consequently the cell is recognized by a CTL, in other words, a CTL is activated (induced).

The characteristics of the protein of the invention as mentioned above can be easily determined by a known method or a method equivalent thereto, such as ⁵¹Cr release assay (J. Immunol., 159: 4753, 1997), LDH release assay using LDH Cytotoxicity Detection Kit (Takara Bio, Inc.), measurement of cytokines, and the like. The detailed protocol of assay will hereinafter be illustrated.

First, a host cell such as 293-EBNA cell (Invitrogen) is co-transfected with an expression vector comprising a DNA encoding the protein of the invention and an expression vector comprising a DNA encoding an HLA antigen. The DNA encoding an HLA antigen includes a DNA encoding HLA-A24 antigen or HLA-A2 antigen. Examples of a DNA encoding HLA-A24 antigen include HLA-A2402 cDNA (Cancer Res., 55: 4248-4252 (1995), Genbank Accession No. M64740). Examples of DNA encoding HLA-A2 antigen include HLA-A0201 cDNA (GenBank Acc.No.M84379) .

The transfection as mentioned above can be conducted by Lipofectin method using lipofectamine reagent (GIBCO BRL), and the like. Then, a CTL restricted to the HLA antigen used is added and allowed to react, followed by measurement of various cytokines (for example, IFN-γ) produced by the activated (reacting) CTL by a method such as ELISA, for example. The CTL usable herein may be prepared by stimulating peripheral blood lymphocytes with the protein of the invention or established according to the method of Int. J. Cancer, 39, 390-396, 1987, N. Eng. J. Med, 333, 1038-1044, 1995, or the like.

The CTL-inducing activity of the protein of the invention can also be examined in vivo by an assay where a model animal for human is used (WO 02/47474; Int. J. Cancer. 100, 565-570 (2002)).
The protein of the present invention can be prepared by a method known per se that is used for purifying a protein from natural products (e.g., cancer cell lines) or by a method hereinafter described comprising culturing a transformant carrying a nucleic acid comprising a polynucleotide encoding the protein of the present invention.

### 2) Peptide Derived from the Protein of the Invention

The peptide of the present invention, which may be referred to as "the peptide of the invention" or "the tumor antigen peptide of the present invention", is a tumor antigen peptide which comprises a partial peptide of Lengsin, BJ-TSA-9, C20orf42, BUB1, C10orf3 or HIFPH3 and is capable of binding to an HLA antigen and is recognized by a CTL. Thus, the peptide of the present invention may comprise a peptide corresponding to any position of the amino acid sequence of the protein of the invention and being of any length, as long as the peptide comprises a part of the amino acid sequence of the protein of the invention as defined above and can form a complex with an HLA antigen that is recognized by a CTL.

The peptide of the invention can be identified by synthesizing a candidate peptide, which is a partial fragment of Lengsin, BJ-TSA-9, C20orf42, BUB1, C10orf3 or HIFPH3, and subjecting the candidate peptide to an assay to examine whether or not a CTL recognizes a complex between the candidate peptide and an HLA antigen, that is, whether or not the candidate peptide has the activity as a tumor antigen peptide.
Synthesis of the peptide can be conducted according to a method generally used in the field of peptide chemistry. Such a method can be found in literatures including Peptide Synthesis, Interscience, New York, 1966; The Proteins, Vol. 2, Academic Press Inc., New York, 1976; Peptide-Gosei, Maruzen, Inc., 1975; Peptide-Gosei no Kiso to Jikken, Maruzen, Inc., 1985; and Iyakuhin no Kaihatsu (Zoku), Vol. 14, Peptide-Gosei, Hirokawa-syoten, 1991.

The method for identification of the tumor antigen peptide of the present invention will hereinafter be described in detail.
The regularity (motif) in an amino acid sequence of a tumor antigen peptide that binds to an HLA antigen to be presented has been elucidated in relation to some HLA types such as HLA-A1, -A0201, -A0204, -A0205, -A0206, -A0207, - A11, -A24, -A31, -A6801, -B7, -B8, -B2705, -B37, -Cw0401, and -Cw0602. See, Immunogenetics, 41: p. 178, 1995, etc. For example, the motifs for HLA-A24 are known to have an amino acid sequence of 8 to 11 amino acids, wherein the amino acid at position 2 is tyrosine, phenylalanine, methionine or tryptophan, and the C-terminal amino acid phenylalanine, leucine, isoleucine, tryptophan or methionine (J. Immunol., 152, p3913, 1994, Immunogenetics, 41: p178, 1995. J. Immunol., 155 :p4307, 1994). As for motifs for HLA-A2, those listed in Table 1 are known (Immunogenetics, 41, p178, 1995, J. Immunol., 155: p4749, 1995).

**[Table 1]**

| HLA-A2 type | Amino acid at position 2 from N-terminus | Amino acid at C-terminus |
|---|---|---|
| HLA-A0201 | L, M | V, L |
| HLA-A0204 | L | L |
| HLA-A0205 | V, L, I, M | L |
| HLA-A0206 | V, Q | V, L |
| HLA-A0207 | L | L |

| | | |
|---|---|---|
| * All the peptides are 8 to 11 amino acids in length. | | |

Recently, it has become possible to search a peptide sequence expected to be capable of binding to an HLA antigen via the internet using BIMAS software; NIH (http://bimas.dcrt.nih.gov/molbio/hla_bind/).
As for the length of the peptide, analysis of antigen peptides binding to various HLA molecules revealed that it is generally about 8 to 14 amino acids (Immunogenetics, 41: 178, 1995). However, in the cases of HLA-DR, -DP, and -DQ, peptides consisting of 14 amino acids or more are also known.

It is easy to select a portion corresponding to the peptide from the amino acid sequence of the protein of the invention considering the motif. For example, a sequence expected to be capable of binding to an HLA antigen may be easily selected by means of BIMAS software. The peptide of the present invention can be identified by synthesizing the selected candidate peptide by the above-mentioned method, and examining whether or not the candidate peptide binds to an HLA antigen and is recognized by a CTL, that is, whether or not the candidate peptide has an activity as a tumor antigen peptide.
Specifically, identification can be done by the method descried in J. Immunol., 154, p2257, 1995. Thus, a candidate peptide is added to stimulate in vitro peripheral blood lymphocytes isolated from a human subject positive for an HLA antigen which is expected to present the candidate peptide. When a CTL specifically recognizing the HLA-positive cell pulsed with the candidate peptide is induced, the candidate peptide is possibly a tumor antigen peptide. Whether or not the induction of CTL occurs may be examined by, for example, measuring the amount of various cytokines (e.g., IFN-γ) produced by the CTL in response to the antigen-presenting cell using ELISA or the like. Alternatively, the induction of CTL can also be examined by ⁵¹Cr release assay wherein the cytotoxicity of a CTL against an antigen-presenting cell labeled with ⁵¹Cr is measured (Int. J. Cancer, 58: p317, 1994).

Furthermore, the induction of CTL can be examined by pulsing a cell such as 293-EBNA cell (Invitrogen) with a candidate peptide, wherein the cell has been introduced with an expression plasmid for cDNA encoding a type of HLA antigens expected to present the candidate peptide, reacting the cell with a CTL restricted to the HLA antigen of the aforementioned type that is expected to present the the candidate peptide, and measuring various cytokines (e.g., IFN-γ) produced by the CTL (J. Exp. Med., 187: 277, 1998).
Examples of the HLA antigen include HLA-A24 antigen and HLA-A2 antigen. To select an HLA-A24-restricted tumor antigen peptide, HLA-A2402 cDNA (Cancer Res., 55: 4248-4252 (1995), Genbank Accession No. M64740) can be used as the cDNA encoding the HLA antigen. To select an HLA-A2-restricted tumor antigen peptide, HLA-A0201 cDNA (GenBank Acc.No.M84379) can be used as the cDNA encoding the HLA antigen.

As for CTLs, in addition to those obtained by stimulating human peripheral blood lymphocytes with a peptide, CTLs established by a method described in literatures (Int. J. Cancer, 39, 390-396, 1987; N. Eng. J. Med, 333, 1038-1044, 1995) may be used:
The in vivo activity of the peptide of the present invention can be determined by an assay which uses an animal model for human (WO 02/47474, Int J. Cancer 100, 565-570 (2002)).

In the above case, the regularity (motif) of the sequence of a tumor antigen peptide is known; however, when the motif of a peptide is unknown, as is the case for HLA-B55 or HLA-A26, the tumor antigen peptide of the present invention can be identified according to the method described in, for example, WO97/46676 only if a CTL cell line capable of recognizing a complex between the HLA antigen and a tumor antigen peptide is available.

Specific examples of the peptide of the present invention include a partial peptide derived from Lengsin consisting of the amino acid sequence of SEQ ID NO: 2, BJ-TSA-9 consisting of the amino acid sequence of SEQ ID NO: 4, C20orf42 consisting of the amino acid sequence of SEQ ID NO: 6, BUB1 consisting of the amino acid sequence of SEQ ID NO: 8, C10orf3 consisting of the amino acid sequence of SEQ ID NO: 10, or HIFPH3 consisting of the amino acid sequence of SEQ ID NO: 12, and being capable of binding to an HLA antigen and being recognized by a CTL. Preferred examples include a peptide capable of binding to HLA-A24 or HLA-A2 antigen, considering the HLA antigen to which the peptide of the present invention binds. The length of the peptide may be preferably 8 to 14 amino acids, more preferably 8 to 11 amino acids.

Specifically, the peptide of the present invention includes a peptide comprising the amino acid sequence of any one of SEQ ID NOS: 13 to 201 and being capable of binding to an HLA antigen and being recognized by a CTL. The length of the peptide may be preferably 9 to 14 amino acids, more preferably 9 to 11 amino acids. More specifically, as an HLA-A24-binding tumor antigen peptide, a peptide consisting of any one of the amino acid sequences of SEQ ID NOS: 13 to 31, 42 to 49, 59 to 78, 89 to 117, 158 to 165, 176 to 183 and 195 to 201, being capable of binding to HLA-A24 antigen and being recognized by a CTL (see Tables 5, 7, 9, 11, 13 and 15 below) is exemplified. Preferably, a peptide consisting of the amino acid sequence of SEQ ID NO: 42, 43, 44, 45, 46, 47, 49, 59, 60, 61, 62, 63, 64, 65, 66, 67, 68, 69, 70, 71, 72, 73, 74, 75, 76, 77, 78, 158, 159, 160, 161, 15, 16, 21, 22, 23, 24, 25, 26, 27, 30, 195, 197, 198, 199, 200, 201, 177, 178, 179 or 183 is exemplified.
More preferably, a peptide consisting of the amino acid sequence of SEQ ID NO: 158, 22, 23, 26, 27, 198, 200, 201, 177, 178, 179 or 183 is exemplified.

Further, as an HLA-A2-binding tumor antigen peptide, a peptide consisting of any one of the amino acid sequences of SEQ ID NOS: 32 to 41, 50 to 58, 79 to 88, 118 to 157, 166 to 175 and 184 to 194, being capable of binding to HLA-A2 antigen and being recognized by a CTL is exemplified (see Tables 6, 8, 10, 12, 14 and 16 below).

In the scope of the present invention, the peptide of the present invention includes not only a peptide consisting of a part of the amino acid sequence of SEQ ID NO: 2, 4, 6, 8, 10 or 12 but also a variant (modified) peptide produced by partly modifying the aforementioned peptide, provided that the variant peptide has characteristics of being capable of binding to an HLA antigen and being recognized by a CTL. Specifically, a variant peptide comprising an amino acid sequence which is the same as the amino acid sequence of the peptide of the present invention consisting of a part of the amino acid sequence of Lengsin, BJ-TSA-9, C20orf42, BUB1, C10orf3 or HIFPH3, specifically the amino acid sequence of SEQ ID NO: 2, 4, 6, 8, 10 or 12, except that at least one amino acid modification has been introduced, and having an activity as a tumor antigen peptide, i.e. being capable of binding to an HLA antigen and being recognized by a CTL, falls within the scope of the present invention.

The "modification" of an amino acid residue means substitution, deletion and/or addition of an amino acid residue including addition to the N- and/or C-terminus of peptide, and is preferably substitution of an amino acid residue. When the modification involves substitution of an amino acid residue, the number or position of the amino acid residue(s) to be substituted can be selected arbitrarily as far as an activity as a tumor antigen peptide is maintained; however, it is preferred that the substitution involves 1 to several amino acid residues since tumor antigen peptides are generally about 8-14 amino acids in length as mentioned above.

The variant peptide of the present invention is preferably 8 to 14 amino acids in length (in the cases of HLA-DR, -DP, or -DQ, however, peptides consisting of 14 amino acids or more are acceptable).
As mentioned above, the motif in an antigen peptide that binds to an HLA antigen and is presented is known in regard to certain HLA types, such as HLA-A1, -A0201, -A0204, -A0205, -A0206, -A0207, -A11, -A24, -A31, -A6801, -B7, -B8, -B2705, -B37, -Cw0401 and -Cw0602. Further, it is possible to search for a peptide sequence expected to be able to bind to an HLA antigen via internet (http://bim.as.dcrt.nih.gov/molbio/hla_bind/). Thus, one can prepare the variant peptide above on the basis of the motif and the like.

For example, as hereinbefore described, the motif of an antigen peptide being capable of binding to HLA-A24 and being presented is known as a sequence characterized in that, in an 8 to 11 amino acids peptide, the amino acid at position 2 is tyrosine, phenylalanine, methionine or tryptophan, and the C terminal amino acid is phenylalanine, leucine, isoleucine, tryptophan or methionine (J. Immunol., 152: p3913, 1994; Immunogenetics, 41: p178, 1995; J. Immunol., 155: p4307, 1994). As for HLA-A2, the motif is known as a sequence characterized in that, in a 8 to 11 amino acids peptide, the amino acid at position 2 is leucine, methionine, valine, isoleucine or glutamine and the C terminal amino acid is valine or leucine (Immunogenetics, 41: p178, 1995; J. Immunol., 155: p4749, 1995). Furthermore, some peptide sequences that are expected to be able to bind to an HLA antigen are published via internet (httt:// bimas.dcrt.nih.gov/molbio/hla bind/). Amino acids having similar characteristics to those available for the motif above are also acceptable. Thus, the present invention includes a variant peptide comprising an amino acid sequence which is the same as the amino acid sequence of the peptide of the present invention except that an amino acid(s) at position(s) available for substitution in light of the motif (in the case of HLA-A24 and HLA-A2, position 2 and C-terminus) is substituted by another amino acid, preferably by an amino acid expected to provide a binding activity from the internet site as mentioned above, for example, and having an activity of binding to the HLA antigen and being recognized by a CTL.

More preferably, the present invention includes a variant peptide whose amino acid residue(s) at the aforementioned position(s) is substituted by another amino acid known to be available in light of the motif and having an activity as a tumor antigen peptite. Thus, in the case of HLA-A24-binding peptides as shown in SEQ ID NOS: 13 to 31, 42 to 49, 59 to 78, 89 to 117, 158 to 165, 176 to 183 and 195 to 201, examples of variant peptides include those comprising an amino acid sequence which is the same as the amino acid sequence of any one of SEQ ID NOS: 13 to 31, 42 to 49, 59 to 78, 89 to 117, 158 to 165, 176 to 183 and 195 to 201 except that the amino acid at position 2 is substituted by tyrosine, phenylalanine, methionine or tryptophan, and/or the C terminal amino acid by phenylalanine, leucine, isoleucine, tryptophan or methionine, and being capable of binding to HLA-A24 antigen and being recognized by a CTL. Above all, a peptide whose amino acid at position 2 is substituted by tyrosine is more preferred.

More preferably, the variant peptide consists of an amino acid sequence which is the same as the amino acid sequence of any one of SEQ ID NOS: 42, 43, 44, 45, 46, 47, 49, 59, 60, 61, 62, 63, 64, 65, 66, 67, 68, 69, 70, 71, 72, 73, 74, 75, 76, 77, 78, 158, 159, 160, 161, 15, 16, 21, 22, 23, 24, 25, 26, 27, 30, 195, 197, 198, 199, 200, 201, 177, 178, 179 and 183 except that the amino acid at position 2 is substituted by tyrosine, phenylalanine, methionine or tryptophan, and/or the C terminal amino acid by phenylalanine, leucine, isoleucine, tryptophan or methionine, and is capable of binding to HLA-A24 antigen and is recognized by a CTL. More preferably, the variant peptide consists of an amino acid sequence which is the same as the amino acid sequence of any one of SEQ ID NOS: 158, 22, 23, 26, 27, 198, 200, 201, 177, 178, 179 and 183 except that the amino acid at position 2 and/or the C terminal amino acid are substituted as mentioned above, and is capable of binding to HLA-A24 antigen and is recognized by a CTL.

In.the case of HLA-A2-binding peptides as shown in SEQ ID NOS: 32 to 41, 50 to 58, 79 to 88, 118 to 157, 166 to 175 and 184 to 194, a preferable variant peptide is that comprising an amino acid sequence which is the same as the amino acid sequence of any one of SEQ ID NOS: 32 to 41, 50 to 58, 79 to 88, 118 to 157, 166 to 175 and 184 to 194 except that the amino acid at position 2 is substituted by leucine, methionine, valine, isoleucine or glutamine and/or the C terminal amino acid by valine or leucine, and being capable of binding to HLA-A2 antigen and being recognized by a CTL.

The peptide of the present invention further includes an epitope peptide comprising the tumor antigen peptide of the present invention as mentioned above.
Recently, a peptide composed of multiple (plural) CTL epitopes (antigen peptides) ligated together ("epitope peptide") has been shown to induce CTLs efficiently. For example, it has been reported that a peptide (about 30-mer) composed of CTL epitopes originated from a tumor antigen protein PSA each restricted to HLA-A2-, -A3, -A11, or B53 ligated together induced in vivo CTLs specific for respective CTL epitopes (Journal of Immunology 1998, 161: 3186-3194).

In addition, a peptide (epitope peptide)composed of a CTL epitope and a helper epitope ligated together has been shown to induce a CTL efficiently. In this context, "helper epitope" means a peptide capable of activating CD4-positive T cells (Immunity., 1:751, 1994), and examples thereof include HBVc128-140 of hepatitis B virus origin, TT947-967 of tetanus toxin origin, and the like. CD4⁺ T cells activated with the helper epitope exert some activities including induction and maintenance of CTLs, and activation of effectors such as macrophages, and hence are considered to be important in the immunological anti-tumor response. As a specific example of the peptide composed of a helper epitope and a CTL epitope ligated together, it is reported that a DNA (minigene) composed of six kinds of HBV-derived HLA-A2-restricted antigen peptides, three kinds of HLA-A11-restricted antigen peptides and a helper epitope induced in vivo CTLs directed to the respective epitopes efficiently (Journal of Immunology 1999, 162: 3915-3925). Practically, a peptide composed of a CTL epitope (a tumor antigen peptide corresponding to position 280-288 of melanoma antigen gp100) and a helper epitope (tetanus toxin-derived T helper epitope) ligated has been subjected to clinical test (Clinical Cancer Res., 2001, 7:3012-3024).
Accordingly, the tumor antigen peptide of the present invention also includes a peptide (epitope peptide) composed of multiple epitopes including the peptide of the present invention ligated together and having an activity of inducing a CTL.

In this respect, the "epitope peptide" is defined as (1) a peptide composed of two or more CTL epitopes (tumor antigen peptides) ligated together, or (2) a peptide composed of a CTL epitope(s) and a helper epitope(s) ligated together, which is processed in an antigen-presenting cell(s) to give a tumor antigen peptide(s) then being presented by the cell(s) and induces a CTL(s).

When a CTL epitope is ligated to the peptide of the present invention, the CTL epitope may be that derived from the amino acid sequence of Lengsin as shown in SEQ ID NO: 2, BJ-TSA-9 as shown in SEQ ID NO: 4, C20orf42 as shown in SEQ ID NO: 6, BUB1 as shown in SEQ ID NO: 8, C10orf3 as shown in SEQ ID NO: 10 or HIFPH3 as shown in SEQ ID NO: 12 and being restricted to HLA-A1, -A0201, -A0204, -A0205, -A0206, -A0207, -A11, -A24, -A31, -A6801, -B7, -B8, -B2705, -B37,-B55, -Cw0401, -Cw0602, and the like. CTL epitopes derived from other tumor antigen proteins are also usable. Plural number of CTL epitopes can be ligated together, and the length of a CTL epitope may be about 8-14 amino acids based on the analysis of antigen peptides binding to various HLA molecules (Immunogenetics, 41: 178, 1995).
When the a helper epitope is ligated to the peptide of the present invention, the helper epitope may be the aforementioned HBVc128-140 of hepatitis B virus origin, TT947-967 of tetanus toxin origin, and the like. The helper epitope may be about 13-30 amino acids, preferably about 13-17 amino acids in length.

The peptide (epitope peptide) composed of multiple epitopes ligated together can be prepared by the aforementioned conventional method for peptide synthesis. It can also be prepared by a conventional method for DNA synthesis and genetic engineering on the basis of the sequence information of a polynucleotide encoding an epitope peptide composed of multiple epitopes ligated together. That is, an epitope peptide composed of multiple epitopes ligated together can be prepared by inserting a polynucleotide encoding the epitope peptide into a known expression vector, transforming a host cell with the resultant recombinant expression vector, culturing the transformant, and recovering the desired epitope peptide from the culture. These processes can be conducted according to, for example, a method described in literatures (Molecular Cloning, T. Maniatis et al., CSH Laboratory (1983), DNA Cloning, DM. Glover, IRL PRESS (1985)).
The epitope peptide produced as mentioned above, which is composed of multiple epitopes ligated together, can be examined for CTL-inducing activity in vitro by means of an assay as mentioned above, or in vivo by means of an assay described in WO02/47474 or Int J. Cancer. 100, 565-570 (2002) using a model animal for human.

Also, the amino group of the N-terminal amino acid or the carboxyl group of the C-terminal amino acid of the tumor antigen peptide of the present invention can be modified. The peptide undergone such modification also falls within the scope of the present invention.
The modification of the amino group of the N-terminal amino acid involves 1 to 3 groups selected from C₁₋₆ alkyl group, phenyl group, cycloalkyl group and acyl group, for example. The acyl group specifically includes C₁₋₆ alkanoyl group, C₁₋₆ alkanoyl group substituted by phenyl group, carbonyl group substituted by C₅₋₇ cycloalkyl group, C₁₋₆ alkylsulfonyl group, phenylsulfonyl group, C₂₋₆ alkoxycarbonyl group, alkoxycarbonyl group substituted by phenyl group, carbonyl group substituted by C₅₋₇ cycloalkoxy group, phenoxycarbonyl group, and the like.
The peptide modified at the carboxyl group of the C-terminal amino acid may be ester or amide form. The ester specifically includes C₁₋₆ alkyl ester, C₀₋₆ alkyl ester substituted by phenyl group, C₅₋₇ cycloalkyl ester, and the like. The amide specifically includes amide, amide substituted by one or two C₁₋₆ alkyl groups, amide substituted by one or two C₀₋₆ alkyl groups wherein the alkyl group is substituted by phenyl group, amide forming 5- to 7-membered azacycloalkane including nitrogen atom of amide group, and the like.

### 3) Nucleic acid of the present invention

The nucleic acid of the present invention specifically refers to
(1) a nucleic acid comprising the polynucleotide encoding Lengsin, BJ-TSA-9, C20orf42, BUB1, C10orf3 or HIFPH3, and
(2) a nucleic acid comprising the polynucleotide encoding the peptide of the present invention.

### (1) Polynucleotide encoding Lengsin, BJ-TSA-9, C20orf42, BUB1, C10orf3 or HIFPH3 and nucleic acid comprising the same

The polynucleotide encoding Lengsin, BJ-TSA-9, C20orf42, BUB1, C10orf3 or HIFPH3 can be cDNA or mRNA, cRNA or genomic DNA of various cells or tissues such as those originated from prostate cancer, or synthetic DNA. It may be either single- or double-stranded. Specifically, the polynucleotide includes the followings:
(a) a polynucleotide comprising the base sequence of SEQ ID NO: 1, 3, 5, 7, 9 or 11;
(b) a polynucleotide comprising a base sequence encoding the amino acid sequence of SEQ ID NO: 2, 4, 6, 8, 10 or 12; and
a polynucleotide comprising a base sequence similar to that of the polynucleotide (a) or (b).

In this respect, the base sequence of SEQ ID NO: 1 corresponds to the base sequence of human Lengsin gene which is registered with GenBank database under Accession No. NM_016571. The amino acid sequence of SEQ ID NO: 2 corresponds to that of human Lengsin which is registered with GenBank database under Accession No. NM_016571, Accession No. NP_057655. The human Lengsin gene was disclosed in Non-patent literature 7 (Mol. Vis. Jun. 15;8:185-95 (2002)).

The base sequence of SEQ ID NO: 3 corresponds to the base sequence of human BJ-TSA-9 gene which is registered with GenBank database under Accession No. NM_032899. The amino acid sequence of SEQ ID NO: 4 corresponds to that of human BJ-TSA-9 which is registered with GenBank database under Accession No. NM_032899, Accession No. NP_116288. The human BJ-TSA-9 gene was disclosed in Non-patent literature 8 (Proc. Natl. Acad. Sci. U S A., 99(26):16899-903 (2002)).

The base sequence of SEQ ID NO: 5 corresponds to the base sequence of human C20orf42 gene which is registered with GenBank database under Accession No. NM_017671. The amino acid sequence of SEQ ID NO: 6 corresponds to that of human C20orf42 which is registered with GenBank database under Accession No. NM_017671, Accession No. NP_060141. The human C20orf42 gene was disclosed in Non-patent literature 9 (Biochim. Biophys. Acta 1637: 207-216 (2003)).

The base sequence of SEQ ID NO: 7 corresponds to the base sequence of human BUB1 gene which is registered with GenBank database under Accession No. NM_004336. The amino acid sequence of SEQ ID NO: 8 corresponds to that of human BUB1 which is registered with GenBank database under Accession No. NM_004336, Accession No. NP_004327. The human BUB1 gene was disclosed in a literature (Genomics 46:379-388(1997)).

The base sequence of SEQ ID NO: 9 corresponds to the base sequence of human C10orf3 gene which is registered with GenBank database under Accession No. NM_018131. The amino acid sequence of SEQ ID NO: 10 corresponds to that of human C10orf3 which is registered with GenBank database under Accession No. NM_018131, Accession No. NP_060601. The human C10orf3 gene was disclosed in Non-patent literature 16 (Proc. Natl. Acad. Sci. U S A., Dec 24;99(26):16899-903 (2002)).

The base sequence of SEQ ID NO: 11 corresponds to the base sequence of human HIFPH3 gene which is registered with GenBank database under Accession No. NM_022073. The amino acid sequence of SEQ ID NO: 12 corresponds to that of human HIFPH3 which is registered with GenBank database under Accession No. NM_022073, Accession No. NP_071356. The human HIFPH3 gene was disclosed in Non-patent literature 17 (Cell 107: 43-54 (2001)).

The aforementioned (a) polynucleotide comprising the base sequence of SEQ ID NO: 1, 3, 5, 7, 9 or 11 and (b) polynucleotide comprising a base sequence encoding the amino acid sequence of SEQ ID NO: 2, 4, 6, 8, 10 or 12 specifically include a polynucleotide consisting of the base sequence of SEQ ID NO: 1, 3, 5, 7, 9 or 11 and a polynucleotide consisting of a base sequence encoding the amino acid sequence of SEQ ID NO: 2, 4, 6, 8, 10 or 12. Further example includes a polynucleotide consisting of a base sequence which contains the base sequence of SEQ ID NO: 1, 3, 5, 7, 9 or 11 or that encoding the amino acid sequence of SEQ ID NO: 2, 4, 6, 8, 10 or 12, to which an additional base sequence is added at the 5'- and/or 3'-terminus. "Additional base sequence" may be a base sequence encoding a structural gene other than Lengsin, BJ-TSA-9, C20orf42, BUB1, C10orf3 and HIFPH3.
Such a polynucleotide encoding Lengsin, BJ-TSA-9, C20orf42, BUB1, C10orf3 or HIFPH3 is characterized in that the protein encoded by the polynucleotide has an activity as a tumor antigen protein. The activity and methods of determining the same are described in "1) The Protein of the Present Invention".

A polynucleotide comprising the base sequence of SEQ ID NO: 1 can be cloned by screening a cDNA library derived from, for example, a lung adenocarcinoma cell line (such as 1-87) using an appropriate portion of the base sequence disclosed in GenBank Accession No. NM_016571 or herein disclosed in SEQ ID NO: 1 as a probe for hybridization or a primer for PCR.

A polynucleotide comprising the base sequence of SEQ ID NO: 3 can be cloned by screening a cDNA library derived from, for example, a lung adenocarcinoma cell line (such as 1-87) using an appropriate portion of the base sequence disclosed in GenBank Accession No. NM_032899 or herein disclosed in SEQ ID NO: 3 as a probe for hybridization or a primer for PCR.

A polynucleotide comprising the base sequence of SEQ ID NO: 5 can be cloned by screening a cDNA library derived from, for example, a colon cancer cell line (such as SW480 (ATCC Number:CCL-228)) using an appropriate portion of the base sequence disclosed in GenBank Accession No. NM_017671 or herein disclosed in SEQ ID NO: 5 as a probe for hybridization or a primer for PCR.

A polynucleotide comprising the base sequence of SEQ ID NO: 7 can be cloned by screening a cDNA library derived from, for example, a pancreas cancer cell line (such as PUN) using an appropriate portion of the base sequence disclosed in GenBank Accession No. NM_004336 or herein disclosed in SEQ ID NO: 7 as a probe for hybridization or a primer for PCR.

A polynucleotide comprising the base sequence of SEQ ID NO: 9 can be cloned by screening a cDNA library derived from, for example, a lung adenocarcinoma cell line (such as 1-87) using an appropriate portion of the base sequence disclosed in GenBank Accession No. NM_018131 or herein disclosed in SEQ ID NO: 9 as a probe for hybridization or a primer for PCR.

A polynucleotide comprising the base sequence of SEQ ID NO: 11 can be cloned by screening a cDNA library derived from, for example, a renal cancer cell line (such as SMKTR-1) using an appropriate portion of the base sequence disclosed in GenBank Accession No. NM_022073 or herein disclosed in SEQ ID NO: 11 as a probe for hybridization or a primer for PCR.

One ordinary skilled in the art can easily conduct the cloning according to the method described in Molecular Cloning 2nd Edt. Cold Spring Harbor Laboratory Press (1989), etc.

A polynucleotide comprising a base sequence similar to that of the polynucleotide (a) or (b) above specifically includes the followings:
(c) a polynucleotide capable of hybridizing to a complementary strand of the polynucleotide (a) or (b) under stringent conditions, which encodes a protein having an activity as a tumor antigen protein;
(d) a polynucleotide comprising a base sequence having at least 70% sequence identity with the polynucleotide (a) or (b), which encodes a protein having an activity as a tumor antigen protein; and
(e) a polynucleotide encoding a protein comprising an amino acid sequence which is the same as the amino acid sequence encoded by the polynucleotide (a) or (b) except that one or more amino acids are deleted, substituted and/or added, wherein the protein has an activity as a tumor antigen protein.

Preferred examples include a polynucleotide consisting of a base sequence similar to that of the polynucleotide (a) or (b) above. The polynucleotide consisting of a base sequence similar to that of the polynucleotide (a) or (b) above includes the polynucleotides (c') to (e') below:
(c') a polynucleotide capable of hybridizing to a complementary strand of the polynucleotide (a) or (b) under stringent conditions, which encodes a protein having an activity as a tumor antigen protein;
(d') a polynucleotide consisting of a base sequence having at least 70% sequence identity with the polynucleotide (a) or (b), which encodes a protein having an activity as a tumor antigen protein; and
(e') a polynucleotide encoding a protein consisting of an amino acid sequence which is the same as the amino acid sequence encoded by the polynucleotide (a) or (b) except that one or more amino acids are deleted, substituted and/or added, wherein the protein has an activity as a tumor antigen protein.

Examples of the "polynucleotide capable of hybridizing to a complementary strand of the polynucleotide (a) or (b) above under stringent conditions" include a polynucleotide comprising a base sequence having at least about 40%, preferably about 60%, more preferably about 70%, still more preferably about 80%, further more preferably about 90%, and most preferably about 95% sequence identity with the base sequence of the polynucleotide (a) or (b) above, and specifically, a polynucleotide consisting of a partial sequence of the polynucleotide (a) or (b) above.

Hybridization can be conducted according to a method known per se or a method equivalent thereto, for example, a method described in a fundamental text "Molecular Cloning 2nd Edt. Cold Spring Harbor Laboratory Press (1989)", and the like. Also, it can be performed using a commercially available library according to the instructions attached thereto.
The "stringent conditions" herein used can be determined on the basis of the melting temperature (Tm) of nucleic acids forming a complex or a nucleic acid binding to a probe as described in literatures (Berger and Kimmel, 1987, "Guide to Molecular Cloning Techniques Methods in Enzymology", Vol. 152, Academic Press, San Diego CA; or "Molecular Cloning" 2nd Edt. Cold Spring Harbor Laboratory Press (1989)).

For example, hybridization can be carried out in a solution containing 6xSSC (20xSSC corresponds to 333 mM sodium citrate, 333 mM NaCl), 0.5% SDS and 50% formamide at 42°C, or in a solution containing 6xSSC (without 50% formamide) at 65°C.
Washing after the hybridization can be conducted under a condition around "1xSSC, 0.1% SDS, 37°C". The complementary strand preferably remains to bind to the target forward strand when washed under such washing conditions. More stringent hybridization conditions may involve washing under the conditions of around "0.5xSSC, 0.1% SDS, 42°C " and still more stringent hybridization conditions around "0.1xSSC, 0.1% SDS, 65°C", although it is not limited thereto.

The "polynucleotide comprising a base sequence having at least 70% sequence identity with the polynucleotide of (a) or (b) above" includes a polynucleotide comprising a base sequence having at least about 70%, preferably about 80%, more preferably about 90%, and most preferably about 95% sequence identity with the base sequence of the polynucleotide of (a) or (b) above, and specifically, a polynucleotide consisting of a partial sequence of the polynucleotide of (a) or (b) above.

The term "sequence identity" herein used refers to identity or homology between two polynucleotides. The "sequence identity" is determined by comparing two sequences by aligning them optimally over the region corresponding to the sequence to be compared. In this context, The optimum alignment of the two polynucleotides to be compared may have an addition or deletion (e.g., "gap"). Such sequence identity can be calculated by preparing alignment using, for example, Vector NTI, ClustalW algorithm (Nucleic Acid Res., 22 (22): 4673-4680(1994)). The sequence identity can be determined using software for sequence analysis, specifically, Vector NTI or GENETYX-MAC, or a sequencing tool provided by a public database. Such a public database is commonly available at Web site (http: / /www.ddbj.nig.ac.ip).

A polynucleotide having such sequence identity can be prepared according to the aforementioned hybridization method, or conventional PCR reaction or a reaction for modifying a polynucleotide (deletion, addition or substitution) hereinafter described.

The "polynucleotide encoding a protein comprising an amino acid sequence which is the same as the amino acid sequence of the protein encoded by the polynucleotide (a) or (b) above except that one or more amino acids are deleted, substituted and/or added " includes a nucleic acid encoding a variant protein produced artificially or an allele variant present in a living body.
In this respect, there is no limitation regarding the number or position of amino acid modification (mutation) as far as the activity of the protein of the invention is maintained. Criteria based on which one can determine the number or position of the amino acid residue to be deleted, substituted and/or added without reducing the activity can be obtained using a computer program well known in the art, such as DNA Star software. For example, the number of mutation would typically be within 10%, preferably 5% of the total amino acid residues. Furthermore, the amino acid introduced by substitution preferably has similar characteristics such as polarity, charge, solubility, hydrophobicity, hydrophilicity, amphipathicity, and the like, to that to be removed in view of retention of structure. For instance, Ala, Val, Leu, Ile, Pro, Met, Phe and Trp are classified into nonpolar amino acids; Gly, Ser, Thr, Cys, Tyr, Asn and Gln into non-charged amino acids; Asp and Glu into acidic amino acids; and Lys, Arg and His into basic amino acids. One of ordinary skill in the art can select an appropriate amino acid(s) within the same group on the basis of these criteria.

The polynucleotide encoding such a variant protein may be prepared by various methods such as site-directed mutagenesis and PCR technique described in Molecular Cloning 2nd Edt., Cold Spring Harbor Laboratory Press (1989). It also can be prepared by a known method such as Gapped duplex or Kunkel method using a commercially available kit.

The polynucleotide encoding the protein of the invention as mentioned above encodes a protein having an activity as a tumor antigen protein. "Having an activity as a tumor antigen protein" means that the protein is positive in a conventional assay for the activity of a tumor antigen protein. Specifically, it refers to the characteristics that a cell expressing the polynucleotide encoding the protein of the invention is recognized by a CTL, that is, the cell exhibits reactivity to a CTL, in other words, the protein of the invention or a tumor antigen peptide derived therefrom activates or induces a CTL. The activity and the method of determination thereof are as described in "1) Protein of the present invention" above.

The nucleic acid comprising the polynucleotide of the present invention may be either single- or double-stranded and may be either DNA or RNA. When the polynucleotide of the present invention is double stranded, an expression vector for expressing the protein of the present invention can be constructed by incorporating the above-mentioned polynucleotide into an expression vector. Thus, the nucleic acid of the present invention encompasses a recombinant expression vector constructed by inserting a double-stranded polynucleotide of the present invention to an expression vector.

A suitable expression vector can be selected depending on the host to be used, purposes and the like, and includes plasmids, phage vectors, virus vectors, and the like.
When the host is Escherichia coli, the vector may be a plasmid vector such as pUC118, pUC119, pBR322 and pCR3; or a phage vector such as λZAPII and λgt11. When the host is yeast, the vector may be pYES2, pYEUra3 and the like. When the host is an insect cell, the vector may be pAcSGHisNT-A and the like. When the host is an animal cell, the vector may be a plasmid vector such as pCEP4, pKCR, pCDM8, pGL2, pcDNA3.1, pRc/RSV and pRc/CMV; or a virus vector such as retrovirus vector, adenovirus vector and adeno-associated virus vector.
The expression vector may optionally contain a factor(s) such as promoter capable of inducing expression, a gene encoding a signal sequence, a marker gene for selection and terminator.

Furthermore, the expression vector may contain an additional sequence for expressing the protein as a fusion protein with thioredoxin, His-tag, GST (glutathione S-transferase), or the like, so as to facilitate isolation and purification of the protein. The vector usable in such a case includes a GST fusion protein vector containing an appropriate promoter (lac, tac, trc, trp, CMV, SV40 early promoter and the like) that functions in a host cell, such as pGEX4T; a vector containing Tag sequence (Myc, His and the like) such as pcDNA3.1/Myc-His; and a vector capable of expressing a fusion protein with thioredoxin and His such as pET32a.
By transforming a host cell with the expression vector obtained in the above, a transformant containing the vector of the present invention can be prepared.

The host cell usable herein includes Escherichia coli, yeast, insect cells and animal cells. Examples of Escherichia coli include strains of E. coli K-12 such as HB101, C600, JM109, DH5α and AD494 (DE3). Examples of yeast include Saccharomyces cerevisiae. Examples of animal cells include L929, BALB/c3T3, C127, CHO, COS, Vero, Hela and 293-EBNA cells. Examples of insect cells include sf9.
Introduction of an expression vector into a host cell can be done using a conventional method suited for the respective host cell above. Specifically, it can be done with calcium phosphate method, DEAE-dextran method, electroporation method, or a method using lipid for gene transfer (Lipofectamine, Lipofectin; Gibco-BRL). Following the introduction, the cell is cultured in a conventional medium containing a selection marker, whereby the transformant containing the expression vector can be selected.

The protein of the invention can be produced by culturing the transformant under appropriate conditions. The resultant protein may be further isolated and purified according to standard biochemical procedures. The purification procedure includes salting out, ion exchange chromatography, absorption chromatography, affinity chromatography, gel filtration chromatography, and the like. When the protein of the present invention is expressed as a fusion protein with thioredoxin, His tag, GST, or the like, as mentioned above, it can be isolated and purified by an appropriate purification procedure making use of the characteristics of such a fusion protein or tag.

### (2) Polynucleotide encoding the peptide of the present invention and nucleic acid comprising the same

As mentioned above, a nucleic acid comprising a polynucleotide encoding the peptide of the present invention falls within the scope of the nucleic acid of the present invention.
The polynucleotide encoding the peptide of the present invention may be either DNA or RNA and single- or double-stranded. The polynucleotide encoding the peptide of the present invention can be easily prepared on the basis of information about the amino acid sequence of the peptide or DNA encoding the same. Specifically, it can be prepared by a conventional method such as DNA synthesis or amplification by PCR.

Specifically, the polynucleotide encoding the peptide of the present invention includes a polynucleotide encoding the epitope peptide as mentioned above.
The nucleic acid comprising the polynucleotide encoding the peptide of the present invention may be either single- or double-stranded and also either DNA or RNA. When the polynucleotide of the present invention is double-stranded, a recombinant expression vector for expressing the peptide (epitope peptide) of the present invention can be constructed by introducing the above-mentioned polynucleotide into an expression vector.
The expression vector, host cell, method for transforming a host cell, and the like herein used are similar to those described in (1) above.

### 4) Antigen-Presenting Cell of the Present Invention

An antigen-presenting cell can be prepared by bringing a cell having an antigen-presenting ability into contact in vitro with any one of the protein, peptide and nucleic acid of the present invention as mentioned above. Specifically, the present invention provides a method of preparing an antigen-presenting cell characterized in that a cell having an antigen-presenting ability isolated from a tumor patient is brought into contact in vitro with any one of the protein, peptide and nucleic acid of the present invention and the antigen presenting cell prepared thereby.

In this context, the "cell having an antigen-presenting ability" is not limited to a particular cell and may be any cell that expresses on the surface an HLA antigen capable of presenting the peptide of the present invention; however, dendritic cells known to have especially high antigen-presenting ability are preferred.
Further, any of the protein, peptide and nucleic acid of the present invention may be used for preparing the antigen-presenting cell of the present invention from a cell having an antigen-presenting ability.

The antigen-presenting cell of the present invention can be prepared by isolating, from a tumor patient, cells having an antigen-presenting ability, pulsing the cells in vitro with the protein or peptide of the present invention, and allowing the cells to present a complex between an HLA antigen and the peptide of the present invention (Cancer Immunol. Immunother., 46: 82, 1998; J. Immunol. 158: p1796, 1997; Cancer Res., 59:1184, 1999). When dendritic cells are used, the antigen-presenting cell of the present invention may be prepared, for example, by isolating lymphocytes from peripheral blood of a tumor patient using Ficoll method, removing non-adherent cells, incubating the adherent cells in the presence of GM-CSF and IL-4 to induce dendritic cells, and incubating and pulsing the dendritic cells with the protein or peptide of the present invention.
When the antigen-presenting cell of the present invention is prepared by introducing the nucleic acid of the present invention into the cell having an antigen-presenting ability, the nucleic acid may be in the form of DNA or RNA. In particular, DNA may be used according to the teaching in Cancer Res., 56:5672, 1996 or J. Immunol., 161: p5607, 1998, and RNA according to the teaching in J. Exp. Med., 184:p465, 1996, for example.

The antigen presenting cell of the present invention is characterized in that the cell presents a complex between the peptide of the present invention and an HLA antigen, and specifically includes an antigen presenting cell which is a dendritic cell and presents a complex between the peptide consisting of the amino acid sequence of SEQ ID NO: 42, 43, 44, 45, 46, 47, 49, 59, 60, 61, 62, 63, 64, 65, 66, 67, 68, 69, 70, 71, 72, 73, 74, 75, 76, 77, 78, 158, 159, 160, 161, 15, 16, 21, 22, 23, 24, 25, 26, 27, 30, 195, 197, 198, 199, 200, 201, 177, 178, 179 or 183 (preferably the amino acid sequence of 158, 22, 23, 26, 27, 198, 200, 201, 177, 178, 179 or 183) and HLA-A24 antigen on the cell surface. Such an antigen presenting cell can be prepared by isolating cells having an antigen-presenting ability from a HLA-A24⁺ prostate cancer patient, pulsing the cells in vitro with the peptide consisting of the amino acid sequence of SEQ ID NO: SEQ ID NO: 42, 43, 44, 45, 46, 47, 49, 59, 60, 61, 62, 63, 64, 65, 66, 67, 68, 69, 70, 71, 72, 73, 74, 75, 76, 77, 78, 158, 159, 160, 161, 15, 16, 21, 22, 23, 24, 25, 26, 27, 30, 195, 197, 198, 199, 200, 201, 177, 178, 179 or 183 (preferably the amino acid sequence of 158, 22, 23, 26, 27, 198, 200, 201, 177, 178, 179 or 183), and allowing the cells to present a complex between the peptide and HLA-A24 antigen.

### 5) CTL of the Present Invention

Any one of the protein, peptide and nucleic acid of the present invention can be used to induce a CTL in vitro when brought into contact with peripheral blood lymphocytes. Specifically, the present invention provides a method of inducing a CTL wherein peripheral blood lymphocytes from a tumor patient are brought into contact in vitro with any one of the protein, peptide and nucleic acid of the present invention and the CTL induced thereby.

For melanoma, adoptive immunotherapy has shown a therapeutic effect, wherein tumor-infiltrating T cells obtained from a patient were cultured ex vivo in large quantities and returned into the same patient (J. Natl. Cancer. Inst., 86: 1159, 1994). Further, in mouse melanoma, suppression of metastasis has been observed by stimulating splenocytes with a tumor antigen peptide TRP-2 in vitro to induce proliferation of a CTL specific to the tumor antigen peptide, and administering the CTL to a melanoma-grafted mouse (J. Exp. Med., 185:453, 1997). This resulted from the in vitro proliferation of a CTL that specifically recognizes the complex between an HLA antigen on antigen-presenting cells and the tumor antigen peptide. Accordingly, a therapeutic method comprising stimulating in vitro peripheral blood lymphocytes from a patient with the protein, peptide or nucleic acid of the present invention to proliferate a tumor-specific CTL, and returning the CTL into the patient is believed to be effective.

The CTL used in the adoptive immunotherapy can be prepared by isolating peripheral blood lymphocytes from a patient and stimulating the lymphocytes in vitro with the protein, peptide or nucleic acid of the present invention (Journal of Experimental Medicine 1999, 190:1669).

The CTL of the invention is characterized in that it is induced by bringing peripheral blood lymphocytes into contact in vitro with any one of the protein, peptide and nucleic acid of the present invention, and may be either a single CTL clone or CTL mixture (group) composed of various CTL clones. A specific example of such a CTL is that specifically recognizing a complex between the peptide consisting of the amino acid sequence of SEQ ID NO: 42, 43, 44, 45, 46, 47, 49, 59, 60, 61, 62, 63, 64, 65, 66, 67, 68, 69, 70, 71, 72, 73, 74, 75, 76, 77, 78, 158, 159, 160, 161, 15, 16, 21, 22, 23, 24, 25, 26, 27, 30, 195, 197, 198, 199, 200, 201, 177, 178, 179 or 183 and HLA-A24 antigen. More preferable example of such a CTL is that specifically recognizing a complex between the peptide consisting of the amino acid sequence of SEQ ID NO: 158, 22, 23, 26, 27, 198, 200, 201, 177, 178, 179 or 183 and HLA-A24 antigen.

### 6) Pharmaceutical composition of the present invention

The protein, peptide, nucleic acid, antigen presenting cell and CTL of the present invention as mentioned above can be an active ingredient of a pharmaceutical composition in an appropriate form for each substance. The pharmaceutical composition of the present invention can be an active ingredient of an inducer of CTL, that is, a cancer vaccine, which is described in detail below.

### (1) Inducer of CTL comprising the protein of the invention as an active ingredient

The protein of the present invention (Lengsin, BJ-TSA-9, C20orf42, BUB1, C10orf3, HIFPH3) has an activity of inducing CTLs and therefore, the protein of the present invention can be used as an active ingredient of a medicament for the treatment or prevention of tumor (cancer vaccine). Thus, the inducer of CTL comprising the protein of the present invention as an active ingredient exerts a therapeutic or preventive effect on tumor. The protein, when administered to a tumor patient, is incorporated by antigen-presenting cells and intracellularly degradated; the resultant tumor antigen peptide(s) generated by the intracellular degradation binds to an HLA antigen to form a complex; the complex is then presented on the surface of antigen-presenting cells; and a CTL specific for the complex efficiently proliferates in the body and destroys the tumor cells. In this way, treatment or prevention of tumor is achieved.

The inducer of CTL comprising the protein of the present invention as an active ingredient can be administered to any tumor patient who is positive for Lengsin, BJ-TSA-9, C20orf42, BUB1, C10orf3 or HIFPH3 protein.
Specifically, the inducer of CTL comprising Lengsin as an active ingredient can be used for the prevention or treatment of cancer (tumor) such as lung adenocarcinoma, lung squamous cell carcinoma or gastric cancer.
The inducer of CTL comprising BJ-TSA-9 as an active ingredient can be used for the prevention or treatment of cancer (tumor) such as leukemia, lung adenocarcinoma, lung squamous cell carcinoma, small cell lung cancer, oral cancer, gastric cancer, pancreas cancer or lymphoma.

The inducer of CTL comprising C20orf42 as an active ingredient can be used for the prevention or treatment of cancer (tumor) such as lung squamous cell carcinoma, lung adenocarcinoma, liver cancer, gastric cancer, leukemia, tissues of malignant lymphoma, rectal cancer, colon cancer or pancreas cancer.
The inducer of CTL comprising BUB1 as an active ingredient can be used for the prevention or treatment of cancer (tumor) such as breast cancer, lung adenocarcinoma, lung squamous cell carcinoma, ovarian cancer, oral squamous cell carcinoma, renal cancer, large bowel cancer (colon cancer, rectal cancer), gastric cancer, pancreas cancer, liver cancer, leukemia, lymphoma or melanoma.

The inducer of CTL comprising C10orf3 as an active ingredient can be used for the prevention or treatment of cancer (tumor) such as breast cancer, colon cancer, rectal cancer, renal cancer, gastric cancer, ovarian cancer, liver cancer, pancreas cancer, lung squamous cell carcinoma, lung adenocarcinoma, small cell lung cancer or melanoma.
The inducer of CTL comprising HIFPH3 as an active ingredient can be used for the prevention or treatment of cancer (tumor) such as breast cancer, colon cancer, gastric cancer, renal cancer, pancreas cancer, liver cancer, lung adenocarcinoma or lung squamous cell carcinoma.

The inducer of CTL comprising the protein of the present invention as an active ingredient may be administered as a mixture with, or together with, a pharmaceutically acceptable carrier, for example, an appropriate adjuvant, so that cellular immunity can be established effectively.

Examples of adjuvant applicable include those described in a literature (Clin. Microbiol. Rev., 7:277-289, 1994). Specifically, the followings are contemplated: a component derived from a microorganism or derivatives thereof, cytokines, a component derived from a plant or derivatives thereof, a component derived from a marine organism or derivatives thereof, mineral gels such as aluminium hydroxide, surfactants such as lysolecithin and Pluronic® polyols, polyanion, peptide, oil emulsion (emulsion preparation) and the like. In addition, liposomal preparations, particulate preparations in which the ingredient is bound to beads having a diameter of several µm, preparations in which the ingredient is attached to lipids, microsphere preparations, and microcapsules are also contemplated.

In this context, the "component derived from a microorganism or derivatives thereof" can be specifically classified into (1) killed bacteria, (2) Cell Wall Skeleton (hereinafter, "CWS") derived from bacteria, and (3) a particular component derived from a microorganism and derivatives thereof.
(1) Examples of the killed bacteria include powdery hemolytic streptococcus (e.g., Picibanil® , Chugai Co., Ltd.), cocktail of killed bacterium suspension (e.g., Broncasma Berna®, Sanwa Kagaku Kenkyusho Co., Ltd) or killed bacteria of Mycobacterium tuberculosis.
(2) Examples of CWS derived from bacteria include CWS from Microbacterium (e.g., Mycobacterium bovis CWS), CWS from Nocardia (e.g., Nocardia rubra CWS), Corynebacterium CWS.

(3) Examples of a particular component derived from a microorganism and derivatives thereof include microorganism-derived polysaccharides such as polysaccharides from Mycobacterium tuberculosis (e.g., Ancer®, Zeria Pharmaceutical Co., Ltd.); polysaccharides from Basidiomycetes (Lentinan®, Ajinomoto, Co., Ltd.,; Krestin®, Sankyo, Co., Ltd.; Basidiomycetes, Coriolus versicolor (Fr) Quel); muramyl dipeptide (MDP) associated compounds; lipopolysaccharides (LPS); lipid A (MPL) associated compounds; glycolipids trehalose dimycolate (TDM); bacterium DNA (e.g., CpG oligonucleotide); and derivatives thereof.
   These microorganism-derived components and derivatives thereof can be available from commercial source or can be produced and isolated according to the methods described in known literatures (e.g., Cancer Res., 33, 2187-2195 (1973); J. Natl. Cancer Inst., 48, 831-835(1972), J. Bacteriol., 94, 1736-1745 (1967); Gann, 69, 619-626 (1978), J. Bacteriol., 92, 869-879 (1966) or J. Natl. Cancer Inst., 52, 95-101 (1974)).

The term "cytokine", for example, refers to IFN-α, IL-12, GM-CSF, IL-2, IFN-γ, IL-18 or IL-15. The cytokine may be a product of nature or genetic engineering. When the cytokine is commercially available, one can pursue and use the same. Alternatively, cytokine can be prepared recombinantly by cloning a desired gene in a conventional manner on the basis of the base sequence registered with database such as GenBank, EMBL or DDBJ, ligating the gene into an appropriate expression vector, transforming a host cell with the resultant recombinant expression vector, and allowing the cell to express and produce the intended cytokine.

Examples of the "component derived from a plant or derivatives thereof" include saponin-derived component Quil A (Accurate Chemical & Scientific Corp), QS-21 (Aquila Biopharmaceuticals Inc.), or glycyrrhizin (SIGMA-ALDRICH, etc.).
Examples of the "component derived from a marine organism or derivatives thereof." include sponge-derived glycolipid α-galactosylceramide.
Examples of oil emulsion (emulsion preparation) include emulsion preparations of water-in-oil type (w/o), oil-in-water type (o/w) and water-in-oil-in-water type (w/o/w).

In the water-in-oil type (w/o) emulsion preparation, an active ingredient is dispersed in water used as the disperse phase. In the oil-in-water type (o/w) emulsion preparation, an active ingredient is dispersed in water used as the disperse medium. Further, in the water-in-oil-in-water type (w/o/w) emulsion preparation, an active ingredient is dispersed in water which is the most internal phase. Such emulsion preparations can be produced in accordance with the teaching in, for example, JP-A-8-985, JP-A-9-122476, or the like.

The "liposomal preparation" refers to a microparticle wherein an active ingredient is encapsulated in a liposome having a lipid bilayer structure in the water phase or within the lipid bilayer. Representative lipids for preparation of the liposome include phosphatidyl choline and sphingomyelin. Dicetyl phosphate, phosphatidic acid, phosphatidyl serine or the like that confers charge may also be added for stabilization of liposomes. The method of producing liposomes include ultrasonic method, ethanol injection method, ether injection method, reverse phase evaporation method, French press extraction method, and the like.

The "microsphere preparation" refers to a microparticle composed of a homogeneous polymer matrix wherein an active ingredient is dispersed in the matrix. The matrix can be composed of a biodegradable polymer such as albumin, gelatin, chitin, chitosan, starch, polylactic acid, polyalkyl cyanoacrylate, and the like. The microsphere preparation can be prepared by any of known methods without limitation, including those described in literatures (Eur. J. Pharm. Biopharm. 50:129-146, 2000; Dev. Biol. Stand. 92:63-78, 1998; Pharm. Biotechnol. 10:1-43, 1997, etc.).

The "microcapsule preparation" refers to a microparticle containing an active ingredient as a core substance which is enveloped with a film. The coating material used for the film includes a film-forming polymer such as carboxymethylcellulose, cellulose acetate phthalate, ethyl cellulose, gelatin, gelatin/acacia, nitrocellulose, polyvinyl alcohol, hydroxypropyl cellulose, and the like. The microcapsule preparation can be prepared by coacervation method, surface polymerization, and the like.
Administration may be achieved, for example, intradermally, subcutaneously, intramuscularly, or intravenously. Although the dosage of the protein of the present invention in the formulation to be administered may be adjusted as appropriate depending on, for example, the disease to be treated, the age and the body weight of the patient, it is usually within the range of 0.0001 - 1000 mg, preferably, 0.001 - 100 mg, more preferably 0.01 - 10mg, which can be administered once in every several days to every several months.

### (2) Inducer of CTL comprising a peptide of the present invention as an active ingredient

The peptide of the present invention has an activity of inducing a CTL. The induced CTL can exert an anti-tumor effect through cytotoxic action or production of lymphokines. Accordingly, the peptide of the present invention can be used as an active ingredient of a medicament for the treatment or prevention of tumor (cancer vaccine). When an inducer of CTL comprising the peptide of the present invention as an active ingredient is administered to a tumor patient, the peptide of the present invention is presented to an HLA antigen in antigen-presenting cells. Then, a CTL specific for the presented binding complex between the HLA antigen and the peptide of the present invention proliferates, which in turn destroys tumor cells. In this way, the treatment or prevention of tumor in a patient can be achieved.
The inducer of CTL comprising the peptide of the present invention as an active ingredient can be administered to any tumor patient who is positive for the protein of the invention. Specifically, it can be used for the prevention or treatment of cancer (tumor) as described in 6)-(1) above.

The inducer of CTL comprising the peptide of the present invention as an active ingredient may comprise as an active ingredient a single CTL epitope (peptide of the present invention) or a epitope peptide composed of the peptide of the present invention and other peptide(s) (CTL epitope or helper epitope) ligated together. Recently, an epitope peptide composed of multiple (plural) CTL epitopes (antigen peptides) ligated together has been shown to have an activity of inducing CTLs efficiently. For example, it has been reported that an epitope pepitde of approximately 30-mer composed of CTL epitopes each restricted to HLA-A2-, -A3,- -All or B53 originated from tumor antigen protein PSA ligated induced CTLs specific for respective CTL epitopes (Journal of Immunology 1998, 161: 3186-3194). In addition, it has been reported that an epitope peptide composed of a CTL epitope and a helper epitope ligated can induce a CTL efficiently. When the peptide of the present invention is administered in the form of an epitope peptide, the peptide is incorporated into antigen-presenting cells; then the antigen peptide generated by intracellular degradation binds to an HLA antigen to form a complex; the complex is presented on the surface of antigen-presenting cells in high density; a CTL specific for the complex efficiently proliferates in the body, and destroys tumor cells. In this way, treatment or prevention of tumor is achieved.

Specific examples of the inducer of CTL comprising the peptide of the present invention as an active ingredient include one comprising as an active ingredient the tumor antigen peptide consisting of the amino acid sequence of any one of SEQ ID NOS: 13 to 201. Preferable example of the inducer of CTL is one comprising as an active ingredient the peptide consisting of the amino acid sequence of any one of SEQ ID NOS: 42, 43, 44, 45, 46, 47, 49, 59, 60, 61, 62, 63, 64, 65, 66, 67, 68, 69, 70, 71, 72, 73, 74, 75, 76, 77, 78, 158, 159, 160, 161, 15, 16, 21, 22, 23, 24, 25, 26, 27, 30, 195, 197, 198, 199, 200, 201, 177, 178, 179 and 183. More preferable example of the inducer of CTL is one comprising as an active ingredient the peptide consisting of the amino acid sequence of any one of SEQ ID NOS: 158, 22, 23, 26, 27, 198, 200, 201, 177, 178, 179 and 183.

The inducer of CTL comprising the peptide of the present invention as an active ingredient may be administered in a mixture with, or together with, a pharmaceutically acceptable carrier, for example, an appropriate adjuvant, so that cellular immunity can be established effectively.
Examples of adjuvant applicable include those described in a literature (Clin. Microbiol. Rev., 7:277-289, 1994). Specifically, the followings are contemplated: a component derived from a microorganism or derivatives thereof, cytokines, a component derived from a plant or derivatives thereof, a component derived from a marine organism or derivatives thereof, mineral gels such as aluminium hydroxide, surfactants such as lysolecithin and Pluronic® polyols, polyanion, peptides, oil emulsion (emulsion preparation) and the like. In addition, liposomal preparations, particulate preparations in which the ingredient is bound to beads having a diameter of several µm, preparations in which the ingredient is attached to lipids, microsphere preparations, and microcapsules are also contemplated. Concrete examples of these adjuvants are the same as those descried in the above "6)-1) Inducer of CTL comprising the protein of the invention as an active ingredient".

Administration may be achieved, for example, intradermally, subcutaneously, intramuscularly, or intravenously. Although the dosage of the peptide of the present invention in the formulation to be administered may be adjusted as appropriate depending on, for example, the disease to be treated, the age and the body weight of the patient, it is usually within the range of 0.0001 - 1000 mg, preferably 0.001 - 1000 mg, more preferably 0.1 - 10 mg, which can be administered once in every several days to every several months.

### (3) Inducer of CTL comprising the nucleic acid of the present invention as an active ingredient

The nucleic acid of the present invention has an activity of inducing a CTL and thus can be an active ingredient of a medicament for the treatment or prevention of tumor (cancer vaccine). The inducer of CTL comprising the nucleic acid of the present invention as an active ingredient, when administered, can exert a therapeutic or preventive effect on tumor through the expression of the nucleic acid.

For example, when the nucleic acid of the present invention incorporated into an expression vector is administered to a tumor patient in the following manner, the tumor antigen protein is highly expressed in antigen-presenting cells. Thereafter, tumor antigen peptides generated by intracellular degradation form a complex with an HLA antigen; the complex is then presented on the surface of antigen-presenting cells in high density; and tumor-specific CTLs proliferate in the body efficiently and destroy tumor cells. In this way, treatment or prevention of tumor is achieved.
The inducer of CTL comprising the nucleic acid of the present invention as an active ingredient can be administered to any tumor patient who is positive for the protein of the invention. Specifically, it can be used for the prevention or treatment of cancer (tumor) as described in 6)-(1) above.

Administration and introduction of the nucleic acid of the present invention into cells may be achieved by using a viral vector or by other procedures (Nikkei-Science, April, 1994, pp. 20-45; Gekkan-Yakuji, 36(1), 23-48 (1994); Jikken-Igaku-Zokan, 12(15), 1994, and references cited therein).
The method of introducing a viral vector may comprises incorporation of the DNA of the present invention into a DNA or RNA virus such as retrovirus, adenovirus, adeno-associated virus, herpesvirus, vaccinia virus, poxvirus, poliovirus, or Sindbis virus, and introduction of the virus into cells. Above all, the method involving a retrovirus, adenovirus, adeno-associated virus, or vaccinia virus is particularly preferred.

Other than the above metnod, a method wherein an expression plasmid is directly injected intramuscularly (DNA vaccination), liposome method, Lipofectin method, microinjection, calcium phosphate method and electroporation are exemplified, and among them DNA vaccination and liposome method are particularly preferred.
The nucleic acid of the present invention can act as a medicament in practice in, for example, an in vivo method wherein the nucleic acid is directly introduced into the body, or an ex vivo method wherein the nucleic acid is introduced extracorporeally into a certain cell obtained from a human subject and the cell is reintroduced into the body of the subject (Nikkei-Science, April, 1994, pp. 20-45; Gekkan-Yakuji, 36(1), 23-48 (1994); Jikkenn-Igaku-Zokan, 12(15), 1994; and references cited therein). An in vivo method is more preferred.

In case of the in vivo method, administration can be effected through any appropriate route depending on the disease and symptom to be treated and other factors. For example, it may be administered via intravenous, intraarterial, subcutaneous, intracutaneous, intramuscular route, or the like. When administered in the in vivo method, the nucleic acid of the present invention may be formulated into a liquid preparation, and typically into an injectable form containing the nucleic acid of the present invention as an active ingredient, to which a pharmaceutically acceptable carrier may also be added, if necessary. As to a liposome or membrane-fused liposome (such as Sendai virus (HVJ)-liposomes) containing the nucleic acid of the present invention, a liposomal formulation in the form of suspension, frozen preparation, centrifugally-concentrated frozen preparation, or the like may be accepted.
Although the dosage of the nucleic acid of the present invention in the formulation to be administered may be adjusted as appropriate depending on, for example, the disease to be treated, the age and the body weight of the patient, it is usually, as the amount of polynucleotide in the nucleic acid, within the range of 0.0001 - 100 mg, preferably, 0.001 - 10 mg, which can be administered once in every several days to every several months.

Recently, a polynucleotide encoding an epitope peptide composed of multiple (plural) CTL epitopes (tumor antigen peptides) ligated or of a CTL epitope(s) and a helper epitope(s) ligated has been shown to induce CTLs in vivo efficiently. For example, it is reported that a DNA (minigene) encoding an epitope peptide composed of six kinds of HBV-originated HLA-A2-restricted antigen peptides, three kinds of HLA-All-restricted antigen peptides and a helper epitope ligated induced in vivo CTLs directed to the respective epitopes efficiently (Journal of Immunology 1999, 162: 3915-3925).
Accordingly, a polynucleotide prepared by ligating one or more polynucleotides each encoding the peptide of the present invention, and optionally other polynucleotide(s) encoding different peptide(s), can be an active ingredient of an inducer of CTL when introduced into an appropriate expression vector. Such an inducer of CTL may be applied in the same administration manner or form that described above.

### (4) Inducer of CTL comprising the antigen presenting cell of the present invention as an active ingredient

The antigen presenting cell of the present invention has an activity of inducing a CTL and thus can be an active ingredient of a medicament for the treatment or prevention of tumor (cancer vaccine). The inducer of CTL comprising the antigen presenting cell of the present invention as an active, ingredient can exert a therapeutic or preventive effect on tumor through administration of the antigen presenting cell to a tumor patient.
The inducer of CTL comprising the antigen presenting cell of the present invention as an active ingredient can be administered to any tumor patient who is positive for the protein of the invention. Specifically, it can be used for the prevention or treatment of cancer (tumor) as described in 6)-(1) above.

The inducer of CTL comprising the antigen presenting cell as an active ingredient preferably contains physiological saline, phosphate buffered saline (PBS), medium, or the like to stably maintain the antigen-presenting cell. It may be administered, for example, intravenously, subcutaneously, or intradermally. Dosage of the inducer is exemplified in the previous literature. Reintroduction of the inducer of CTL comprising the antigen-presenting cell as an active ingredient into a patient positive for the protein of the invention can cause efficient induction of a specific CTL in the body of the patient, and, result in the treatment of tumor.

### (5) Cancer vaccine comprising the CTL of the present invention as an active ingredient

The CTL of the present invention has a cytotoxic activity against tumor cells and thus can be an active ingredient of a medicament for the treatment or prevention of tumor (cancer vaccine).
The pharmaceutical composition comprising the CTL of the present invention as an active ingredient for treating or preventing tumor can be administered to any tumor patient who is positive for the protein of the invention. Specifically, it can be used for the prevention or treatment of cancer (tumor) as described in 6)-(1) above.
The pharmaceutical composition comprising the CTL of the present invention as an active ingredient for treating or preventing tumor preferably contains physiological saline, phosphate buffered saline (PBS), medium, or the like to stably maintain the CTL. It may be administered, for example, intravenously, subcutaneously, or intradermally. Reintroduction of the pharmaceutical composition comprising the CTL of the present invention as an active ingredient into a patient positive for the protein of the invention can cause promotion of the cytotoxic activity of CTLs against tumor cells in the body of the patient, followed by destruction of the tumor cells, and result in treatment of tumor.

### 7) Antibody against the peptide of the present invention

The present invention provides an antibody specifically binding to the peptide of the present invention. The antibody of the present invention is not limited in terms of the form, and may be a polyclonal or monoclonal antibody raised against the peptide of the present invention.
The antibody of the present invention may not be limited in any sense provided that it specifically binds to the peptide of the present invention as mentioned above, and the specific example is an antibody that specifically binds to a tumor antigen peptide consisting of the amino acid sequence of any one of SEQ ID NOS: 13 to 201, preferably the amino acid sequence of SEQ ID NO: 42, 43, 44, 45, 46, 47, 49, 59, 60, 61, 62, 63, 64, 65, 66, 67, 68, 69, 70, 71, 72, 73, 74, 75, 76, 77, 78, 158, 159, 160, 161, 15, 16, 21, 22, 23, 24, 25, 26, 27, 30, 195, 197, 198, 199, 200, 201, 177, 178, 179 or 183, more preferably, 158, 22, 23, 26, 27, 198, 200, 201, 177, 178, 179 or 183.

Methods of preparing an antibody are well known in the art and the antibody of the present invention can be prepared according to any one of these conventional methods (Current protocols in Molecular Biology edit. Ausubel et al. (1987) Publish. John Wiley and Sons. Section 11.12-11.13, Antibodies; A Laboratory Manual, Lane, H, D. et al., ed., Cold Spring Harber Laboratory Press, New York 1989).

Specifically, the antibody of the present invention can be obtained by immunizing a non-human animal such as rabbit using the peptide of the present invention (e.g., a peptide consisting of the amino acid sequence of any one of SEQ ID NOS: 42, 43, 44, 45, 46, 47, 49, 59, 60, 61, 62, 63, 64, 65, 66, 67, 68, 69, 70, 71, 72, 73, 74, 75, 76, 77, 78, 158, 159, 160, 161, 15, 16, 21, 22, 23, 24, 25, 26, 27, 30, 195, 197, 198, 199, 200, 201, 177, 178, 179 and 183) as an immunogen, and recovering the antibody from serum of the immunized animal in a conventional manner. When the antibody is monoclonal, it can be obtained by immunizing a non-human animal such as mouse with the peptide of the present invention (e.g., a peptide consisting of the amino acid sequence of any one of SEQ ID NOS: 42, 43, 44, 45, 46, 47, 49, 59, 60, 61, 62, 63, 64, 65, 66, 67, 68, 69, 70, 71, 72, 73, 74, 75, 76, 77, 78, 158, 159, 160, 161, 15, 16, 21, 22, 23, 24, 25, 26, 27, 30, 195, 197, 198, 199, 200, 201, 177, 178, 179 and 183), subjecting the resultant splenocyte to cell fusion with a myeloma cell to prepare a hybridoma cell, and recovering the antibody from the hybridoma cell (Current protocols in Molecular Biology edit. Ausubel et al. (1987) Publish. John Wiley and Sons. Section 11.4-11.11).

The antibody against the peptide of the present invention can also be produced while enhancing the immunological response using different adjuvants depending on the host. Examples of the adjuvants include Freund adjuvant; mineral gels such as aluminium hydroxide; surfactants such as lysolecithin and Pluronic® polyol, polyanion, peptides, oil emulsion, keyhole limpet hemocyanin and dinitorophenol; human adjuvants such as BCG (Bacille de Calmette-Guerin) and Corynebacterium.

As mentioned above, an antibody that recognizes the peptide of the present invention and an antibody that neutralizes the activity of the peptide may easily be prepared by immunizing an animal in a conventional manner. The antibody may be used in affinity chromatography, immunological diagnostic method, and the like. Immunological diagnostic method may be selected as appropriate from immunoblotting, radioimmunoassay (RIA), enzyme-linked immunosorbent assay (ELISA), fluorescent or luminescent assay, and the like. The immunological diagnostic method would be effective for diagnosing cancer which expresses the gene encodig the protein of the present invention.

### 8) HLA monomer, HLA dimer, HLA tetramer and HLA pentamer of the present invention

The present invention also provides an HLA monomer, HLA dimer, HLA tetramer or HLA pentamer comprising the tumor antigen peptide of the present invention and an HLA antigen.
In cancer immunotherapy, a significant indicator for selecting a patient highly responsive to a tumor antigen (tumor antigen peptide), monitoring the therapeutic effect, or evaluating the suitability to treatment can be obtained through examination of frequency or amount of CTL precursor cells directed to the tumor antigen (tumor antigen peptide) in a patient before initiation of treatment, or examination of frequency or amount of CTLs in a patient undergoing treatment with the tumor antigen (tumor antigen peptide). An HLA monomer, HLA dimer, HLA tetramer and HLA pentamer each comprising a tumor antigen peptide and an HLA antigen are useful as a reagent in detection of a CTL specific for the antigen (antigen peptide), specifically, in the measurement of frequency or amount of the CTL.

As used herein, the HLA tetramer refers to a tetramer prepared by biotinylating a complex composed of HLA antigen α-chain and β2-microglobulin associated with a peptide (antigen peptide) (HLA monomer), and allowing to bind to avidin for tetramerization (Science 279: 2103- 2106 (1998); and Science 274: 94-96 (1996)).
The HLA monomer is a monomer that is used in the preparation of the above-mentioned HLA tetramer and is formed by biotinylating an associate of HLA antigen α-chain, β-microglobulin and an antigen peptide.

The HLA dimer is a dimer prepared by fusing HLA antigen α-chain and Ig (immunoglobulin, for example, IgGl), and binding the resultant fusion to β2-microglobulin and an antigen peptide (Proc. Natl. Acad. Sci. USA 90: 6671-6675 (1993)). The CTL specific for the antigen peptide bound to the HLA dimer can be detected by, for example, allowing labeled anti-IgGl antibody to bind to the IgGl.
The HLA pentamer is a recently developed technique and refers to a pentamer comprising five molecules of a complex of an HLA antigen and an antigen peptide polymerized through Coiled-Coil domain. Since the complex of an HLA antigen and an antigen peptide can be labeled with fluorescence or the like, the analysis can be carried out by flow cytometry or the like similarly to the HLA tetramer (see, http://www.proimmune.co.uk/).

The HLA-monomer, dimer, tetramer and pentamer as mentioned above are all available by custom production from a manufacture such as ProImmune or BD Biosciences. At present, HLA tetramers and the like which comprise different antigen peptides are commercially available (Medical & Biological Laboratories Co., Ltd., etc.).

Examples of the HLA monomer, dimer, tetramer and pentamer of the present invention, specifically, include a HLA monomer, dimer, tetramer and pentamer each comprising the peptide consisting of the amino acid sequence of SEQ ID NO: 42, 43, 44, 45, 46, 47, 49, 59, 60, 61, 62, 63, 64, 65, 66, 67, 68, 69, 70, 71, 72, 73, 74, 75, 76, 77, 78, 158, 159, 160, 161, 15, 16, 21, 22, 23, 24, 25, 26, 27, 30, 195, 197, 198, 199, 200, 201, 177, 178, 179 and 183, preferably 158, 22, 23, 26, 27, 198, 200, 201, 177, 178, 179 and 183 and HLA-A24 antigen. Above all, an HLA tetramer or an HLA pentamer is preferred for detection of a CTL.

The HLA monomer, HLA tetramer and HLA pentamer are preferably labeled with fluorescence so that the bound CTL can be easily sorted out or detected by a known detection method such as flow cytometry, fluorescent microscopy, and the like. Examples include HLA monomers, tetramers and pentamers labeled with phycoerythrin(PE), fluorescein isothiocyanate (FITC), peridinyl chlorophyll protein (PerCP), allophycocyanin (APC), or the like.

Among HLA antigens which may be a component of the HLA monomer, dimer, tetramer and pentamer of the present invention, HLA-A24 antigen (α-chain of HLA-A24 antigen) can be cloned easily by a conventional method such as PCR on the basis of a known base sequence of HLA-A2402 disclosed in Genbank Accession No.M64740. HLA-A2 antigen (α-chain of HLA-A2 antigen) can be cloned easily by a conventional method such as PCR on the basis of a known base sequence of HLA-A0201 gene disclosed in Genbank Accession No.M84379.

The β2-microglobulin which is a component of the HLA monomer, dimer, tetramer and pentamer of the present invention is preferably originated from human. The human β2-microglobulin can be cloned easily by a conventional method such as PCR on the basis of a known base sequence of human β2-microglobulin disclosed in Genbank Accession No. AB021288.

The processes for preparing the HLA monomer, dimer, tetramer and pentamer are well known from the respective literatures mentioned above. The preparation will be hereinafter described briefly regarding the HLA tetramer of HLA-A24.
First, an appropriate host cell such as E. coli or a mammalian cell capable of expressing a protein is transformed with an HLA-A24 α-chain expression vector and a β2-microglobulin expression vector, and allowed to express them. E. coli (e.g., BL21) is preferably used here. The resultant HLA-A24 complex in a monomeric form and the peptide of the invention are then mixed to form a soluble HLA-peptide complex. The C-terminal sequence of HLA-A24 α-chain of the HLA-peptide complex is biotinylated with BirA enzyme. When the biotinylated HLA-peptide complex and fluorescently labeled avidin are mixed at the molar ratio of 4:1, an HLA tetramer is formed. It is preferred to purify the resulting protein in each step above by gel filtration or the like.

The HLA monomer, dimer, tetramer and pentamer described above may be used effectively as a detecting reagent for a CTL specific for a tumor antigen peptide derived from the protein of the invention.
The CTL-detecting reagent of the present invention can be used for the following purposes, for example.
1) To examine the frequency or amount of a CTL precursor for the tumor antigen peptide of the present invention before the initiation of treatment with the protein, peptide or nucleic acid of the present invention. In doing so, responsiveness of a patient to the tumor antigen peptide can be assessed.
2) To examine the frequency or amount of CTLs in a patient under treatment with the protein, peptide or nucleic acid of the present invention. In doing so, it becomes possible to conduct monitoring of a therapeutic effect, evaluation of suitability of treatment, confirmation of favorable progress of treatment, and the like.

Detection of a CTL can be carried out by, specifically, isolating a biological sample containing CTLs (e.g., PBMCs) from a subject patient, bringing the HLA tetramer or the like of the present invention into contact with the biological sample, and measuring the frequency or amount of the existing CTL specific for the peptide of the invention bound to the HLA tetramer by, for example, flow cytometry.

### 9) Disease marker of the invention

The present invention also provides a disease marker for cancer (tumor) making use of Lengsin, BJ-TSA-9, C20orf42, BUB1, C10orf3 or HIFPH3 gene, or a expression product thereof.

As described above, the inventors found that the expression levels and/or frequencies of Lengsin, BJ-TSA-9, C20orf42, BUB1, C10orf3 and HIFPH3 genes were significantly increased in cancer tissues compared to normal tissues. Antibodies recognizing those genes or expression products thereof (i. e., proteins) are , therefore, useful as disease markers for cancers expressing those genes.
Use of the antibody recognizing a polynucleotide of Lengsin, BJ-TSA-9, C20orf42, BUB1, C10orf3 or HIFPH3 gene or an expression product thereof (i. e., a protein) as a disease marker is explained hereinafter.

### (1) Disease marker for cancer and application thereof

### (1-1) Polynucleotide

As described above, human Lengsin, BJ-TSA-9, C20orf42, BUB1, C10orf3 and HIFPH3 genes are known in the art.
The present invention is, as already mentioned, based on the finding that the expression levels and/or frequencies of Lengsin, BJ-TSA-9, C20orf42, BUB1, C10orf3 and HIFPH3 genes were specifically increased in tissues of cancer patients compared to normal tissues, and also the idea that detection of presence or absence of expression, or expression levels, of those genes can provide specific determination of presence or absence of, severity of, or recovery from cancer and thus precise diagnosis of the cancer.
Accordingly, the present invention provides a polynucleotide useful as a tool (i. e., a disease marker) which allows diagnosis of presence or absence or severity of cancer in a test subject through detection of presence or absence of expression, or expression levels, of those genes in the test subject.

The "polynucleotide" herein used includes RNA and DNA and may be either single- or double-stranded. The "DNA" includes cDNA, genomic DNA and a synthetic DNA. The "RNA" includes total RNA, mRNA and a synthetic RNA. The disease marker of the present invention is characterized in that it consists of a polynucleotide and/or a complementary polynucleotide thereof, wherein the polynucleotide comprises at least 15 contiguous nucleotides from the base sequence of Lengsin, BJ-TSA-9, C20orf42, BUB1, C10orf3 or HIFPH3 gene.

Specifically, the disease marker of the present invention may consist of a polynucleotide and/or a complementary polynucleotide thereof, wherein the polynucleotide comprises at least 15 contiguous nucleotides from the base sequence of Lengsin gene (SEQ ID NO: 1), BJ-TSA-9 gene (SEQ ID NO: 3), C20orf42 gene (SEQ ID NO: 5), BUB1 gene (SEQ ID NO: 7), C10orf3 gene (SEQ ID NO: 9) or HIFPH3 gene (SEQ ID NO: 11).

In this context, the term "complementary polynucleotide (complementary strand, reverse strand)" refers to a polynucleotide which is complementary based on the base-pairing rules (e.g., A:T, G:C) to a full sequence of the polynucleotide consisting of any one of the base sequences of the above SEQ ID NOS or a partial sequence thereof comprising at least 15 contiguous nucleotides from any one of those base sequences, which is herein also referred to as "forward strand" for convenience. The "complementary strand" may be a sequence being completely complementary to a base sequence of a target forward strand, or a sequence being complementary enough to hybridize with the base sequence of the target forward strand under stringent conditions. The stringent conditions herein used can be determined on the basis of the melting temperature (Tm) of nucleic acids forming a complex or binding to a probe as described in literatures (Berger and Kimmel, 1987, "Guide to Molecular Cloning Techniques Methods in Enzymology", Vol. 152, Academic Press, San Diego CA). For example, washing after hybridization can be conducted under a condition around "1 x SSC, 0.1% SDS, 37°C in usual. The complementary strand preferably remains bound to the target forward strand when washed under such washing conditions. More stringent hybridization conditions may involve washing under the conditions of around "0.5 x SSC, 0.1% SDS, 42°C" and still more stringent hybridization conditions involve washing conditions of around "0.1 x SSC, 0.1% SDS, 65°C", although it is not limited thereto. Specifically, such a complementary strand may consist of a base sequence having a complete complementarity, or a homology of at least 90%, preferably 95% compared to a base sequence of a target forward strand.

The polynucleotide of a forward strand may have a full or partial sequence of the base sequence of Lengsin, BJ-TSA-9, C20orf42, BUB1, C10orf3 or HIFPH gene, or may be complementary to the base sequence of the above-mentioned complementary strand.
The polynucleotide of forward or complementary (reverse) strand as mentioned above can be used as a disease marker in either single-stranded or double-stranded form.

Specifically, the disease marker of the present invention may be a polynucleotide consisting of a base sequence (full sequence) of Lengsin, BJ-TSA-9, C20orf42, BUB1, C10orf3 or HIFPH3 gene, or a complementary sequence thereof. Alternatively, the disease marker may be a polynucleotide consisting of a partial sequence of the full sequence as mentioned above or a complementary sequence thereof as long as the polynucleotide selectively (specifically) recognizes Lengsin, BJ-TSA-9, C20orf42, BUB1, C10orf3 or HIFPH3 gene or a polynucleotide originated therefrom. The partial sequence may be a polynucleotide comprising at least 15 contiguous nucleotides selected as appropriate from the base sequence of the aforementioned full sequence or a complementary sequence thereof.

The term "selectively (specifically) recognizes" used herein means that, in the case of Northern blotting, Lengsin, BJ-TSA-9, C20orf42, BUB1, C10orf3 or HIFPH3 gene or a polynucleotide derived therefrom can be specifically detected and in the case of RT-PCR method, Lengsin, BJ-TSA-9, C20orf42, BUB1, C10orf3 or HIFPH3 gene or a polynucleotide derived therefrom is produced. However, the above definition is not restrictive and any criteria can be used as long as one ordinary skilled in the art can determine that the substance detected or a product generated by using the polynucleotide originates from any of those genes.

For example, the disease marker of the invention can be designed from the base sequence of Lengsin gene (SEQ ID NO: 1), BJ-TSA-9 gene (SEQ ID NO: 3), C20orf42 gene (SEQ ID NO: 5), BUB1 gene (SEQ ID NO: 7), C10orf3 gene (SEQ ID NO: 9) or HIFPH3 gene (SEQ ID NO: 11) by means of primer 3 (http://www.genome.wi.mit.edu/cgi-bin/primer/primer3.cgi) or a vector NTI (Infomax). Specifically, a candidate sequence obtained from the base sequence of Lengsin, BJ-TSA-9, C20orf42, BUB1, C10orf3 or HIFPH3 gene by using primer 3 or vector NTI software, or a sequence comprising at least a part of the candidate sequence, can be used as a primer or probe.
The disease marker of the present invention comprises at least 15 contiguous nucleotides in length as mentioned above, and depending on the application of the marker, the length can be appropriately selected and designated.

### (1-2) Polynucleotide as a probe or primer

Detection' (diagnosis) of cancer of the present invention is conducted by examining presence or absence of expression, or expression level, of at least one of Lengsin, BJ-TSA-9, C20orf42, BUB1, C10orf3 and HIFPH3 genes in a biological sample from a test subject. Here, the disease marker of the present invention may be used as a primer which specifically recognizes and amplifies RNA expressed from any of those genes or a polynucleotide derived therefrom, or as a probe for detecting specifically such RNA or a polypeptide derived therefrom.

When the disease maker of the present invention is used as a primer for detecting cancer (i. e., used in genetic diagnosis), the primer may comprise generally 15 to 100 bp, preferably 15 ro 50 bp, more preferably 15 to 35 bp in length. When used as a probe for detecting cancer, the probe may comprise generally 15 bp to 1 kp, preferably 100 bp to 1 kb in length.
The disease marker of the invention can be used as a primer or probe in a conventional manner in any of known methods for detecting specifically a particular gene such as Northern blotting, RT-PCR, in situ hybridization, and the like. Then, presence or absence of expression, or expression level, of Lengsin, BJ-TSA-9, C20orf42, BUB1, C10orf3 or HIFPH3 gene which is involved in cancer can be examined.

A sample to be examined may be total RNA prepared with a conventional method from a biological sample, such as a biopsy sample, of a target tissue of a test subject or polynucleotides prepared from the total RNA.
The expression level of Lengsin, BJ-TSA-9, C20orf42, BUB1, C10orf3 or HIFPH3 gene in a biological sample can be detected or quantified by using a DNA chip. Here, the disease marker of the invention may be used as a probe for the DNA chip (in the case of Gene Chip Human Genome U95 A,B,C,D,E (Affymetrix), used as a polynucleotide probe being 25bp in length). When a DNA chip containing the disease marker of the invention as its probe is hybridized with labeled-DNA or RNA prepared from RNA of a biological sample, the disease marker of the invention (i. e., the probe) and the labeled DNA or RNA form a complex. By detecting the complex by means of the label of the labeled DNA or RNA, presence or absence of expression, or expression level, of Lengsin, BJ-TSA-9, C20orf42, BUB1, C10orf3 or HIFPH3 gene can be examined.

The DNA chip may contain one or more of the disease markers of the invention which can bind to any of Lengsin, BJ-TSA-9, C20orf42, BUB1, C10orf3 and HIFPH3 genes. When the DNA chip contains more than one markers, presence or absence expression, or expression level, can be examined simultaneously for more than one gene in one biological sample.

The disease marker of the present invention is useful for diagnosis or detection of cancer, that is, diagnosis of presence or absence or severity of cancer. Specifically, diagnosis of cancer using the disease marker can be conducted by evaluating the difference in the expression level of Lengsin, BJ-TSA-9, C20orf42, BUB1, C10orf3 or HIFPH3 gene in a biological sample of a tissue between a test subject and a healthy subject.
Here, the difference between the expression levels of both subjects may be presence or absence of expression, or alternatively, when the expression is observed in both subjects, may be at least 1.5-fold, preferably 2-fold, more preferably 3-fold.

A polynucleotide derived from Lengsin gene is specifically useful as a disease marker for lung adenocarcinoma, lung squamous cell carcinoma or gastric cancer.
A polynucleotide derived from BJ-TSA-9 gene is specifically useful as a disease marker for leukemia, lung adenocarcinoma, lung squamous cell carcinoma, small cell lung cancer, oral cancer, gastric cancer, pancreas cancer or lymphoma.
A polynucleotide derived from C20orf42 gene is specifically useful as a disease marker for lung squamous cell carcinoma, lung adenocarcinoma, liver cancer, gastric cancer, leukemia, malignant lymphoma tissues, rectal cancer, colon cancer or pancreas cancer.

A polynucleotide derived from BUB1 gene is specifically useful as a disease marker for breast cancer, lung adenocarcinoma, lung squamous cell carcinoma, ovarian cancer, oral squamous cell carcinoma, renal cancer, large bowel cancer (colon cancer, rectal cancer), gastric cancer, pancreas cancer, liver cancer, leukemia, lymphoma or melanoma.
A polynucleotide derived from C10orf3 gene is specifically useful as a disease marker for breast cancer, colon cancer, rectal cancer, renal cancer, gastric cancer, ovarian cancer, liver cancer, pancreas cancer, lung squamous cell carcinoma, lung adenocarcinoma, small cell lung cancer or melanoma.
A polynucleotide derived from HIFPH3 gene is specifically useful as a disease marker for breast cancer, colon cancer, gastric cancer, renal cancer, pancreas cancer, liver cancer, lung adenocarcinoma or lung squamous cell carcinoma.

### (1-3) Antibody

The present invention provides an antibody specifically recognizing an expression product of Lengsin, BJ-TSA-9, C20orf42, BUB1, C10orf3 or HIFPH3 gene (i. e., protein Lengsin, BJ-TSA-9, C20orf42, BUB1, C10orf3 or HIFPH3) which can be used as a disease marker for cancer.
Specifically, the present invention provides an antibody which specifically recognizes Lengsin, BJ-TSA-9, C20orf42, BUB1, C10orf3 or HIFPH3 protein having the amino acid sequence of SEQ ID NO: 2, 4, 6, 8, 10, or 12, respectively.

The present invention is, as already mentioned, based on the finding that the expression levels and/or frequencies of Lengsin, BJ-TSA-9, C20orf92, BUB1, C10orf3 and HIFPH3 genes were specifically increased in tissues of cancer patients compared to normal cells or tissues and also the idea that detection of presence or absence of expression, or expression levels, of those genes can provide specific determination of presence or absence of, severity of, or recovery from cancer and thus precise diagnosis of the cancer.
Accordingly, the antibody is useful as a tool (i. e., a disease marker) which allows diagnosis of presence or absence or severity of cancer for a test subject through detection of presence or absence of expression, or expression level, of such a protein in the test subject.

As described above, human Lengsin, BJ-TSA-9, C20orf42, BUB1, C10orf3 and HIFPH3 proteins are known in the art.
The antibody of the present invention is not limited in terms of the form, and may be a polyclonal or monoclonal antibody raised against Lengsin, BJ-TSA-9, C20orf42, BUB1, C10orf3 or HIFPH3. Further, the antibody of the present invention includes that having an affinity to a polypeptide consisting of as least, generally 8, preferably 15, more preferably 20 contiguous amino acids from the amino acid sequence of any of those proteins.

Methods of preparing such an antibody are well known in the art and the antibody of the invention can be prepared according to any one of those conventional methods (Current protocols in Molecular Biology edit. Ausubel et al. (1987) Publish. John Wiley and Sons. Section 11.12-11.13). Specifically, when the antibody is polyclonal, it can be obtained by immunizing a non-human animal such as rabbit using as an immunogen Lengsin, BJ-TSA-9, C20orf42, BUB1, C10orf3 or HIFPH3 protein expressed in and purified from E coli in a conventional manner, or an oligopeptide having a part of the amino acid sequence of Lengsin, BJ-TSA-9, C20orf42, BUB1, C10orf3 or HIFPH3 synthesized in a conventional manner, and recovering the antibody from serum of the immunized animal in a conventional manner. When the antibody is monoclonal, it can be obtained by immunizing a non-human animal such as mouse with Lengsin, BJ-TSA-9, C20orf42, BUB1, C10orf3 or HIFPH3 protein expressed in and purified from E .coli in a conventional manner, or an oligopeptide having a part of the amino acid sequence of any of those proteins synthesized in a conventional manner, subjecting the resultant splenocyte to cell fusion with a myeloma cell to prepare a hybridoma cell, and recovering the antibody from the hybridoma cell (Current protocols in Molecular Biology edit. Ausubel et al. (1987) Publish. John Wiley and Sons. Section 11.4-11.11).

The protein Lengsin, BJ-TSA-9, C20orf42, BUB1, C10orf3 or HIFPH3 used as an immunogen in the preparation of the antibody can be obtained from the sequence of any of SEQ ID NOS 1, 3, 5, 7, 9 and 11 provided herein, through procedures such as DNA cloning, construction of an corresponding plasmid, transfection of the plasmid into a host cell, culture of the resulting transformant, and recovery of the protein from culture of the transformant. These procedures can be conducted by any one of methods known to those skilled in the art or described in a literature (Molecular Cloning, T. Maniatis et al., CSH Laboratory (1983), DNA Cloning, DM. Glover, IRL PRESS (1985)), and the like.

Specifically, the protein used as an immunogen for preparation of the antibody of the invention can be obtained by constructing a recombinant DNA providing expression of Lengsin, BJ-TSA-9, C20orf42, BUB1, C10orf3 or HIFPH3 gene in a selected host cell (i. e., an expression vector), transfecting the DNA into a host cell to provide a transformant, culturing the transformant, and recovering the objective protein from culture of the transformant. Also, a partial peptide of Lengsin, BJ-TSA-9, C20orf42, BUB1, C10orf3 or HIFPH3 can be prepared by a usual method for chemical (peptide) synthesis based on the amino acid sequence provided herein (SEQ ID NO: 2, 4, 6, 8, 10 or 12).

Here, Lengsin, BJ-TSA-9, C20orf42, BUB1, C10orf3 or HIFPH3 of the invention includes not only a protein of an amino acid sequence of SEQ ID NO: 2, 4, 6, 8, 10 or 12 but also a homolog thereof. The homolog includes a protein consisting of an amino acid sequence which is the same as the amino acid sequence of SEQ ID NO: 2, 4, 6, 8, 10 or 12 except that one or more amino acids are deleted, substituted and/or added, and having an immunological activity equivalent to that known for the protein.
The homolog having an immunological activity equivalent to that known for the protein is, for example, a protein capable of inducing a specific immunoreaction in an appropriate animal or cells thereof and specifically binding to an antibody against Lengsin, BJ-TSA-9, C20orf42, BUB1, C10orf3 or HIFPH3.

There is no limitation regarding the number or position of the amino acid mutation in the protein as far as the immunological activity of the protein is maintained. Criteria based on which one can determine the number or position of the amino acid residue to be deleted, substituted and/or added without reducing the immunological activity can be obtained using a computer program well known in the art, such as DNA Star software. For example, the number of mutation would typically be within 10%, preferably 5%, more preferably 1% of the total amino acid residues. The amino acid to be substituted is not limited as far as the resulting protein maintains an immunological activity equivalent to Lengsin, BJ-TSA-9, C20orf42, BUB1, C10orf3 or HIFPH3. The amino acid introduced by substitution preferably has similar characteristics to that to be substituted in view of retention of structure, wherein the characteristics include polarity, charge, solubility, hydrophobicity, hydrophilicity, amphipathicity, and the like. For instance, Ala, Val, Leu, Ile, Pro, Met, Phe and Trp are classified into nonpolar amino acids; Gly, Ser, Thr, Cys, Tyr, Asn and Gln into non-charged amino acids; Asp and Glu into acidic amino acids; and Lys, Arg and His into basic amino acids. One of ordinary skill in the art can select an appropriate amino acid(s) falling within the same group on the basis of these criteria.

The antibody of the present invention may be obtained by using an oligopeptide having a part of the amino acid sequence of Lengsin, BJ-TSA-9, C20orf42, BUB1, C10orf3 or HIFPH3. The oligopeptide for the preparation of an antibody is not necessary to possess a functional biological activity, but it is desired to possess a similar immunogenicity to the corresponding protein. Preferably, the oligopeptide has such an immunogenicity and consists of at least 8, preferably 15, more preferably 20 contiguous amino acids from the amino acid sequence of BJ-TSA-9, C20orf42, BUB1, C10orf3 or HIFPH3.

To prepare the antibody against the oligopeptide, various adjuvants may be administered to a host simultaneously with the immunogen so as to enhance the immunoreactivity. Examples of the adjuvants are, but not limited to, Freund adjuvants; mineral gels such as aluminium hydroxide; surfactants such as lysolecithin, Pluronic® polyol, polyanion, peptide, oil emulsion, keyhole limpet hemocyanin and dinitorophenol; human adjuvants such as BCG (Bacille de Calmette-Guerin) or Corynebacterium.
In addition, commercially available antibodies including anti-Bub1 antibody (Upstate), anti-C10orf3 antibody (Abnova), anti-C20orf42 antibody (Imgenex), and anti-HIFPH3 antibody (Bethyl) can be used in the present invention.

The antibody of the invention has a characteristic of specifically binding to Lengsin, BJ-TSA-9, C20orf42, BUB1, C10orf3 or HIFPH3, and therefore can specifically detect the protein (and homologs thereof) expressed in a tissue of a test subject. Thus, the antibody of the invention is useful as a probe for detecting presence or absence of expression, or expression level, of Lengsin, BJ-TSA-9, C20orf42, BUB1, C10orf3 or HIFPH3 in a tissue of a test subject.

Specifically, Lengsin, BJ-TSA-9, C20orf42, BUB1, C10orf3 or HIFPH3 can be detected by obtaining a biological sample of a target tissue of a patient by biopsy and the like, preparing a tissue extract or proteins therefrom in a conventional manner, and conducting detection by a known method such as Western blotting, ELISA, or the like using the antibody as a probe in a conventional manner.
Diagnosis of cancer may be provided by examining the level of at least one of Lengsin, BJ-TSA-9, C20orf42, BUB1, C10orf3 and HIFPH3 in a tissue of a test subject compared to that of a healthy subject. Difference of the protein level may be presence or absence of the protein, or may be at least 2-fold, preferably 3-fold.

The antibody specifically recognizing Lengsin is particularly useful as a disease marker for lung adenocarcinoma, lung squamous cell carcinoma or gastric cancer.
The antibody specifically recognizing BJ-TSA-9 is particularly useful as a disease marker for leukemia, lung adenocarcinoma, lung squamous cell carcinoma, small cell lung cancer, oral cancer, gastric cancer, pancreas cancer or lymphoma.
The antibody specifically recognizing C20orf42 is particularly useful as a disease marker for lung squamous cell carcinoma, lung adenocarcinoma, liver cancer, gastric cancer, leukemia, malignant lymphoma tissues, rectal cancer, colon cancer or pancreas cancer.

The antibody specifically recognizing BUB1 is particularly useful as a disease marker for breast cancer, lung adenocarcinoma, lung squamous cell carcinoma, ovarian cancer, oral squamous cell carcinoma, renal cancer, large bowel cancer (colon cancer, rectal cancer), gastric cancer, pancreas cancer, liver cancer, leukemia, lymphoma or melanoma.
The antibody specifically recognizing C10orf3 is particularly useful as a disease marker for breast cancer, colon cancer, rectal cancer, renal cancer, gastric cancer, ovarian cancer, liver cancer, pancreas cancer, lung squamous cell carcinoma, lung adenocarcinoma, small cell lung cancer or melanoma.
The antibody specifically recognizing HIFPH3 is particularly useful as a disease marker for breast cancer, colon cancer, gastric cancer, renal cancer, pancreas cancer, liver cancer, lung adenocarcinoma or lung squamous cell carcinoma.

### (2) Method for Detecting (or Diagnosing) Cancer

The present invention provides a method for detecting (or diagnosing) cancer which utilizes the disease marker of the invention as mentioned above.
Specifically, the detection method of the present invention comprises collecting a biological sample of a target tissue from a test subject by biopsy and the like, detecting and measuring the expression level (amount) of Lengsin, BJ-TSA-9, C20orf42, BUB1, C10orf3 or HIFPH3 gene, or that of a protein encoded by the gene, and detecting (or diagnosing) presence or absence of, or severity of, cancer. The detection (diagnosis) method of the present invention can be used to detect (or diagnose) whether or not a therapeutic agent administered to a caner patient for amelioration of cancer can actually provide improvement of the disease, and/or how much improvement the therapeutic agent can provide.

The method for detecting cancer of the present invention comprises the following steps (a), (b) and (c):
(a) bringing a biological sample prepared from a test subject into contact with the disease marker of the invention;
(b) measuring the expression level of Lengsin, BJ-TSA-9, C20orf42, BUB1, C10orf3 or HIFPH3 gene in the sample by using the disease marker as an indicator, and
(c) determining whether or not the test subject has cancer based on the result of (b).

The biological sample herein used may be a sample prepared from a target tissue of a test subject. Specifically, the biological sample may be a sample containing RNA prepared from the (target) tissue, a sample containing polynucleotides prepared from the RNA, or a sample containing proteins prepared from the target tissue. Such a sample containing RNA, polynucleotides, or proteins can be prepared by a conventional method after a part of a tissue of a test subject is collected by biopsy and the like
The diagnosis method of the present invention can be conducted as illustrated below depending on the biological sample to be examined.

### (2-1) When the biological sample to be examined is RNA

When the biological sample to be examined is RNA, detection of cancer can be conducted by a method comprising the following steps (a), (b) and (c):
(a) hybridizing the diseas'e marker(s) of the present invention (i. e., a polynucleotide having at least 15 contiguous nucleotides from the base sequence of Lengsin, BJ-TSA-9, C20orf42, BUB1, C10orf3 or HIFPH3 gene and/or a complementary polynucleotide thereof) with RNA prepared from a biological sample of a test subject or complementary polynucleotides transcribed therefrom;
b) detecting RNA prepared from the biological sample or complementary polynucleotides transcribed therefrom hybridized with the disease marker by using the disease marker as an indicator ; and
(c) determining whether or not the test subject has cancer based on the result of the detection in (b).

When RNA is to be examined, the detection (diagnosis) method of the invention can be achieved by detecting and measuring the expression level of Lengsin, BJ-TSA-9, C20orf42, BUB1, C10orf3 or HIFPH3 gene in the RNA. Specifically, the method can be carried out by a known method such as Northern blotting, RT-PCR, DNA chip analysis, in situ hybridization, or the like by using the disease marker(s) of the invention (i. e., a polynucleotide comprising at least 15 contiguous nucleotides from the base sequence of Lengsin, BJ-TSA-9, C20orf42, BUB1, C10orf3 or HIFPH3 gene and/or a polynucleotide complementary thereto) as a primer or probe.

In the case of Northern blotting, the disease marker of the invention is used as a probe, and allows detection and measurement of presence or absence of expression, or expression level, of Lengsin, BJ-TSA-9, C20orf42, BUB1, C10orf3 or HIFPH3 gene in RNA. For example, the disease marker of the invention (which is a complementary strand) is labeled with a radioisotope (32P, 33P, etc.: RI) or a fluorescent substance. Then, the labeled disease marker is hybridized with RNA prepared from a tissue of a test subject which is transferred onto a nylon membrane or the like in a conventional manner. After that, a duplex formed between the labeled disease marker (DNA) and the RNA may be detected by detecting and measuring the signal from the label (RI or fluorescent substance) with a radiation detector (BAS-1800II, Fuji Photo Film., Inc.) or a fluorescence detector. Alternatively, the disease marker (i.e., a probe DNA) may be labeled with AlkPhos Direct Labelling and Detection System (Amersham Pharmacia Biotech) in accordance with the manufacturer's protocol and hybridized with RNA prepared from a tissue of a test subject, and then the signal from the label on the disease marker may be detected and measured using Multi-biomeasure STORM860 (Amersham Pharmacia Biotech).

In the case of RT-PCR, the disease marker of the invention is used as a primer, and presence or absence of expression, or expression level, of Lengsin, BJ-TSA-9, C20orf42, BUB1, C10orf3 or HIFPH3 gene in RNA can be detected and measured. For example, cDNA is prepared from RNA derived from a tissue of a test subject in a conventional manner and the cDNA is hybridized with a pair of primers (a forward primer hybridizing to the above-mentioned cDNA being a reverse strand, and a reverse primer binding to the forward strand) prepared from the disease marker of the present invention in order to amplify a target region corresponding to Lengsin, BJ-TSA-9, C20orf42, BUB1, C10orf3 or HIFPH3 gene using the cDNA as a template. Then, PCR is conducted in a conventional manner and the resulting amplified DNA duplex is detected. The detection of the amplified DNA duplex can be achieved by, for example, a method wherein the PCR is conducted with a primer previously labeled with RI or a fluorescent substance and the resulting labeled DNA duplex is detected; a method wherein the resulting DNA duplex is transferred onto a nylon membrane or the like, and is then detected due to hybridization with a labeled disease marker that serves as a probe. The resulting labeled DNA duplex products can be measured using Agilent 2100 Bioanalyser (Yokogawa Analytical Systems Inc.) or the like. The measurement may also be achieved by preparing RT-PCR reaction solution using SYBR Green RT-PCR Reagents (Applied Biosystems) according to the manufacture's protocol, conducting the reaction using ABI PRISM 7700 Sequence Detection System (Applied Biosystems), and detecting the reaction products.

In the case of DNA chip analysis, for example, a DNA chip on which the disease marker of the invention is attached is prepared as a DNA probe (which is either single- or double-stranded), and the DNA chip is hybridized with cRNA prepared from RNA derived from a tissue of a test subject in a conventional manner. Then, a duplex formed by DNA and cRNA is detected by binding a labeled probe prepared from the disease marker of the invention to the duplex. A DNA chip capable of detecting and measuring the expression level of Lengsin, BJ-TSA-9, C20orf42, BUB1, C10orf3 or HIFPH3 gene may be used for the present invetion. An example of such a DNA chip is Gene Chip Human Genome U95 A, B, C, D, E (Affymetrix). Detection and measurement of the expression level of Lengsin, BJ-TSA-9, C20orf42, BUB1, C10orf3 or HIFPH3 gene in RNA of a test subject using such a DNA chip is described in Examples below.

### (2-2) When the biological sample to be examined is proteins

When the biological sample to be examined is proteins, the detection (diagnosis) method of the invention is conducted by detecting and measuring the level (amount) of Lengsin, BJ-TSA-9, C20orf42, BUB1, C10orf3 or HIFPH3 in a biological sample. Specifically, the method may be comprises the following steps (a), (b) and (c):
(a) allowing proteins prepared from a biological sample of a test subject to bind to the disease marker of the present invention being an antibody (i. e., the antibody recognizing Lengsin, BJ-TSA-9, C20orf42, BUB1, C10orf3 or HIFPH3);
b) detecting proteins prepared from the biological sample or partial peptides derived therefrom bound to the disease marker by using the disease marker as an indicator; and
(c) determining whether or not the test subject has cancer based on the result of the detection in (b).

Specifically, the method may be a known method such as Western blotting for detecting and measuring Lengsin, BJ-TSA-9, C20orf42, BUB1, C10orf3 or HIFPH3 by using the disease marker of the invention being an antibody (i. e., the antibody specifically recognizing Lengsin, BJ-TSA-9, C20orf42, BUB1, C10orf3 or HIFPH3).
Western blotting can be carried out by using the antibody, that is, the disease marker of the invention, as the first antibody and, as the second antibody which binds to the first antibody, an antibody labeled with a radio isotope such as ¹²⁵I, fluorescent substance, or the like, and detecting and measuring the signal from the radio isotope or fluorescent substance in a resulting labeled complex using a radiation detector (BAS-1800II, Fuji Photo Film., Inc., etc), a fluorescence detector, or the like. Alternatively, after using the disease marker of the invention as the first antibody, detection and measurement may be carried out with ECL Plus Western Blotting Detection System (Amersham Pharmacia Biotech) according to the manufacture's protocol, and with Multi-biomeasure STORM860 (Amersham Pharmacia Biotech).

### (2-3) Diagnosis of cancer

Diagnosis of cancer can be carried out by comparing the expression level of Lengsin, BJ-TSA-9, C20orf42, BUB1, C10orf3 or HIFPH3 gene, or the expression level (or amount) of Lengsin, BJ-TSA-9, C20orf42, BUB1, C10orf3 or HIFPH3 protein in a tissue of a test subject with that in a normal tissue and assessing the difference between the two.

A biological sample (RNA or protein) collected or prepared from a normal tissue to be required can be obtained from a tissue of a subject without cancer or from a non-cancerous, normal tissue of a cancer patient, by biopsy or by collecting a postmortem tissue with his consent. The "subject without cancer" means a subject being at least asymptomatic, preferably not diagnosed as cancer as a result of any other diagnosis method such as X-ray examination. The "subject without cancer" may be herein referred to as a "healthy subject" simply.

Comparison of the expression level of the gene or protein in a tissue of a test subject with that in a normal tissue (i. e., a tissue of a subject without cancer or a non-cancerous, normal tissue of a cancer patient) can be carried out by measuring in parallel biological samples of both test and healthy subjects. The expression level of the gene or protein in a healthy subject is not necessarily that measured in parallel and, an average value or a statistical median value of the expression level of Lengsin, BJ-TSA-9, C20orf42, BUB1, C10orf3 or HIFPH3 gene or protein which is determined by a measurement of plural (at least 2, preferably equal to or more than 3, more preferably equal to or more than 5) normal tissue samples under a constant condition may be used.

The test subject is determined as having cancer when the expression level of Lengsin, BJ-TSA-9, C20orf42, BUB1, C10orf3 or HIFPH3 gene or the expression product thereof, that is, Lengsin, BJ-TSA-9, C20orf42, BUB1, C10orf3 or HIFPH3 protein in the tissue of the test subject is 2-fold, preferably 3-fold higher than that of a healthy subject. The test subject is determined as having caner or suspected to have caner when the expression level of the gene or protein in the test subject is higher than that in a healthy subject.

Specifically, the test subject is suspected to have cancer when the expression level of Lengsin gene or protein, among the genes and proteins above, is higher in lung or stomach of the test subject than that in a corresponding normal tissue.
Also, the test subject is suspected to have cancer when the expression level of BJ-TSA-9 gene or protein, is higher in leukocytes, lung, oral cavity, stomach, pancreas or lymph node of the test subject than that in a corresponding normal tissue.
Also, the test subject is suspected to have cancer when the expression level of C20orf42 gene or protein, is higher in lung, liver, stomach, leukocyte, lymph node, rectum, colon or pancreas of the test subject than that in a corresponding normal tissue.

Also, the test subject is suspected to have cancer when the expression level of BUB1 gene or protein, is higher in mammary gland, lung, ovary, oral cavity, kidney, large intestine (colon, rectum), stomach, pancreas, liver, leukocyte, lymph node or skin of the test subject than that in a corresponding normal tissue.
Also, the test subject is suspected to have cancer when the expression level of C10orf3 gene or protein, is higher in mammary gland, colon, rectum, kidney, stomach, ovary, liver, pancreas, lung or skin of the test subject than that in a corresponding normal tissue.
Also, the test subject is suspected to have cancer when the expression level of HIFPH3 gene or protein, is higher in mammary gland, colon, stomach, kidney, pancreas, liver, lung of the test subject than that in a corresponding normal tissue.

The present invention is specifically explained by the following examples, but the examples should not be deemed to limit the present invention in any sense.

### Example 1

### Preparation of total RNA from human tissue samples and human caner cell lines

Total RNA was prepared by a conventional method from samples of cancer and normal tissues of human major organs as follows: lung adenocarcinoma (57 samples), normal lung (derived from lung adenocarcinoma patients) (46 samples), lung squamous cell carcinoma (48 samples), normal lung (derived from lung squamous cell carcinoma patients) (18 samples), colon cancer (108 samples), normal colon (derived from colon cancer patients) (114 samples), rectal cancer (43 samples), normal rectum (derived from rectal cancer patients) (31 samples), gastric cancer (38 samples), normal stomach (derived from gastric cancer patients) (13 samples), breast cancer (237 samples), normal breast (derived from breast cancer patients) (39 samples), ovarian cancer (37 samples), normal ovarian (derived from ovarian cancer patients) (5 samples), liver cancer (19 samples), normal liver (derived from liver cancer patients) (8 samples), renal cancer (89 samples), normal kidney (derived from renal cancer patients) (64 samples), pancreas cancer (55 samples), normal pancreas (derived from pancreas cancer patients) (16 samples), leukemia (6 samples), lymphoma (90 samples), normal bone marrow (2 samples), melanoma (10 samples), and normal skin (72 samples).

### Example 2

### DNA chip analysis

Using the total RNA prepared from the samples as described in Example 1, DNA chip analysis was performed. For the DNA chip analysis, Gene Chip Human Genome U133 set (Affymetrix) was used. Specifically, the analysis comprised the following steps: (1) preparation of cDNA from the total RNA, (2) preparation of labeled cRNA from the cDNA, (3) fragmentation of the labeled cRNA, (4) hybridization of the fragmented cRNA with a probe array, (5) staining of the probe array, (6) scanning of the probe array, and (7) analysis of gene expression.

### (1) Preparation of cDNA from total RNA

A mixture containing each total RNA (10 µg) prepared in Example 1 and T7-(dT)24 primer (Amersham) (100 pmol) (11 µl) was heated at 70°C for 10 min and cooled on ice. After the cooling, 5x First Strand cDNA Buffer (4 µL), 0.1 M DTT (dithiothreitol) (2 µl), and 10 mM dNTP Mix (1 µl) (SuperScript Choice System for cDNA Synthesis (Gibco-BRL)) were added and the mixture was heated at 42°C for 2 min. Then, Super ScriptII RT (2 µl, 400U) (included in the kit as mentioned above) was added and the mixture was heated at 42°C for 1h and then cooled on ice. After the cooling, sterile distilled water treated with DEPC (nacalai tesque) (91 µl) and 5x Second Strand Reaction Buffer (30 µL), 10mM dNTP Mix (3 µl), E. coli DNA Ligase (1 µl, 10U), E. coli DNA Polymerase I (4 µl, 40U) and E. coli RNaseH (1 µl, 2U) (included in the kit as mentioned above) were added, and the mixture was incubated at 16°C for 2 h. After T4 DNA Polymerase (2 µl, 10U) (included in the kit as mentioned above) was added, the mixture was further incubated at 16°C for 5 min, and then added with 0.5M EDTA (10 µl). Phenol/chloroform/isoamyl alcohol solution (NIPPON GENE CO., LTD.) (162 µl) was added and mixed. The mixture was moved to Phase Lock Gel Light (Eppendorf) previously centrifuged at room temperature, at 14,000rpm for 30 seconds, and centrifuged at room temperature, at 14,000rpm for 2 min, and its aqueous phase (145 µl) was moved to an Eppendorf tube. The obtained solution was added with 7.5M ammonium acetate solution (72.5 µl) and ethanol (362.5 µl) and mixed, and then centrifuged at 4°C, at 14,000rpm for 20 min. After the centrifugation, a pellet containing cDNA prepared was obtained by discarding the supernatant. Then, the pellet was added with 80% ethanol (0.5mL) and centrifuged at 4°C, at 14,000rpm for 5 min and the supernatant was discarded. After the same procedure was repeated, the pellet was dried, and lysed with DEPC-treated water (12 µl). As a result, cDNA was obtained from each total RNA prepared in Example 1.

### (2) Preparation of labeled cRNA from cDNA

Each cDNA solution (5 µl) was mixed with DEPC-treated water (17 µl) and 10x HY Reaction Buffer (4 µl), 10x Biotin Labeled Ribonucleotides (4 µl), 10x DTT (4 µl), 10x RNase Inhibitor Mix (4 µl) and 20x T7 RNA Polymerase (2 µl) (BioArray High Yield RNA Transcript Labeling Kit (ENZO)), and incubated at 37°C for 5 h to prepare labeled cRNA. After the incubation, DEPC-treated water (60 µl) was added to the solution, and the labeled cRNA prepared was purified by RNeasy Mini Kit (GIAGEN) according to the manufacture's protocol.

### (3) Fragmentation of labeled cRNA

A solution containing each labeled cRNA (20 µg) was added with 5x Fragmentation Buffer (200 mM Tris-acetic acid pH 8.1 (Sigma), 500 mM potassium acetate (Sigma), 150mM magnesium acetate (Sigma)) (8 µl), and the solution (40 µl) was heated at 94°C for 35 min and placed on ice. As a result, fragmentation of the labeled cRNA was achieved.

### (4) Hybridization of fragmented cRNA with a probe array

Each fragmented cRNA (40 µl) was added with 5nM Control Oligo B2 (Amersham) (4µl), 100x Control cRNA Cocktail (4 µl), Herring sperm DNA (Promega) (40 µg), Acetylated BSA (Gibco-BRL) (200 µg), 2x MES Hybridization Buffer (200mM MES, 2M [Na⁺], 40mM EDTA, 0.02% Tween20 (Pierce), pH 6.5-6.7) (200 µl) and DEPC-treated water (144 µl) to yield a hybridization cocktail (400 µl). Each hybridization cocktail obtained was heated at 99 °C for 5min and then 45°C for 5min. After heated, the hybridization cocktail was centrifuged at room temperature, at 14,000rpm for 5min to obtain the supernatant.

In the meantime, a prove array was prepared by filling Human genome U133 pribe array (Affymetrix) with 1x MES hybridization buffer, rotating the array in a hybridization oven at 45°C, 60rpm for 10 min and then removing the 1x MES hybridization buffer. The supernatant of each hybridization cocktail obtained as above (200 µl) was added to the probe array, and the probe array was rotated in a hybridization oven at 45°C, 60rpm for 16 h to hybridize the fragmented cRNA to the probe array.

### (5) Staining of a probe array

After the hybridization cocktail was removed from each probe array hybridized with the fragmented cRNA, the probe array was filled with Non-Stringent Wash Buffer (6x SSPE (diluted from 20x SSPE (nacalai tesque)), 0.01% Tween20 and 0.005% Antifoam0-30 (Sigma). Then, the probe array hybridized with the fragmented cRNA was placed to a given position in GeneChip Fluidics Station 400 (Affymetrix) set with Non-Stringent Wash Buffer and Stringent Wash Buffer (100 mM MES, 0.1 M NaCl, 0.01% Tween20). After that, according to a staining protocol EuKGE-WS2, the prove array was stained with the first staining solution (10 µg/mL Streptavidin Phycoerythrin (SAPE) (MolecuLar Probe), 2 mg/mL Acetylated BSA, 100 mM MES, 1 M NaCl (Ambion), 0.05% Tween20, 0.005% Antifoam0-30), and with the second staining solution (100 µg/mL Goat IgG (Sigma), 3 µg/mL Biotinylated Anti-Streptavidin antibody (Vector Laboratories), 2 mg/mL Acetylated BSA, 100 mM MES, 1 M NaCl, 0.05% Tween20, 0.005% Antifoam0-30).

### (6) Scanning of a probe array and (7) analysis of gene expression level

The stained probe array was applied to HP GeneArray Scanner (Affymetrix) and the staining pattern was read. Based on the staining pattern, gene expression on the probe array was analyzed by GeneChip Workstation System (Affymetrix). Then, after normalization according to an analysis protocol, expression level (average difference), and presence or absence of expression, of each probe (i.e., each gene) in each sample were calculated. For each probe, an average value of gene expression level was calculated for each of different kinds of samples, and differences in the expression level and frequency between the different kinds of samples was obtained.

### Example 3

### Analysis of variation in expression

As described below, genes showing increase of expression level and/or frequency in cancer tissues compared to normal tissues originated from various major organs (lung, colon, rectum, stomach, breast, liver, kidney, ovary, pancreas) were selected.

According to the result of the DNA chip analysis for gene expression, from a set of probes whose expression level and/or frequency were increased in cancer tissues compared to the corresponding normal tissues, probes showing specific increase of expression level and/or frequency in many cancer tissues were selected. Then, genes corresponding to those selected probes were checked and selected.
As a result, six genes, BUB1, C10orf3, C20orf42, Lengsin, HIFPH3 and BJ-TSA-9 genes, were selected. Ratios of variation in expression of those six genes are described in Table 2 and expression frequencies are in Table 3.

**[Table 2]**

| Gene | Breast cancer | Colon cancer | Renal cancer | Liver cancer | Lung adeno- carcinoma | Lung squamous cell carcinoma | Pancreas cancer | Gastric cancer | Ovarian cancer | Rectal cancer | Leukemia | Malignant lymphoma | Melanoma |
|---|---|---|---|---|---|---|---|---|---|---|---|---|---|
| BUB1 | 2.1 | 2.0 | 2.6 | 3.1 | 20 | 4.0 | 2.0 | 2.4 | 4.2 | 2.3 | 1.4 | 1.3 | 3.1 |
| C10orf3 | 2.5 | 2.3 | 2.9 | 6.7 | 3.0 | 6.2 | 2.4 | 2.4 | 9.0 | 2.9 | 0.9 | 0.9 | 3.9 |
| C20orf42 | 0.5 | 2.8 | 0.4 | 2.0 | 6.0 | 15.4 | 4.2 | 2.4 | 0.3 | 2.4 | 2.0 | 1.3 | 0.4 |
| Lengsin | 1.0 | 1.0 | 0.4 | 0.7 | 8.5 | 2.9 | 2.1 | 2.3 | 1.9 | 1.2 | 1.3 | 1.8 | 0.9 |
| HIFPH3 | 2.3 | 1.3 | 15.5 | 2.6 | 2.3 | 15.9 | 4.4 | 1.2 | 0.9 | 0.9 | 0.6 | 0.6 | 0.2 |
| BJ-TSA-9 | 1.0 | 0.8 | 1.0 | 1.5 | 9.4 | 8.1 | 2.6 | 3.6 | 4.3 | 0.9 | 14.9 | 2.3 | 0.5 |

**[Table 3]**

| Gene | Breast cancer | Normal breast | Colon cancer | Normal colon | Renal cancer | Normal kidney | Liver cancer | Normal liver | Lung adeno-carcinoma | Normal lung |
|---|---|---|---|---|---|---|---|---|---|---|
| BUB1 | 39 | 8 | 69 | 21 | 8 | 0 | 26 | 0 | 39 | 11 |
| C10orf3 | 62 | 15 | 89 | 54 | 15 | 0 | 21 | 0 | 60 | 13 |
| C20orf42 | 2 | 3 | 97 | 66 | 0 | 0 | U | 0 | 18 | 0 |
| Lengsin | 0 | 0 | 0 | 1 | 0 | 17 | 11 | 13 | 53 | 0 |
| HIFPH3 | 14 | 5 | 35 | 25 | 78 | 2 | 11 | 0 | 25 | 7 |
| BJ-TSA-9 | 2 | 3 | 0 | 1 | 0 | 0 | 0 | 0 | 39 | 0 |
| | | | | | | | | | | |

| Gene | Lung squamous cell carcinoma | Normal lung | Pancreas cancer | Normal pancreas | Gastric cancer | Normal stomach | Ovarian cancer | Normal ovanan | Rectal cancer | Normal rectum |
|---|---|---|---|---|---|---|---|---|---|---|
| BUB1 | 63 | 11 | 22 | 6 | 61 | 23 | 70 | 0 | 74 | 13 |
| C10orf3 | 79 | 6 | 56 | 6 | 89 | 46 | 84 | 20 | 84 | 48 |
| C20orf42 | 48 | 0 | 53 | 0 | 68 | 38 | 3 | 20 | 95 | 81 |
| Lengsin | 8 | 0 | 0 | 0 | 13 | 0 | 5 | 0 | 5 | 0 |
| HIFPH3 | 52 | 0 | 25 | 0 | 16 | 8 | 27 | 20 | 26 | 32 |
| BJ-TSA-9 | 23 | 0 | 4 | 0 | 3 | 0 | 5 | 0 | 0 | 0 |
| | | | | | | | | | | |

| Gene | Leukemia | Malignant lymphoma | Normal bone marrow | Melanoma | Normal skin | | | | | |
|---|---|---|---|---|---|---|---|---|---|---|
| BUB1 | 100 | 76 | 100 | 70 | 3 | | | | | |
| C10orf3 | 83 | 81 | 100 | 50 | 7 | | | | | |
| C20orf42 | 0 | 1 | 0 | 10 | 24 | | | | | |
| Lengsin | 0 | 0 | 0 | 0 | 0 | | | | | |
| HIFPH3 | 17 | 0 | 0 | 0 | 63 | | | | | |
| BJ-TSA-9 | 17 | 0 | 0 | 0 | 1 | | | | | |

Values in Table 2 indicate ratio of variation in expression in various cancer tissues compared to the corresponding normal tissues, wherein the expression level in each cancer tissue was defined in relation to the expression level in each normal tissue analyzed with Human Genome U133 Chip regarded as 1. Values in Table 3 indicate expression frequencies (%) of the genes in various cancer and normal tissues.

BUB1 gene showed increase of expression level in all cancer tissues examined compared to normal tissues and the increase was particularly significant in lung squamous cell carcinoma and ovarian cancer. Expression frequency was also increased, and the increase was particularly significant in lung squamous cell carcinoma, ovarian cancer and melanoma tissues.
C10orf3 gene showed increase of expression level in breast cancer, colon cancer, renal cancer, liver cancer, lung adenocarcinoma, lung squamous cell carcinoma, pancreas cancer, gastric cancer, ovarian cancer, rectal cancer and melanoma tissues compared to normal tissues and the increase was particularly significant in ovarian cancer, liver cancer and lung squamous cell carcinoma. Expression frequency was also increased in all cancers except for blood cancer as observed in the expression level, and the increase was particularly significant in lung squamous cell carcinoma, pancreas cancer and ovarian cancer.

C20orf42 gene showed increase of expression level in colon cancer, liver cancer, lung adenocarcinoma, lung squamous cell carcinoma, pancreas cancer, gastric cancer, rectal cancer, leukemia and malignant lymphoma tissues compared to normal tissues, and the increase was particularly significant in lung squamous cell carcinoma and lung adenocarcinoma. Expression frequency was also increased and the increase was particularly significant in lung adenocarcinoma, lung squamous cell carcinoma and pancreas cancer.
Lengsin gene showed increase of expression level in lung adenocarcinoma, lung squamous cell carcinoma and gastric cancer compared to normal tissues, and the increase was particularly significant in lung adenocarcinoma. Expression frequency was also increased in lung adenocarcinoma, lung squamous cell carcinoma and gastric cancer as observed in the expression level, and the increase was particularly significant in lung adenocarcinoma.

HIFPH3 gene showed increase of expression level in tissues of breast cancer, colon cancer, renal cancer, liver cancer, lung adenocarcinoma, lung squamous cell carcinoma, pancreas cancer and gastric cancer compared to normal tissues, and the increase was particularly significant in renal cancer and lung squamous cell carcinoma. Expression frequency was also increased in breast cancer, colon cancer, renal cancer, liver cancer, lung adenocarcinoma, lung squamous cell carcinoma, pancreas cancer and gastric cancer as observed in the expression level, and the increase was particularly significant in renal cancer, lung squamous cell carcinoma and pancreas cancer.
BJ-TSA-9 gene showed increase of expression level in lung adenocarcinoma, lung squamous cell carcinoma and leukemia compared to normal tissues, and the increase was particularly significant in leukemia, lung adenocarcinoma and lung squamous cell carcinoma. Expression frequency was also increased in lung adenocarcinoma, lung squamous cell carcinoma and leukemia as observed in the expression level, and the increase was particularly significant in lung adenocarcinoma and lung squamous cell carcinoma.

AS shown above, the selected six genes showed increase of expression level and frequency specifically in cancer tissues compared to the corresponding normal tissues. Accordingly, those six genes and expression products thereof (i. e., proteins) were found to be useful as disease markers for cancers (especially for cancers as mentioned above). Example 4

### Expression analysis of tumor antigen genes in different cells and tissues by RT-PCR method

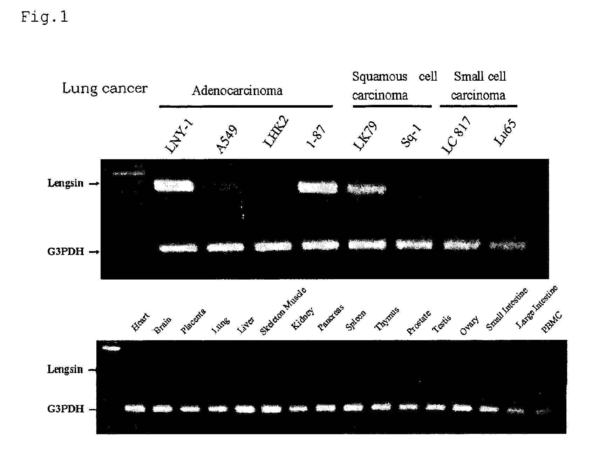

Expression of the above six genes in different cancer cell lines, cancer tissues, and normal tissues were analyzed by RT-PCR method. cDNAs were prepared with oligo dT primer from RNAs extracted from different cancer cell lines and cancer tissues using Isogen reagent (Nippon Gene) or RNAs derived from normal tissues which were commercially available (clontech), and amplified by PCR reactions (40 cycles) using the combinations of primers described in Table 4 (BUB1 gene, primer 1: SEQ ID NO: 208, primer 2: SEQ ID NO: 209; C10orf3 gene, primer 1: SEQ ID NO: 210, primer 2: SEQ ID NO: 211; C20orf42 gene, primer 1: SEQ ID NO: 206, primer 2: SEQ ID NO: 207; Lengsin gene, primer 1: SEQ ID NO: 202, primer 2: SEQ ID NO: 203; HIFPH3 gene, primer 1: SEQ ID NO: 212, primer 2: SEQ ID NO: 213; BJ-TSA-9 gene, primer 1: SEQ ID NO: 204, primer 2: SEQ ID NO: 205). After that, the amplified cDNAs were separated by electrophoresis to be analyzed. As a positive control, expression of G3PDH gene was confirmed by RT-PCR method in the same way as described above. A part of the cancer and normal tissues used were prepared after informed consent was obtained mainly from surgical samples. The results are shown in Figs. 1 to 6.

**[Table 4]**

| Gene | Primer 1 | Primer 2 |
|---|---|---|
| lengsin | tggcactggaagaagatcaa | tcaccacaactttgttgtttgtt |
| BJ-TSA-9 | cagatccaggtgcctctgac | tcaggtaatccaaccaccttg |
| C20orf42 | tgaatttctgaggccttgct | tgttctcagcagcaaacagg |
| BUB1 | ttatctgctggcttggcact | gcttttgccttaacaaatcca |
| C10orf3 | tgtccattgttaagaggtggtg | tgagagggctacatgggttt |
| HIFPH3 | catccctgtcttgtgtgtgg | ccaacagccctggattaaga |

BUB1 gene was not expressed, or expressed at a low level, in most of the normal tissues, but highly expressed in oral squamous cell carcinoma, renal cancer, large bowel cancer, gastric cancer, pancreas cancer, liver cancer, lymphoma and melanoma cell lines.
C10orf3 gene was not expressed, or expressed at a low level, in most of the normal tissues except for testis, but highly expressed in lung adenocarcinoma, lung squamous cell carcinoma and small cell lung cancer cell lines.
C20orf42 gene was not expressed in most of the normal tissues, but highly expressed in colon cancer and pancreas cancer cell lines.
Lengsin gene was not expressed in most of the normal tissues, but highly expressed in lung adenocarcinoma and lung squamous cell carcinoma cell lines.
HIFPH3 gene was expressed in most of the normal tissues, but expressed at a higher level in renal cancer cell lines. In addition, HIFPH3 gene was expressed in many of the cancer tissues originated from renal cancer patients at a higher level compared to the normal tissues.
BJ-TSA-9 was not expressed in most of the normal tissues, but highly expressed in lung adenocarcinoma, lung squamous cell carcinoma, small cell lung cancer, oral cancer, gastric cancer, pancreas cancer and lymphoma cell lines.

In summary, those six genes were highly expressed in various cancer cell lines and tissues, although the types of cancer were different from each other, but not expressed in normal tissues or expressed at a lower lever in normal tissues than in cancer cells or tissues. In addition to the results of the DNA chip analysis, those results strongly suggest that the six genes and expression products thereof (i. e., proteins) are useful as disease markers for cancers (especially for cancers as described above).

### Example 5

### Synthesis and selection of candidate peptides

### (1) Selection of candidate peptides

From the amino acid sequence of Lengsin (SEQ ID NO: 2), peptides consisting of the amino acid sequences of SEQ ID NOS: 13 to 31 and 195 to 201 and those of SEQ ID NOS: 32 to 41 were selected as candidate peptides being potential to bind to HLA-A24 and HLA-A2 molecules, respectively.
From the amino acid sequence of BJ-TSA-9 (SEQ ID NO: 4), peptides consisting of the amino acid sequences of SEQ ID NOS: 42 to 49 and those of SEQ ID NOS: 50 to 58 were selected as candidate peptides being potential to bind to HLA-A24 and HLA-A2 molecules, respectively.
From the amino acid sequence of C20orf42 (SEQ ID NO: 6), peptides consisting of the amino acid sequences of SEQ ID NOS: 59 to 78 and those of SEQ ID NOS: 79 to 88 were selected as candidate peptides being potential to bind to HLA-A24 and HLA-A2 molecules, respectively.

From the amino acid sequence of BUB1 (SEQ ID NO: 8), peptides consisting of the amino acid sequences of SEQ ID NOS: 89 to 117 and those of SEQ ID NOS: 118 to 157 were selected as candidate peptides being potential to bind to HLA-A24 and HLA-A2 molecules, respectively.
From the amino acid sequence of C10orf3 (SEQ ID NO: 10), peptides consisting of the amino acid sequences of SEQ ID NOS: 158 to 165 and those of SEQ ID NOS: 166 to 175 were selected as candidate peptides being potential to bind to HLA-A24 and HLA-A2 molecules, respectively.
From the amino acid sequence of HIFPH3 (SEQ ID NO: 12), peptides consisting of the amino acid sequences of SEQ ID NOS: 176 to 183 and those of SEQ ID NOS: 184 to 194 were selected as candidate peptides being potential to bind to HLA-A24 and HLA-A2 molecules, respectively. The position on the amino acid sequence from which the peptide is derived as well as the length and the amino acid sequence of the peptide are listed in Tables 5 to 16 below.

**[Table 5]**

| Lengsin A24 peptides | | |
|---|---|---|
| Peptide | Amino Acid Sequence | SEQ ID NO |
| Lengsin 295(10) | KYNYIASFFI | 13 |
| Lengsin_368(9) | RYSKDRKDL | 14 |
| Lengsin_447(9) | FYQVEPSEI | 15 |
| Lengsin_489(9) | KYELENEEI | 16 |
| Lengsin_49(9) | DMSNSNDCM | 17 |
| Lengsin_56(9) | CMRDSSQIL | 18 |
| Lengsin_72(9) | RMKHIRQAM | 19 |
| Lengsin_86(10) | QFVRFEATDL | 20 |
| Lengsin_118(10) | CMPRGYLEVI | 21 |
| Lengsin_142(9) | CFNSDIVLM | 22 |
| Lengsin_160(10) | PWADRTARVI | 23 |
| Lengsin_172(10) | TFTVTGEPLL | 24 |
| Lengsin_199(9) | GFSLLSAFI | 25 |
| Lengsin_238(10) | PFMQELVDGL | 26 |
| Lengsin_265(10) | QMEISFLPEF | 27 |
| Lengsin_318(10) | LWDVDRKKNM | 28 |
| Lengsin_383(10) | TWGYNDNSCI | 29 |
| Lengsin_385(9) | GYNDNSCIF | 30 |
| Lengsin_479(9) | TFIRYFVAM | 31 |
| Lengsin_149(11) | LMPELSTFRVL | 195 |
| Lengsin_326(12) | NMFCSTSGTEQL | 196 |
| Lengsin_486(12) | AMKKYELENEEI | 197 |
| Lengsin_185(11) | RYIAKRQLSHL | 198 |
| Lengsin_247(11) | LYHTGANVESF | 199 |
| Lengsin_482(11) | RYFVAMKKYEL | 200 |
| Lengsin_207(12) | IYDFCIFGVPEI | 201 |

**[Table 6]**

| Lengsin A2 peptides | | |
|---|---|---|
| Peptide | Amino Acid Sequence | SEQ ID NO |
| Lengsin_313(9) | ILSHSLWDV | 32 |
| Lengsin_239(9) | FMQELVDGL | 33 |
| Lengsin_246(9) | GLYHTGANV | 34 |
| Lengsin_206(10) | FIYDFCIFGV | 35 |
| Lengsin_149(10) | LMPELSTFRV | 36 |
| Lengsin_79(10) | AMAKNRLQFV | 37 |
| Lengsin_312(10) | GILSHSLWDV | 38 |
| Lengsin_270(10) | FLPEFGISSA | 39 |
| Lengsin_231(10) | FLNNHDQPFM | 40 |
| Lengsin_336(10) | QLTITGKKWL | 41 |

**[Table 7]**

| BJ-TSA-9 A24 peptides | | |
|---|---|---|
| Peptide | Amino Acid Sequence | SEQ ID NO |
| BJ-TSA-9_20(10) | QWVRPARADF | 42 |
| BJ-TSA-9_120(10) | SWASAEKPYL | 43 |
| BJ-TSA-9_138(10) | YFQTVKHNNI | 44 |
| BJ-TSA-9_165(10) | LMDVFTDVEI | 45 |
| BJ-TSA-9_227(10) | IYCAKSGRKF | 46 |
| BJ-TSA-9_273(9) | KFTGQAVEL | 47 |
| BJ-TSA-9 281 (9) | LFDEEFRHL | 48 |
| BJ-TSA-9_289(9) | LYASSKPVM | 49 |

**[Table 8]**

| BJ-TSA-9 A2 peptides | | |
|---|---|---|
| Peptide . | Amino Acid Sequence | SEQ ID NO |
| BJ-TSA-9_164(9) | ILMDVFTDV | 50 |
| BJ-TSA-9_252(9) | VLSGSYSFT | 51 |
| BJ-TSA-9_100(9) | LQSGTYFPV | 52 |
| BJ-TSA-9_99(10) | SLOSGTYFPV | 53 |
| BJ-TSA-9_191(10) | VLLDQGGVKL | 54 |
| BJ-TSA-9_199(10) | KLFQEMCDKV | 55 |
| BJ-TSA-9_62(10) | FLSSVEAQYI | 56 |
| BJ-TSA-9 270(10) | ILSKFTGQAV | 57 |
| BJ-TSA-9_163(10) | VILMDVFTDV | 58 |

**[Table 9]**

| C20orf42 A24 peptides | | |
|---|---|---|
| Peptide | Amino Acid Sequence | SEQ ID NO |
| C20orf42_79(10) | KYGVQADAKL | 59 |
| C20orf42_182(9) | LYSKTMTPI | 60 |
| C20orf42_283(10) | KYDAVRINQL | 61 |
| C20orf42_292(10) | LYEQARWAIL | 62 |
| C20orf42_319(9) | QYHISKLSL | 63 |
| C20orf42_381(10) | SYQPEVLNIL | 64 |
| C20orf42_655(9) | EYIGGYIFL | 65 |
| C20orf42_52(9) | VMLKLVEQI | 66 |
| C20orf42_68(9) | CWLLKTHWTL | 67 |
| C20orf42_89(9) | LFTPQHKML | 68 |
| C20orf42_202(10) | TMTWFSDSPL | 69 |
| C20orf42_249(9) | GWLDSSRSL | 70 |
| C20orf42_271(10) | RFKYYSFFDL | 71 |
| C20orf42_392(10) | YWFIFKDTSI | 72 |
| C20orf42_460(10) | QYAQWMAACM | 73 |
| C20orf42_510(9) | NMDMNPECF | 74 |
| C20orf42_545(9) | QMPLVEAKL | 75 |
| C20orf42_554(9) | RFIQAWQSL | 76 |
| C20orf42_558(10) | AWQSLPEFGL | 77 |
| C20orf42_603(10) | TWRFTNIKQW | 78 |

**[Table 10]**

| C20orf42 A2 peptides | | |
|---|---|---|
| Peptide | Amino Acid Sequence | SEQ ID NO |
| C20orf42_69(9) | WLLKTHWTL | 79 |
| C20orf42_361(9) | SLLEDITDI | 80 |
| C20orf42_377(9) | KLFRPKKLL | 81 |
| C20orf42_417(9) | KLNLRGCEV | 82 |
| C20orf42_300(9) | ILLEEIDCT | 83 |
| C20orf42_610(9) | KQWNVNWET | 84 |
| C20orf42_621(9) | VVIEFDQNV | 85 |
| C20orf42_291(10) | QLYEQARWAI | 86 |
| C20orf42_95(10) | KMLRLRLPNL | 87 |
| C20orf42_88(10) | LLFTPQHKML | 88 |

**[Table 11]**

| Bub1 A24 peptides | | |
|---|---|---|
| Peptide | Amino Acid Sequence | SEQ ID NO |
| Bub1_43(10) | EYLITLLEHL | 89 |
| Bub1_61(9) | KYHNDPRFI | 90 |
| Bub1_89(9) | LYNHGIGTL | 91 |
| Bub1_101(9) | LYIAWAGHL | 92 |
| Bub1_139(9) | QYRLFQTRL | 93 |
| Bub1_670(9) | MYSASLLRL | 94 |
| Bub1_801(9) | VYEATQGDL | 95 |
| Bub1_870(9) | SYGTLLNAI | 96 |
| Bub1_1061(9) | HYTNKIRAL | 97 |
| Bub1_29(10) | RYIQWVEENF | 98 |
| Bub1_77(10) | EYNSDLHQFF | 99 |
| Bub1_206(9) | NMERRVITI | 100 |
| Bub1_235(9) | VMYCKEKLI | 101 |
| Bub1_292(9) | KMDELHKKL | 102 |
| Bub1_353(9) | TYQQTPVNM | 103 |
| Bub1_414(10) | EFKPQSGAEI | 104 |
| Bub1_474(10) | MFQAPTLPDI | 105 |
| Bub1_552(10) | TFGERSVSRL | 106 |
| Bub1_574(9) | EFLDDSTVW | 107 |
| Bub1_581(10) | VWGIRCNKTL | 108 |
| Bub1_741(10) | PWDDKLIFKL | 109 |
| Bub1_837(9) | LMERLKPSM | 110 |
| Bub1_880(10) | LYKNTPEKVM | 111 |
| Bub1_922(9) | NFILGNGFL | 112 |
| Bub1_952(10) | DMKLFPKGTI | 113 |
| Bub1_995(10) | VYCMLFGTYM | 114 |
| Bub1_1018(10) | LFRRLPHLDM | 115 |
| Bub1_1037(10) | MWNEFFHVML | 116 |
| Bub1_1057(10) | VFQQHYTNKI | 117 |

**[Table 12]**

| Bub1 A2 peptides | | |
|---|---|---|
| Peptide | Amino Acid Sequence | SEQ ID NO |
| Bub1_781(9) | QLGSKLVYV | 118 |
| Bub1_998(9) | MLFGTYMKV | 119 |
| Bub1_1026(9) | DMWNEFFHV | 120 |
| Bub1_392(9) | TVTDSMFAV | 121 |
| Bub1_900(9) | RMLYMIEQV | 122 |
| Bub1_1017(9) | GLFRRLPHL | 123 |
| Bub1_903(9) | YMIEQVHDC | 124 |
| Bub1_750(9) | LLSGLSKPV | 125 |
| Bub1_44(9) | YLITLLEHL | 126 |
| Bub1_88(9) | FLYNHGIGT | 127 |
| Bub1_292(9) | KMDELHKKL | 128 |
| Bub1_785(9) | KLVYVHHLL | 129 |
| Bub1_268(9) | WVNEDRHYM | 130 |
| Bub1_699(9) | RLTDTDAAI | 131 |
| Bub1_943(9) | ALIDLGQSI | 132 |
| Bub1_746(9) | LIFKLLSGL | 133 |
| Bub1_235(9) | VMYCKEKLI | 134 |
| Bub1_68(9) | FITYCLKFA | 135 |
| BUb1_613(9) | KLPVESVHI | 136 |
| Bub1_471(9) | IMNMFQAPT | 137 |
| Bub1_780(10) | FQLGSKLVYV | 138 |
| Bub1_575(10) | FLDDSTVWGI | 139 |
| Bub1_792(10) | LLGEGAFAQV | 140 |
| Bub1_749(10) | KLLSGLSKPV | 141 |
| Bub1-_25(10) | GEWERYIQWV | 142 |
| Bub1_954(10) | KLFPKGTIFT | 143 |
| Bub1_997(10) | CMLFGTYMKV | 144 |
| Bub1_745(10) | KLIFKLLSGL | 145 |
| Bub1_886(10) | VLTCEAELGV | 146 |
| Bub1_879(10) | NLYKNTPEKV | 147 |
| Bub1_88(10) | FLYNHGIGTL | 148 |
| Bub1_287(10) | QLLKQKMDEL | 149 |
| Bub1_836(10) | QLMERLKPSM | 150 |
| Bub1_613(10) | KLPVESVHIL | 151 |
| Bub1_722(10) | WMQMSSLGTV | 152 |
| Bub1_470(10) | FIMNMFQAPT | 153 |
| Bub1_141(10) | RLFQTRLTET | 154 |
| Bub1_982(10) | WNYQIDYFGV | 155 |
| Bub1_447(10) | GMVQATPSKV | 156 |
| Bub1_1048(10) | DLLRQKLKKV | 157 |

**[Table 13]**

| C10orf3 A24 peptides | | |
|---|---|---|
| Peptide | Amino Acid Sequence | SEQ ID NO |
| C10orf3_193(10) | VYVKGLLAKI | 158 |
| C10orf3_446(10) | QYPATEHRDL | 159 |
| C10orf3_169(10) | EMEIQLKDAL | 160 |
| C10orf3_355(9) | QMQACTLDF | 161 |
| C10orf3_193(10) | VYVKGLLAKI | 162 |
| C10orf3_446(10) | QYPATEHRDL | 163 |
| C10orf3_169(10) | EMEIQLKDAL | 164 |
| C10orf3_355(9) | QMQACTLDF | 165 |

**[Table 14]**

| C10orf3 A2 peptides | | |
|---|---|---|
| Peptide | Amino Acid Sequence | SEQ ID NO |
| C10orf3_197(9) | GLLAKIFEL | 166 |
| C10orf3_376(9) | QLLVILKEL | 167 |
| C10orf3_99(9) | ALLEQLEET | 168 |
| C10orf3_341(9) | KQQEEQTRV | 169 |
| C10orf3_228(9) | YLQEEKQKC | 170 |
| C10orf3_392(9) | TQLESLKQL | 171 |
| C10orf3_282(10) | LLYSQRRADV | 172 |
| C10orf3_50(10) | KLTDKERHRL | 173 |
| C10orf3_350(10) | ALLEQQMQAC | 174 |
| C10orf3_328(10) | LLSQVQFLYT | 175 |

**[Table 15]**

| HIFPH3 A24 peptides | | |
|---|---|---|
| Peptide | Amino Acid Sequence | SEQ ID ND |
| HIFPH3_6(9) | IMRLDLEKI | 176 |
| HIFPH3_27(9) | GFCYLDNFL | 177 |
| HIFPH3_92(10) | SFLLSLIDRL | 178 |
| HIFPH3_112(10) | YYVKERSKAM | 179 |
| HIFPH3_155(10) | NWDAKLHGGI | 180 |
| HIFPH3_167(9) | IFPEGKSFI | 181 |
| HIFPH3_173(9) | SFIADVEPI | 182 |
| HIFPH3 295(9) | RYAMTVWYF | 183 |

**[Table 16]**

| HIFPH3 A2 peptides | | |
|---|---|---|
| Peptide | Amino Acid Sequence | SEQ ID NO |
| HIFPH3_93(9) | FLLSLIDRL | 184 |
| HIFPH3_30(9) | YLDNFLGEV | 185 |
| HIFPH3_18(9) | YIVPCLHEV | 186 |
| HIFPH3_159(9) | KLHGGILRI | 187 |
| HIFPH3_60(9) | QLAGPRAGV | 188 |
| HIFPH3_42(9) | CVLERVKQL | 189 |
| HIFPH3_22(10) | CLHEVGFCYL | 190 |
| HIFPH3_34(10) | FLGEVVGDCV | 191 |
| HIFPH3_96(10) | SLIDRLVLYC | 192 |
| HIFPH3_93(10) | FLLSLIDRLV | 193 |
| HIFPH3_151(10) | YLNKNWDAKL | 194 |

### (2) Peptide synthesis

Among the peptides as mentioned above (SEQ ID NOS: 13 to 201), 26 peptides derived from Lengsin (Table 5), 7 peptides derived from BJ-TSA-9 (Table 7), 20 peptides derived from C20orf42 (Table 9), and 4 peptides derived from C10orf3 (Table 13) were synthesized by Fmoc method

### Example 6

### Evaluation of binding affinities of the peptides derived from different antigens to HLA-A*2402 and HLA-A*0201

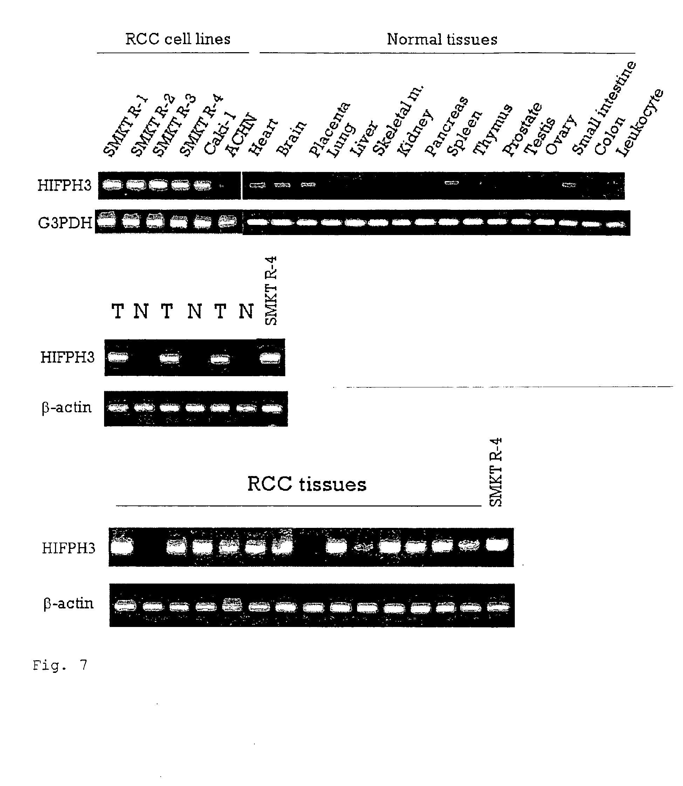

The binding affinities to HLA-A*2402 of the peptides synthesized in Example 1 were determined by the method as described in a literature (J. Immunol. 164:2565, 2000). A cell line RMA-S-A*2402 cell, which was obtained by stably introducing a chimera MHC gene composed of HLA-A*2402 and H-2Kb into a mouse lymphoma cell line RMA-S lacking MHC class I molecule, was incubated at 26°C for 18 hours. RMA-S-A*2402 cells were washed with PBS solution, suspended in culture solution OPTI-MEM (Invitrogen) containing 3µL/mL human (β₂-microglobulin and 100 µL/mL each peptide, and incubated at 26 °C for 3 hours and at 37°C for 3 hours. The cells were washed with PBS solution and treated with anti-HLA-A24 antibodies at 4°C for 30 minutes. Furthermore, the cells were washed with PBS solution, and treated with a PE-labeled anti-mouse IgG antibody at 4°C for 30 minutes. The cells were washed, and suspended in 1 ml of PBS solution containing 1% formalin for fixation. The cells were measured by a device for flow cytometry, FACScan (BD Bioscience), and the binding affinity of the peptide was obtained from the mean fluorescence intensity. The binding affinities of 31 peptides examined are shown in Figs. 7 to 9.

An EB virus-derived peptide (EBV) and HIV virus-derived peptide (HIV), which had been reported to bind to HLA-A*2402 (J. Immunol. 158:3325, 1997 and J. Immunol. 164:2565, 2000, respectively) and were used as positive controls, showed a strong binding activity. An ovalbumin-derived peptide (SL8), which had been reported to bind to H2-Kb (Eur J Immunol. 21:2891, 1991) and was used as a negative control, showed a weak binding activity. All of the 31 peptides examined were demonstrated to bind to HLA-A*2402 in a highly preferable manner. Accordingly, it was found that those 31 peptides were tumor antigen peptides and the proteins from which those peptides derived, BJ-TSA-9, C20orf42 and C10orf3, were tumor antigen proteins.

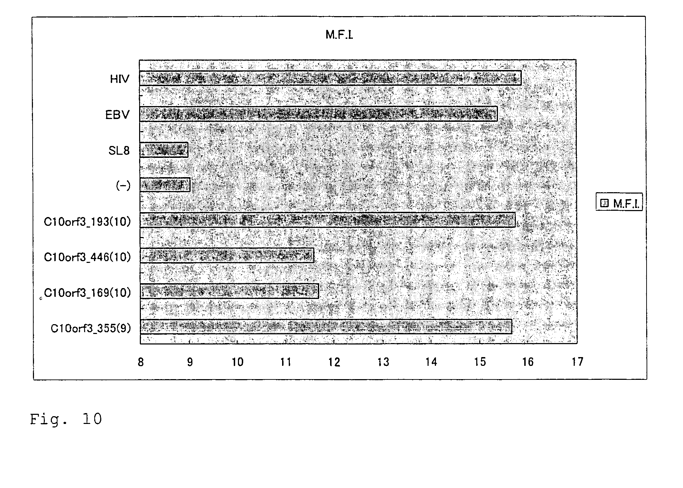

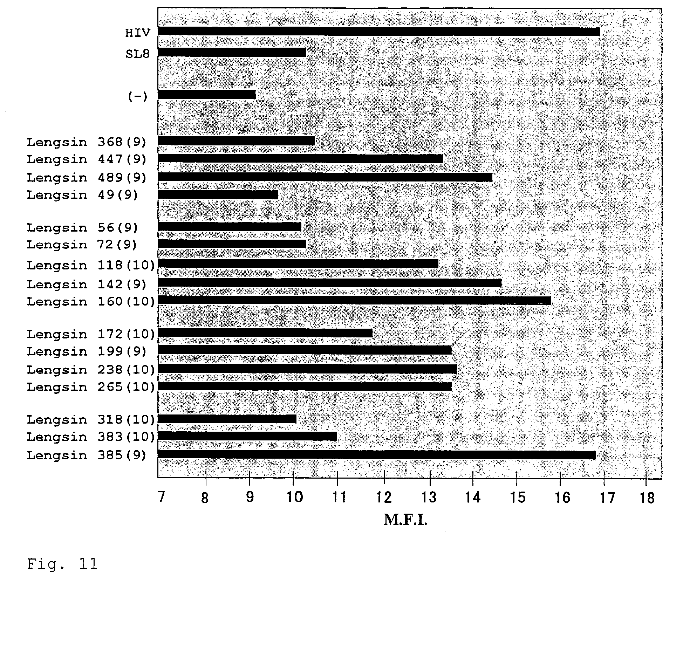

Similarly, the binding affinities of 23 peptides derived from Lengsin to HLA-A*2402 are shown in Figs. 10 and 11. The peptides of SEQ ID NOS: 15, 16, 21, 22, 23, 24, 25, 26, 27, 30, 195, 197, 198, 199, 200 and 201 showed a significant binding affinity to HLA-A*2402. Accordingly, it was found that those peptides were tumor antigen peptides and the protein from which those peptides derived, Lengsin, was a tumor antigen protein.
The binding affinity to HLA-A*0201 can be confirmed by using a human lymphoma cell line T2 in the same way. Also, the binding affinities of the peptides derived from BUB1 and HIFPH3 to HLA can be confirmed similarly.

### Example 7

### CTL induction by tumor antigen-derived peptides

The fact that the proteins BUB1, C10orf3, C20orf42, Lengsin, HIFPH3 and BJ-TSA-9 are tumor antigen proteins and partial peptides thereof are tumor antigen peptides can be confirmed by evaluating the activities of CTLs induced by the peptides showing the binding affinity to HLA in Example 6. Induction of CTL by a peptide and evaluation of the activity of the induced CTL can be conducted according to a known assay using human peripheral blood mononuclear cells or model animals for human as described in a literature (J. Immunol. 169:1611, 2002, WO 02/47474, Int J. Cancer:100,565-570 (2002)). In the case of an assay using human peripheral blood mononuclear cells, peripheral blood is collected from a cancer patient or a healthy subject after obtaining informed-consent, and mononuclear cells are separated by density gradient centrifugation method and cultured in AIM-V culture solution (Invitrogen). After 24-hour-cultivation, nonadherent cells are collected and cultured in AIM-V containing 100 U/mL IL-2. For preparation of antigen-presenting cells, adherent cells are cultured in AIM-V culture solution containing 1000 U/mL IL-4 and 1000 U/mL GM-CSF for 5 days, for another 1 day after addition of each of different tumor antigen peptides, and for another 1 day after addition of 10 ng/mL TNF and 1000 U/mL IFN-α. Non-adherent cells are cultured together with the antigen-presenting cells pulsed with the peptide. The non-adherent cells containing the peptide-pulsed CTL are received the second and third peptide-stimulations on 7 and 14 days after the first peptide-stimulation by using peptide-pulsed antigen presenting cells. After one week from the third stimulation, the cells are co-cultured with target cells (cells expressing HLA to which the administered peptide binds and also a tumor antigen providing the administered peptide or the administered peptide itself, or pulsed with the corresponding peptide previously) labeled with a radioactive agent such as ⁵¹Cr or a nonradioactive agent, and then lysis of the target cells by the CTL are evaluated from the amount of the radioactive or nonradioactive agent in the conditioned medium to assess the activity of the CTL. The activity of CTL can also be assessed by measuring IFN-γ produced in the conditioned medium as result of response between CTL and the target cells using ELISA or ELISPOT.

When using a model animal for human to assess the activity, a tumor antigen peptide suitably formulated is administered to a transgenic mouse expressing human HLA and after about 1 week, spleen cells or other lymphatic tissues are obtained. The cells obtained are stimulated *in vitro* with the same peptide as administered and cultured for about 5 days. The resulting cells are regard to correspond to a CTL population and the activity of the CTL can be assessed as described above.

### Example 8

### CTL induction by the C10orf3-derived peptide

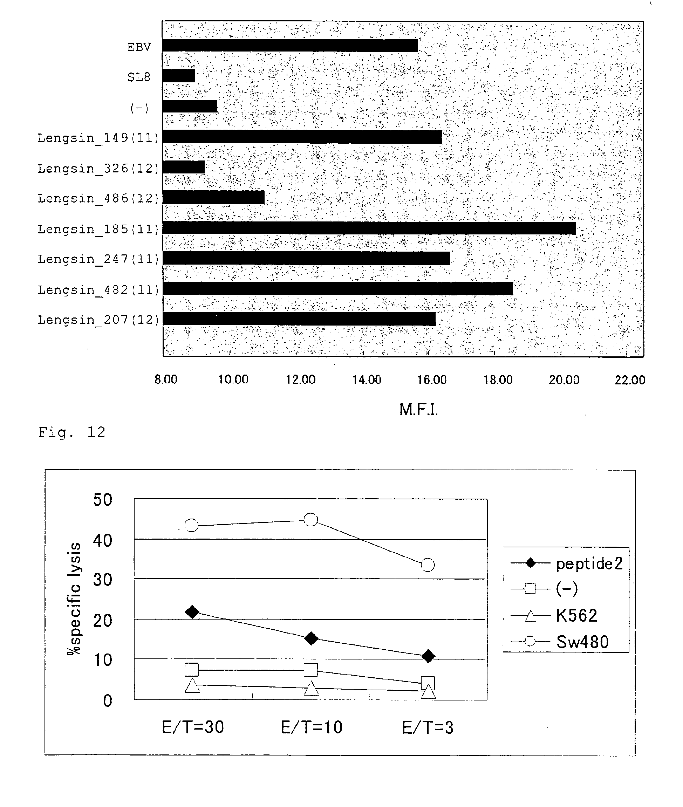

As described in Example 7, peripheral blood mononuclear cells (PBMCs) of a HLA-A*2402-positive cancer patient were stimulated with a C10orf3-derived peptide, C10orf3_193(10), to induce a CTL. The CTL activity was determined by using as target cells T2A24 cell (produced by introducing HLA-A*2402 gene into T2 cell (ATCC No. CRL-1992) which does not have the gene) and a cancer cell line Sw480 which was positive for HLA-A*2402 and C10orf3 and the results are shown in Fig. 12. The CTL clone induced showed a peptide-specific cytotoxic activity. In addition, the CTL clone induced killed the HLA-A*2402 and C10orf3-positive cancer cell line but not HLA-negative K562 cell. This result confirms that the peptide C10orf3_193(10) (SEQ ID NOS: 158, 162) is a tumor antigen peptide and also that C10orf3 is a tumor antigen protein.

### Example 9

### CTL induction by the Lengsin-derived peptide

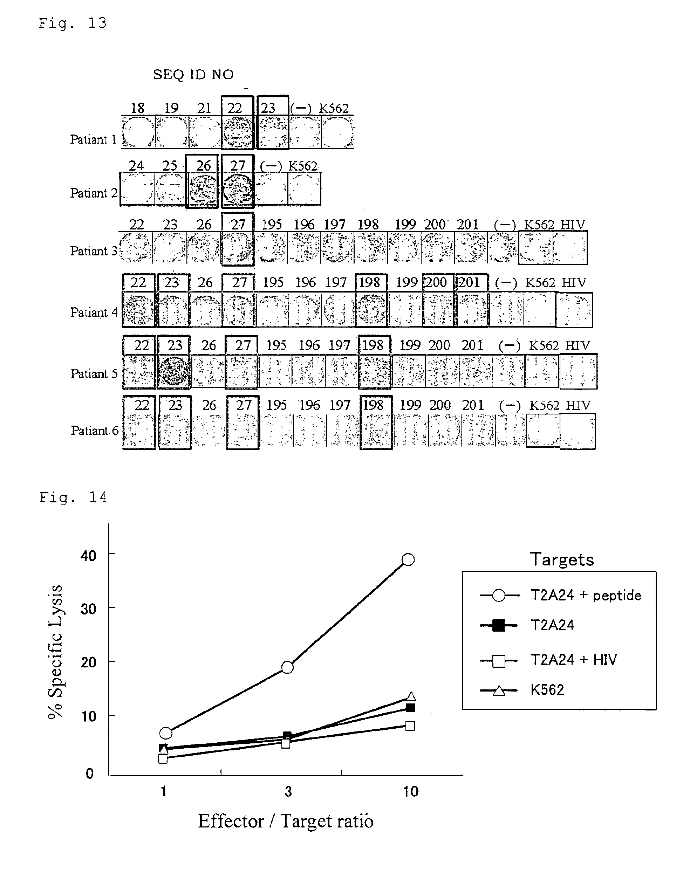

Some of the synthesized peptides being potential to bind to HLA-A*2402 were mixed and used for stimulation of PBMCs of HLA-A*2402-positive cancer patients to induce CTLs, as described in Example 7. After the induction, response to each peptide was detected by measuring IFN-γ by ELISPOT and the results are shown in Fig. 13. The peptides of SEQ ID NOS: 22, 23, 26, 27, 198, 200 and 201 induced peptide-specific CTLs. Those results confirm that the peptides of SEQ ID NOS: 22, 23, 26, 27, 198, 200 and 201 are tumor antigen peptides and also that Lengsin is a tumor antigen protein.
Then, PBMCs of a cancer patient was stimulated with the peptide of SEQ ID NO: 22 to induce a CTL and the CTL-inducing activity was detected as a cytotoxic activity. The result is shown in Fig. 14. The CTL showed a cytotoxic activity specifically against the target cell pulsed with the same peptide used for stimulation.

### Example 10

### CTL induction by the HIFPH3-derived peptide

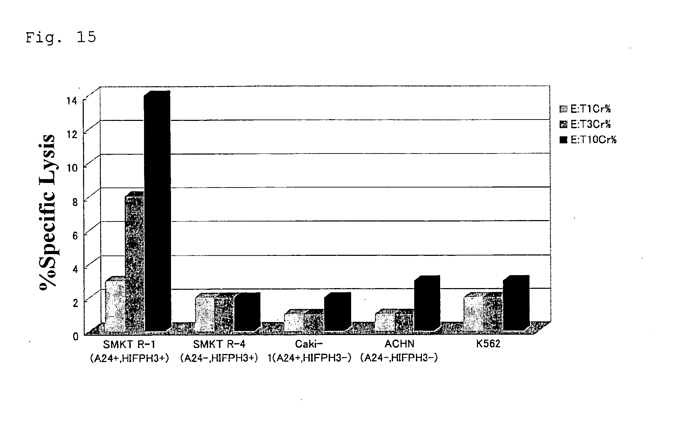

The peptides of SEQ ID NOS: 177, 178, 179 and 183 were mixed and used for stimulation of PBMCs of a HLA-A*2402-positive cancer patient to induce CTLs, as described in Example 7. After the induction, cytotoxic activities against different renal cancer cell lines were detected and the results are shown in Fig. 15. Lymphocytes stimulated with the peptides showed a cytotoxic activity against HLA-A24⁺ HIFPH3⁺ SMKT R-1 cell but not against HLA-A24⁻ HIFPH3⁺ SMKT R-4 cell, HLA-A24⁺ HIFPH3⁻ Caki-1 cell, HLA-A24⁻ HIFPH3⁻ ACHN cell, and HLA⁻ K562 cell. Those results indicate that HLA-A24-restricted CTLs against HIFPH3 were induced by the peptide stimulation and then confirm that peptides HIFPH3_27(9) (SEQ ID NO: 177), HIFPH3_92(10) (SEQ ID NO: 178), HIFPH3_112(10) (SEQ ID NO: 179) and HIFPH3_295(9) (SEQ ID NO: 183) are tumor antigen peptides and that HIFPH3 is a tumor antigen protein.

### Example 11

### CTL induction by the C20orf42-derived peptide

Twenty peptides synthesized for HLA-A*2402 were divided into 5 groups each containing 4 peptides. PBMCs of a HLA-A*2402-positive cancer patients were plated onto 96-well plates and stimulated with each of the groups of peptides to induce CTLs according to Example 7. When PBMCs in 384 wells were stimulated with each group and the cytotoxic activities against the peptides used for the stimulation were examined, cytotoxic activitis specific for the peptides were detected in 8 to 14 wells. This result confirms that the C20orf42-derived peptides are tumor antigen peptides and that C20orf42 is a tumor antigen protein.

### Industrial applicability

The present invention provides novel tumor antigen proteins and peptides as well as uses thereof in the field of cancer immunity. The tumor antigen proteins and peptides derived therefrom can be used for treating cancer patients expressing the tumor antigen proteins.

### Brief Description of Drawings

[Fig.1] Fig. 1 shows the expression level of mRNA of Lengsin gene in major different normal tissues and lung cancer cell lines analyzed by RT-PCR. In the figure, the arrows of Lengsin and G3PDH (positive control) indicate the positions of the bands showing the mRNA level of the genes, respectively. The different normal or cancer tissues are as follows: Heart, Brain, Placenta, Lung, Liver, Skeleton Muscle, Kidney, Pancreas, Spleen, Thymus, Prostate, Testis, Ovary, Small Intestine, Large Intestine, PBMC, Adenocarcinoma, Squamous cell carcinoma, and Small cell carcinoma. The followings are the names of the different lung cancer cell lines: LNY-1, A549, LHK2, 1-87, LK79, Sq-1, LC817 and Lu65.

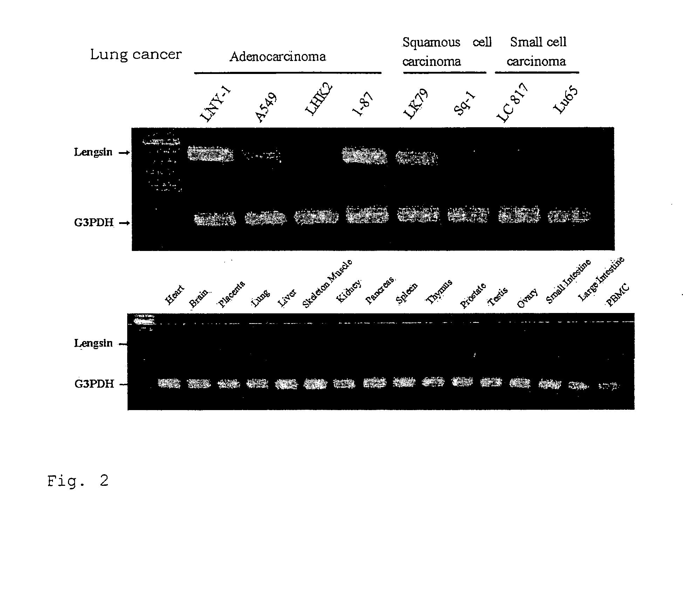

[Fig. 2] Fig. 2 shows the expression level of mRNA of BJ-TSA-9 gene in major different normal tissues and lung, oral, and other cancer cell lines analyzed by RT-PCR. In the figure, the arrows of BJ-TSA-9 and G3PDH (positive control) indicate the positions of the bands showing the mRNA level of the genes, respectively. The different normal or cancer tissues are as follows: Heart, Brain, Placenta, Lung, Liver, Skeleton Muscle, Kidney, Pancreas, Spleen, Thymus, Prostate, Testis, Ovary, Small Intestines, Large Intestine, PBMC, Adenocarcinoma, Squamous cell carcinoma, and Small cell carcinoma. The followings are the names of the cancer cell lines originated from different organs: LNY-1, A549, LHK2, 1-87, LK79, Sq-1, LC817, Lu65, OSC19, OSC20, OSC30, OSC40, OSC70, HSC2, HSC3, HSC4, KOSC3, HO-1-N-1, R3, SW450, SSTW, HS776, CHC20, CHC32, C1R, T2, 888mel, and LG2mel.

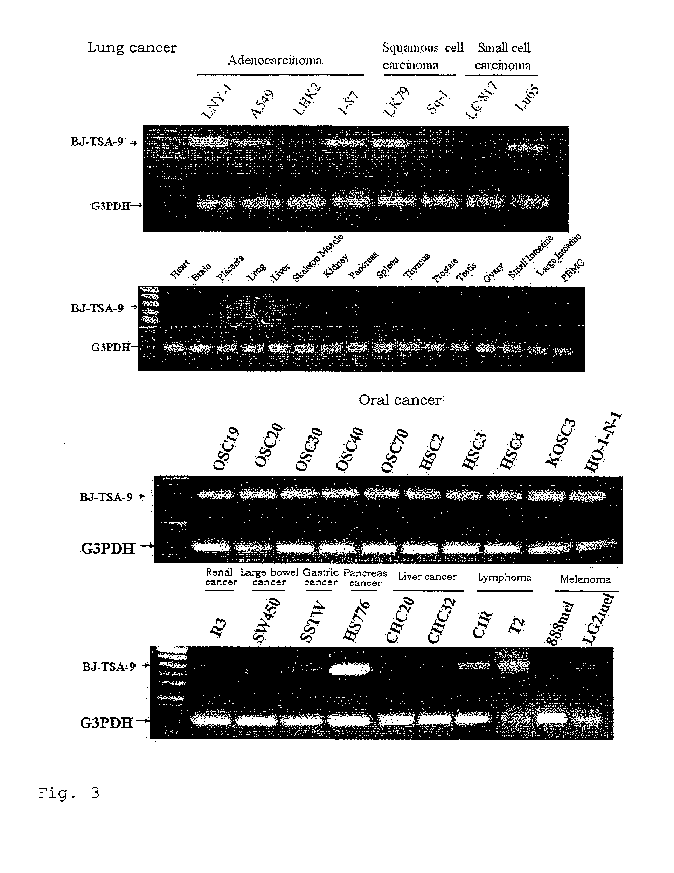

[Fig. 3] Fig. 3 shows the expression level of mRNA of C20orf42 gene in major different normal tissues and colon and pancreas cancer cell lines analyzed by RT-PCR. In the figure, the arrows of C20orf42 and G3PDH (positive control) indicate the positions of the bands showing the mRNA level of the genes, respectively. The different normal or cancer tissues are as follows: Heart, Brain, Placenta, Lung, Liver, Skeleton Muscle, Kidney, Pancreas, Spleen, Thymus, Prostate, Testis, Ovary, Small Intestine, colon, leukocyte, colon cancer, and pancreatic cancer. The followings are the names of the different colon and pancreas cancer cell lines: SW480, SW620, colo205, WiDR, CFPAC, PK8, SuSu86 and PUN.

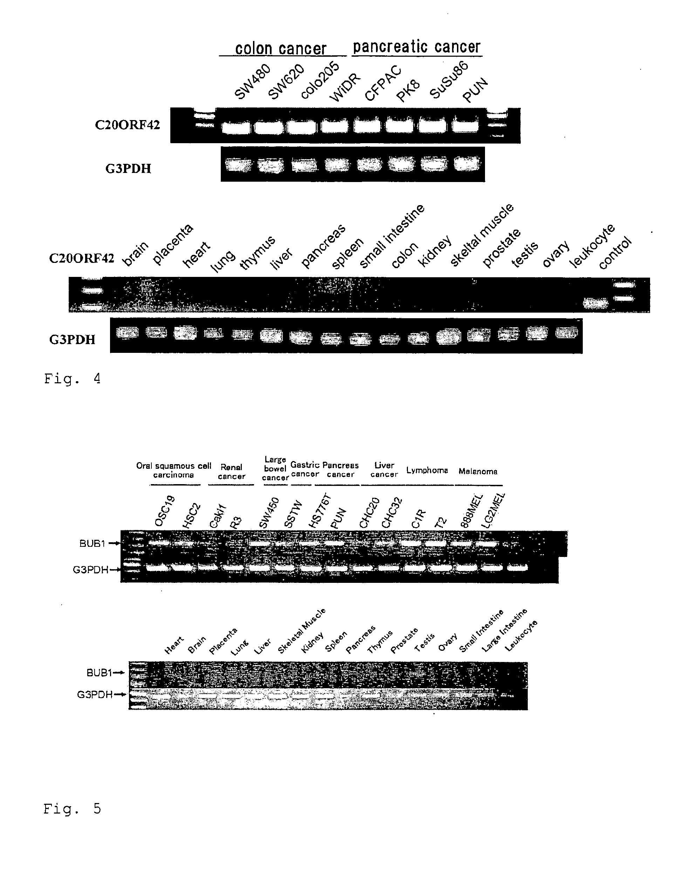

[Fig. 4] Fig. 4 shows the expression level of mRNA of BUB1 gene in major different normal tissues and oral squamous cell carcinoma, renal cancer, large bowel cancer, gastric cancer, pancreas cancer, liver cancer, lymphoma, and melanoma cell lines analyzed by RT-PCR. In the figure, the arrows of BUB1 and G3PDH (positive control) indicate the positions of the bands showing the mRNA level of the genes, respectively. The followings are the names of the cancer cell lines originated from different organs: OSC19, HSC2, Cakil, R3, SW450, SSTW, HS776T, PUN, CHC20, CHC32, C1R, T2, 888mel, and LG2MEL.

[Fig. 5] Fig. 5 shows the expression level of mRNA of C10orf3 gene in major different normal tissues and lung cancer cell lines analyzed by RT-PCR. In the figure, the arrows of C10orf3 and G3PDH (positive control) indicate the positions of the bands showing the mRNA level of the genes, respectively. The different normal or cancer tissues are as follows: Heart, Brain, Placenta, Lung, Liver, Skeleton Muscle, Kidney, Pancreas, Spleen, Thymus, Prostate, Testis, Ovary, Small Intestine, Large Intestine, PBMC, Adenocarcinoma, and Squamous cell carcinoma, Small cell carcinoma. The followings are the names of the cancer cell lines originated from different organs: LNY-1, A549, LHK2, 1-87, LK79, Sq-1, LC817, and Lu65.

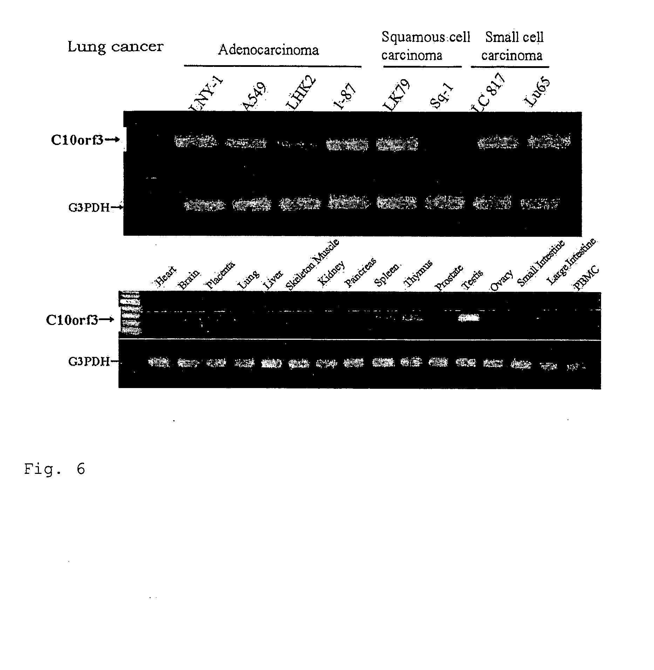

[Fig. 6] Fig.6 shows the expression level of mRNA of HIFPH3 gene in major different normal tissues and renal cacer tissues as well as renal cancer cell lines analyzed by RT-PCR. In the figure, the arrows of HIFPH3 and G3PDH and β-actin (positive controls) indicate the positions of the bands showing the mRNA level of the genes, respectively. The different normal or cancer tissues are as follows: Heart, Brain, Placenta, Lung, Liver, Skeleton Muscle, Kidney, Pancreas, Spleen, Thymus, Prostate, Testis, Ovary, Small Intestine, colon, leukocyte, RCC (renal cell cancer). T and N mean tumor and normal tissues, respectively. The followings are the names of the renal cancer cell lines: SMKTR-1, SMKTR-2, SMKTR-3, SMKTR-4, Caki-1, and ACHN.

[Fig. 7] Fig 7 shows the binding affinities to HLA-A*2402 of 8 peptides derived from BJ-TSA-9, a EB-virus-derived peptide (EBV) and a HIV-derived peptide (HIV) (positive controls), and an Ovalbumin-derived peptide (SL8) (negative control). In the figure, the horizontal axis indicates mean fluorescence intensity (MFI, corresponding to the binding affinity), and the vertical axis indicates the names of the peptides. The results obtained without peptide addition are mentioned as (-).

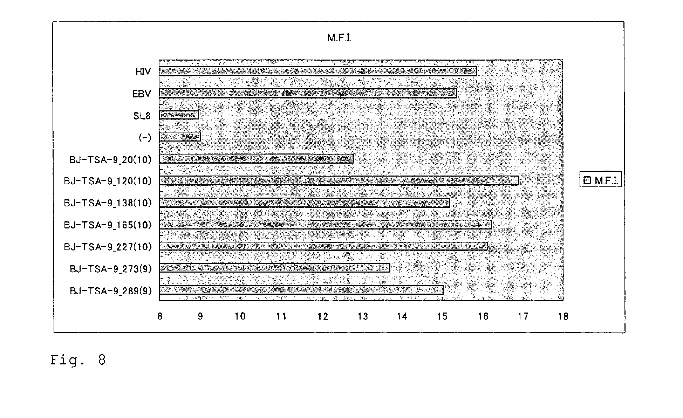

[Fig. 8] Fig. 8 shows the binding affinities to HLA-A*2402 of 20 peptides derived from C20orf42, a EB-virus-derived peptide (EBV) and a HIV-derived peptide (HIV) (positive controls), and an Ovalbumin-derived peptide (SL8) (negative control). In the figure, the horizontal axis indicates mean fluorescence intensity (MFI, corresponding to the binding affinity), and the vertical axis indicates the names of the peptides. The results obtained without peptide addition are mentioned as (-).

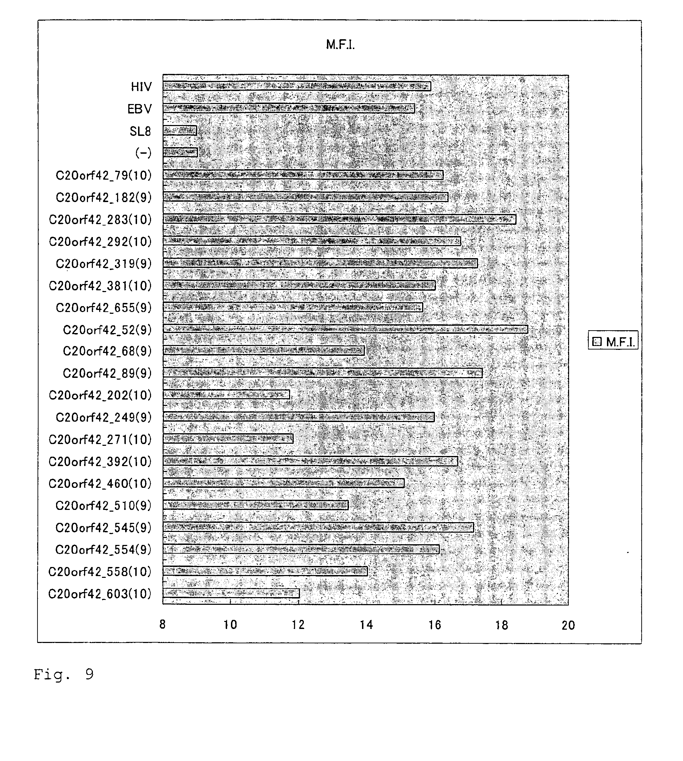

[Fig. 9] Fig. 9 shows the binding affinities to HLA-A*2402 of 4 peptides derived from C10orf3, a EB-virus-derived peptide (EBV) and a HIV-derived peptide (HIV) (positive controls), and an Ovalbumin-derived peptide (SL8) (negative control). In the figure, the horizontal axis indicates mean fluorescence intensity (MFI, corresponding to the binding affinity), and the vertical axis indicates the names of the peptides. The results obtained without peptide addition are mentioned as (-).

[Fig. 10] Fig. 10 shows the binding affinities to HLA-A*2402 of 16 peptides derived from Lengsin, a HIV-derived peptide (HIV) (positive control), and an Ovalbumin-derived peptide (SL8) (negative control). In the figure, the horizontal axis indicates mean fluorescence intensity (MFI, corresponding to the binding affinity), and the vertical axis indicates the names of the peptides. The results obtained without peptide addition are mentioned as (-).

[Fig. 11] Fig. 11 shows the binding affinities to HLA-A*2402 of 7 peptides derived from Lengsin, a EB-virus-derived peptide (EBV) (positive control), and an Ovalbumin-derived peptide (SL8) (negative control). In the figure, the horizontal axis indicates mean fluorescence intensity (MFI, corresponding to the binding affinity), and the vertical axis indicates the names of the peptides. The results obtained without peptide addition are mentioned as (-).

[Fig. 12] Fig. 12 shows the cytotoxic activity of the CTL induced by stimulating PBMCs of a HLA-A*2402-positive cancer patient with C10orf3_193(10) peptide. In the figure, "peptide2" and "Sw480" indicate the results using as target cells T2-A24 cell pulsed with the peptide and a cancer cell line Sw480 which is positive for HLA-A*2402 and C10orf3, respectively. Similarly, "K562" and "(-)" indicate the results using as target cells HLA-negative K562 cell and T2-A24 cell without peptide addition, respectively.

[Fig. 13] Fib. 13 shows a response of a CTL induced by stimulating PBMCs of HLA-A*2402-positive cancer patients with a Lengsin-derived peptide which was detected by measuring INF-γ with ELOSPOT.

[Fig. 14] Fig.14 shows a cytotoxic activity of a CTL induced by stimulating PBMCs of a cancer patient with Lengsin_142(9) peptide. In the figure, "T2A24+peptide" and "T2A24" indicate the results using as target cells T2-A24 cells pulsed and not pulsed with the peptide, respectively. Similarly, "T2A24 + HIV" and "K562" indicate the results using as target cells T2-A24 cell pulsed with a HIV-derived peptide and HLA-negative K562 cell, respectively.

[Fig. 15] Fig.15 is a graph showing the result of inducing CTLs by stimulating PBMCs of a HLA-A*2402-positive cancer patient with a mixture of HIFPH3-derived peptides (SEQ ID NOS: 177, 178, 179 and 183). The vertical axis indicates the cytotoxic activity. In the figure, "E:T1Cr % ", "E:T3Cr%" and "E:T10Cr%" mean that the ratios between the peptide-stimulated PBMCs and cancer cells are 1, 3, and 10, respectively.

### Sequence Listing Free Text

The amino acid sequences of SEQ ID NOS: 13 to 201 refer to synthetic peptides.
The base sequences of SEQ ID NOS: 202 to 213 refer to primers.

## Claims

1. A peptide which comprises a partial peptide derived from Lengsin (Glutamate-ammonia ligase (glutamine synthase) domain containing 1, also referred to as GLULD1), BJ-TSA-9 (Hypothetical protein MGC14128), C20orf42 (URP1, also referred to as Kindlerin), BUB1, C10orf3 or HIFPH3 (egl nine homolog3, also referred to as EGLN3) and is capable of binding to an HLA antigen and is recognized by a CTL.

2. The peptide of Claim 1, wherein the HLA antigen is HLA-A24 or HLA-A2 antigen.

3. The peptide of Claim 2, which comprises the amino acid sequence of any one of SEQ ID NOS: 13 to 201.

4. A peptide which comprises an amino acid sequence which is the same as the amino acid sequence of any one of SEQ ID NOS: 13 to 31, 42 to 49, 59 to 78, 89 to 117, 158 to 165, 176 to 183, and 195 to 201 except that the amino acid at position 2 is substituted by tyrosine, phenylalanine, methionine or tryptophan, and/or the C terminal amino acid by phenylalanine, leucine, isoleucine, tryptophan or methionine, and is capable of binding to HLA-A24 antigen and is recognized by a CTL.

5. A peptide which comprises an amino acid sequence which is the same as the amino acid sequence of any one of SEQ ID NOS:32 to 41, 50 to 58, 79 to 88, 118 to 157, 166 to 175, and 184 to 194 except that the amino acid at position 2 is substituted by leucine, methionine, valine, isoleucine or glutamine and/or the C terminal amino acid by valine or leucine, and is capable of binding to HLA-A2 antigen and is recognized by a CTL.

6. An epitope peptide which comprises the peptide of any one of Claims 1 to 5.

7. A pharmaceutical composition which comprises the peptide of any one of Claims 1 to 6 and a pharmaceutically acceptable carrier.

8. A nucleic acid which comprises a polynucleotide encoding the peptide of any one of Claims 1 to 6.

9. A pharmaceutical composition which comprises the nucleic acid of Claim 8 and a pharmaceutically acceptable carrier.

10. A pharmaceutical composition which comprises Lengsin, BJ-TSA-9, C20orf42, BUB1, C10orf3 or HIFPH3 and a pharmaceutically acceptable carrier.

11. The pharmaceutical composition of Claim 10, wherein Lengsin comprises the amino acid sequence of SEQ ID NO: 2.

12. The pharmaceutical composition of Claim 10, wherein BJ-TSA-9 comprises the amino acid sequence of SEQ ID NO: 4.

13. The pharmaceutical composition of Claim 10, wherein C20orf42 comprises the amino acid sequence of SEQ ID NO: 6.

14. The pharmaceutical composition of Claim 10, wherein BUB1 comprises the amino acid sequence of SEQ ID NO: 8.

15. The pharmaceutical composition of Claim 10, wherein C10orf3 comprises the amino acid sequence of SEQ ID NO: 10.

16. The pharmaceutical composition of Claim 10, wherein HIFPH3 comprises the amino acid sequence of SEQ ID NO: 12.

17. A pharmaceutical composition which comprises a nucleic acid comprising a polynucleotide encoding Lengsin, BJ-TSA-9, C20orf42, BUB1, C10orf3 or HIFPH3 and a pharmaceutically acceptable carrier.

18. The pharmaceutical composition of Claim 17, wherein the polynucleotide encoding Lengsin comprises the base sequence of SEQ ID NO: 1, or encodes the amino acid sequence of SEQ ID NO: 2.

19. The pharmaceutical composition of Claim 17, wherein the polynucleotide encoding BJ-TSA-9 comprises the base sequence of SEQ ID NO: 3, or encodes the amino acid sequence of SEQ ID NO: 4.

20. The pharmaceutical composition of Claim 17, wherein the polynucleotide encoding C20orf42 comprises the base sequence of SEQ ID NO: 5, or encodes the amino acid sequence of SEQ ID NO: 6.

21. The pharmaceutical composition of Claim 17, wherein the polynucleotide encoding BUB1 comprises the base sequence of SEQ ID NO: 7, or encodes the amino acid sequence of SEQ ID NO: 8.

22. The pharmaceutical composition of Claim 17, wherein the polynucleotide encoding C10orf3 comprises the base sequence of SEQ ID NO: 9, or encodes the amino acid sequence of SEQ ID NO: 10.

23. The pharmaceutical composition of Claim 17, wherein the polynucleotide encoding HIFPH3 comprises the base sequence of SEQ ID NO: 11, or encodes the amino acid sequence of SEQ ID NO: 12.

24. A method of preparing an antigen presenting cell, wherein a cell having an antigen-presenting ability is brought into contact in vitro with any one of:
(a) the peptide of any one of Claims 1 to 6,
(b) the nucleic acid of Claim 8,
(c) Lengsin, BJ-TSA-9, C20orf42, BUB1, C10orf3 or HIFPH3, and
(d) a nucleic acid comprising a polynucleotide encoding Lengsin, BJ-TSA-9, C20orf42, BUB1, C10orf3 or HIFPH3.

25. An antigen presenting cell prepared by the method of Claim 24.

26. A pharmaceutical composition which comprises the antigen presenting cell of Claim 25 and a pharmaceutically acceptable carrier.

27. A method of inducing a CTL, wherein peripheral blood lymphocytes are brought into contact in vitro with any one of:
(a) the peptide of any one of Claims 1 to 6,
(b) the nucleic acid of Claim 8,
(c) Lengsin, BJ-TSA-9, C20orf42, BUB1, C10orf3 or HIFPH3, and
(d) a nucleic acid comprising a polynucleotide encoding Lengsin, BJ-TSA-9, C20orf42, BUB1, C10orf3 or HIFPH3.

28. A CTL induced by the method of Claim 27.

29. A pharmaceutical composition which comprises the CTL of Claim 28 and a pharmaceutically acceptable carrier.

30. The pharmaceutical composition of Claim 7, 9 to 23, 26, or 29, which is used as an inducer of CTL.

31. The pharmaceutical composition of Claim 7, 9 to 23, 26, or 29, which is used as a cancer vaccine.

32. An antibody which specifically binds to the peptide of any one of Claims 1 to 5.

33. An HLA monomer, HLA dimer, HLA tetramer or HLA pentamer which comprises the peptide of any one of Claims 1 to 5 and an HLA antigen.

34. A reagent for detecting a CTL specific to a tumor antigen peptide derived from Lengsin, BJ-TSA-9, C20orf42, BUB1, C10orf3 or HIFPH3, which comprises as a component the HLA monomer, HLA dimer, HLA tetramer or HLA pentamer of Claim 33.

35. A disease marker consisting of a polynucleotide and/or a complementary polynucleotide thereof, wherein the polynucleotide comprises at least 15 contiguous nucleotides from the base sequence of Lengsin, BJ-TSA-9, C20orf42, BUB1, C10orf3 or HIFPH3 gene.

36. The disease marker of Claim 35, which is used as a probe or primer for detecting cancer.

37. The disease marker of Claim 35 or 36, wherein the disease marker derived from Lengsin gene is used for lung adenocarcinoma, lung squamous cell carcinoma or gastric cancer.

38. The disease marker of Claim 35 or 36, wherein the disease marker derived from BJ-TSA-9 gene is used for leukemia, lung adenocarcinoma, lung squamous cell carcinoma, small cell lung cancer, oral cancer, gastric cancer, pancreas cancer or lymphoma.

39. The disease marker of Claim 35 or 36, wherein the disease marker derived from C20orf42 gene is used for lung squamous cell carcinoma, lung adenocarcinoma, liver cancer, gastric cancer, leukemia, malignant lymphoma tissues, rectal cancer, colon cancer or pancreas cancer.

40. The disease marker of Claim 35 or 36, wherein the disease marker derived from BUB1 gene is used for breast cancer, lung adenocarcinoma, lung squamous cell carcinoma, ovarian cancer, oral squamous cell carcinoma, renal cancer, large bowel cancer (colon cancer, rectal cancer), gastric cancer, pancreas cancer, liver cancer, leukemia, lymphoma or melanoma.

41. The disease marker of Claim 35 or 36, wherein the disease marker derived from C10orf3 gene is used for breast cancer, colon cancer, rectal cancer, renal cancer, gastric cancer, ovarian cancer, liver cancer, pancreas cancer, lung squamous cell carcinoma, lung adenocarcinoma, small cell lung cancer or melanoma.

42. The disease marker of Claim 35 or 36, wherein the disease marker derived from HIFPH3 gene is used for breast cancer, colon cancer, gastric cancer, renal cancer, pancreas cancer, liver cancer, lung adenocarcinoma or lung squamous cell carcinoma.

43. A method for detecting cancer which comprises the following steps (a), (b) and (c):
(a) allowing RNA prepared from a biological sample of a test subject or complementary polynucleotides transcribed therefrom to hybridize with the disease marker of any one of Claims 35 to 42;
b) detecting RNA prepared from the biological sample or complementary polynucleotides transcribed therefrom hybridized with the disease marker by using the disease marker as an indicator; and
(c) determining whether or not the test subject has cancer based on the result of the detection in (b).

44. The method of Claim 43, wherein the test subject is determined to have cancer in the step (C) when the result of the detection from the test subject is compared with that from a healthy subject and the level of hybridization to the disease marker observed in the test subject is higher than that observed in the healthy subject.

45. A disease marker for cancer which comprises an antibody specifically recognizing Lengsin, BJ-TSA-9, C20orf42, BUB1, C10orf3 or HIFPH3.

46. The disease marker of Claim 45, which is used as a probe for detecting cancer.

47. A method for detecting cancer which comprises the following steps (a), (b) and (c):
(a) allowing proteins prepared from a biological sample of a test subject to bind to the disease marker of Claim 45 or 46;
b) detecting proteins prepared from the biological sample or partial peptides derived therefrom bound to the disease marker by using the disease marker as an indicator; and
(c) determining whether or not the test subject has cancer based on the result of the detection in (b).

48. The method of Claim 47, wherein the test subject is determined to have cancer in the step (C) when the result of the detection from the test subject is compared with that from a healthy subject and the level of binding to the disease marker observed in the test subject is higher than that observed in the healthy subject.
